# EUROPEAN PATENT SPECIFICATION

(11) **EP 3 133 170 B1**
(45) Date of publication and mention of the grant of the patent: **18.03.2020**
(21) Application number: 16180033.9
(22) Date of filing: 10.09.2009
(51) Int. Cl.: C12Q 1/6827, C12Q 1/6841

(54) **IMAGING INDIVIDUAL MRNA MOLECULES USING MULTIPLE SINGLY LABELED PROBES**
ABBILDUNG EINZELNER MRNA-MOLEKÜLE MITHILFE MEHRERER INDIVIDUELL MARKIERTER SONDEN
IMAGERIE DE MOLÉCULES D'ARNM INDIVIDUELLES UTILISANT DES SONDES MULTIPLES À MARQUAGE UNIQUE

(30) Priority: 10.09.2008 US 191724 P
(43) Date of publication of application: 22.02.2017
(62) Divisional of application: 09813630.2
(73) Proprietor: Rutgers, the State University of New Jersey, New Brunswick, NJ 08909 (US)
(72) Inventor: RAJ, Arjun, Wynnewood, PA 19096 (US); TYAGI, Sanjay, New York, NY 10038 (US)
(74) Representative: Rees, Kerry

(56) References cited:
- WO-A1-97/14816
- WO-A1-2006/132710
- WO-A2-02/22874
- WO-A2-2007/001986
- US-B1- 6 329 152
- FEMINO A M ET AL: "Visualization of single RNA transcripts in situ", SCIENCE, AMERICAN ASSOCIATION FOR THE ADVANCEMENT OF SCIENCE, WASHINGTON, DC; US, vol. 280, no. 5363, 24 April 1998 (1998-04-24), pages 585-590, XP002306891, ISSN: 0036-8075, DOI: 10.1126/SCIENCE.280.5363.585
- LEE SANGHOON ET AL.: "Use of multiple 16S rRNA-targeted fluorescent probes to increase signal strength and measure cellular RNA from natural planktonic bacteria", MARINE ECOLOGY PROGRESS SERIES, vol. 101, 4 November 1993 (1993-11-04), pages 193-201, XP002685594, ISSN: 0171-8630
- ARJUN RAJ ET AL: "Imaging individual mRNA molecules using multiple singly labeled probes", NATURE METHODS, vol. 5, no. 10, 1 October 2008 (2008-10-01), pages 877-879, XP055041223, ISSN: 1548-7091, DOI: 10.1038/nmeth.1253
- KOSMAN D ET AL: "Multiplex detection of RNA expression in Drosophila embryos", SCIENCE, AMERICAN ASSOCIATION FOR THE ADVANCEMENT OF SCIENCE, vol. 305, no. 5685, 6 August 2004 (2004-08-06), page 846, XP002394668, ISSN: 0036-8075, DOI: 10.1126/SCIENCE.1099247

### SEQUENCE LISTING

<110> University of Medicine and Dentistry of New Jersey
<120> Imaging Individual mRNA Molecules Using Multiple Singly Labeled Probes
<130> 96738.00072
<150> 61/191,724
   <151> 2008-09-10
<160> 482
<170> PatentIn version 3.5
<210> 1
   <211> 1128
   <212> DNA
   <213> Artificial Sequence
<220>
   <223> Probe for Beta-actin
<400> 1
   atggatgacg atatcgctgc gctcgtcgtc gacaacggct ccggcatgtg caaggccggc      60
   ttcgcgggcg acgatgctcc ccgggccgtc ttcccctcca tcgtgggccg ccctaggcac     120
   cagggtgtga tggtgggtat gggtcagaag gactcctacg tgggcgacga ggcccagagc     180
   aagagaggca tcctgaccct gaagtacccc attgaacacg gcattgtcac caactgggac     240
   gatatggaga agatttggca ccacactttc tacaatgagc tgcgtgtggc ccctgaggag     300
   caccctgtgc tgctcaccga ggcccctctg aaccctaagg ccaaccgtga aaagatgacc     360
   cagatcatgt ttgagacctt caacacccca gccatgtacg tagccatcca ggctgtgttg     420
   tccctgtatg cctctggtcg taccactggc attgtgatgg actccggaga cggggtcacc     480
   cacactgtgc ccatctatga gggttacgcg ctccctcatg ccatcctgcg tctggacctg     540
   gctggccggg acctgacaga ctacctcatg aagatcctga ccgagcgtgg ctacagcttc     600
   accaccacag ctgagaggga aatcgtgcgt gacattaaag agaagctgtg ctatgttgcc     660
   ctagacttcg agcaagagat ggccactgcc gcatcctctt cctccctgga gaagagctat     720
   gagctgcctg acggtcaggt catcactatc ggcaatgagc ggttccgatg ccccgaggct     780
   ctcttccagc cttccttcct gggtatggaa tcctgtggca tccatgaaac tacattcaat     840
   tccatcatga agtgtgacgt tgacatccgt aaagacctct atgccaacac agtgctgtct     900
   ggtggcacca ccatgtaccc aggcattgct gacaggatgc agaaggagat tactgccctg     960
   gctcctagca ccatgaagat caagatcatt gctcctcctg agcgcaagta ctctgtgtgg    1020
   attggtggct ctatcctggc ctcactgtcc accttccagc agatgtggat cagcaagcag    1080
   gagtacgatg agtccggccc ctccatcgtg caccgcaaat gcttctag                 1128
<210> 2
   <211> 20
   <212> DNA
   <213> Artificial Sequence
<220>
   <223> Probe for Beta-actin
<400> 2
   atgccggagc cgttgtcgac 20
<210> 3
   <211> 20
   <212> DNA
   <213> Artificial Sequence
<220>
   <223> Probe for Beta-actin
<400> 3
   cgcccgcgaa gccggccttg 20
<210> 4
   <211> 20
   <212> DNA
   <213> Artificial Sequence
<220>
   <223> Probe for Beta-actin
<400> 4
   gaagacggcc cggggagcat 20
<210> 5
   <211> 20
   <212> DNA
   <213> Artificial Sequence
<220>
   <223> Probe for Beta-actin
<400> 5
   ctagggcggc ccacgatgga 20
<210> 6
   <211> 20
   <212> DNA
   <213> Artificial Sequence
<220>
   <223> Probe for Beta-actin
<400> 6
   tacccaccat cacaccctgg 20
<210> 7
   <211> 20
   <212> DNA
   <213> Artificial Sequence
<220>
   <223> Probe for Beta-actin
<400> 7
   tacccaccat cacaccctgg 20
<210> 8
   <211> 20
   <212> DNA
   <213> Artificial Sequence
<220>
   <223> Probe for Beta-actin
<400> 8
   cacgtaggag tccttctgac 20
<210> 9
   <211> 20
   <212> DNA
   <213> Artificial Sequence
<220>
   <223> Probe for Beta-actin
<400> 9
   ggtacttcag ggtcaggatg 20
<210> 10
   <211> 20
   <212> DNA
   <213> Artificial Sequence
<220>
   <223> Probe for Beta-actin
<400> 10
   ggtgacaatg ccgtgttcaa 20
<210> 11
   <211> 20
   <212> DNA
   <213> Artificial Sequence
<220>
   <223> Probe for Beta-actin
<400> 11
   atcttctcca tatcgtccca 20
<210> 12
   <211> 20
   <212> DNA
   <213> Artificial Sequence
<220>
   <223> Probe for Beta-actin
<400> 12
   cattgtagaa agtgtggtgc 20
<210> 13
   <211> 20
   <212> DNA
   <213> Artificial sequence
<220>
   <223> Probe for Beta-actin
<400> 13
   ctcctcaggg gccacacgca 20
<210> 14
   <211> 20
   <212> DNA
   <213> Artificial sequence
<220>
   <223> Probe for Beta-actin
<400> 14
   gcctcggtga gcagcacagg 20
<210> 15
   <211> 20
   <212> DNA
   <213> Artificial Sequence
<220>
   <223> Probe for Beta-actin
<400> 15
   ggttggcctt agggttcaga 20
<210> 16
   <211> 20
   <212> DNA
   <213> Artificial sequence
<220>
   <223> Probe for Beta-actin
<400> 16
   catgatctgg gtcatctttt 20
<210> 17
   <211> 20
   <212> DNA
   <213> Artificial Sequence
<220>
   <223> Probe for Beta-actin
<400> 17
   gctggggtgt tgaaggtctc 20
<210> 18
   <211> 20
   <212> DNA
   <213> Artificial Sequence
<220>
   <223> Probe for Beta-actin
<400> 18
   cagcctggat ggctacgtac 20
<210> 19
   <211> 20
   <212> DNA
   <213> Artificial Sequence
<220>
   <223> Probe for Beta-actin
<400> 19
   accagaggca tacagggaca 20
<210> 20
   <211> 20
   <212> DNA
   <213> Artificial Sequence
<220>
   <223> Probe for Beta-actin
<400> 20
   tccatcacaa tgccagtggt 20
<210> 21
   <211> 20
   <212> DNA
   <213> Artificial Sequence
<220>
   <223> Probe for Beta-actin
<400> 21
   tgtgggtgac cccgtctccg 20
<210> 22
   <211> 20
   <212> DNA
   <213> Artificial sequence
<220>
   <223> Probe for Beta-actin
<400> 22
   gtaaccctca tagatgggca 20
<210> 23
   <211> 20
   <212> DNA
   <213> Artificial Sequence
<220>
   <223> Probe for Beta-actin
<400> 23
   cgcaggatgg catgagggag 20
<210> 24
   <211> 20
   <212> DNA
   <213> Artificial Sequence
<220>
   <223> Probe for Beta-actin
<400> 24
   ggtcccggcc agccaggtcc 20
<210> 25
   <211> 20
   <212> DNA
   <213> Artificial sequence
<220>
   <223> Probe for Beta-actin
<400> 25
   gatcttcatg aggtagtctg 20
<210> 26
   <211> 20
   <212> DNA
   <213> Artificial Sequence
<220>
   <223> Probe for Beta-actin
<400> 26
   aagctgtagc cacgctcggt 20
<210> 27
   <211> 20
   <212> DNA
   <213> Artificial Sequence
<220>
   <223> Probe for Beta-actin
<400> 27
   tttccctctc agctgtggtg 20
<210> 28
   <211> 20
   <212> DNA
   <213> Artificial sequence
<220>
   <223> Probe for Beta-actin
<400> 28
   cttctcttta atgtcacgca 20
<210> 29
   <211> 20
   <212> DNA
   <213> Artificial sequence
<220>
   <223> Probe for Beta-actin
<400> 29
   aagtctaggg caacatagca 20
<210> 30
   <211> 20
   <212> DNA
   <213> Artificial sequence
<220>
   <223> Probe for Beta-actin
<400> 30
   cggcagtggc catctcttgc 20
<210> 31
   <211> 20
   <212> DNA
   <213> Artificial Sequence
<220>
   <223> Probe for Beta-actin
<400> 31
   cttctccagg gaggaagagg 20
<210> 32
   <211> 20
   <212> DNA
   <213> Artificial Sequence
<220>
   <223> Probe for Beta-actin
<400> 32
   tgaccgtcag gcagctcata 20
<210> 33
   <211> 20
   <212> DNA
   <213> Artificial sequence
<220>
   <223> Probe for Beta-actin
<400> 33
   gctcattgcc gatagtgatg 20
<210> 34
   <211> 20
   <212> DNA
   <213> Artificial sequence
<220>
   <223> Probe for Beta-actin
<400> 34
   gagagcctcg gggcatcgga 20
<210> 35
   <211> 20
   <212> DNA
   <213> Artificial Sequence
<220>
   <223> Probe for Beta-actin
<400> 35
   atacccagga aggaaggctg 20
<210> 36
   <211> 20
   <212> DNA
   <213> Artificial sequence
<220>
   <223> Probe for Beta-actin
<400> 36
   tttcatggat gccacaggat 20
<210> 37
   <211> 20
   <212> DNA
   <213> Artificial sequence
<220>
   <223> Probe for Beta-actin
<400> 37
   cttcatgatg gaattgaatg 20
<210> 38
   <211> 20
   <212> DNA
   <213> Artificial Sequence
<220>
   <223> Probe for Beta-actin
<400> 38
   tctttacgga tgtcaacgtc 20
<210> 39
   <211> 20
   <212> DNA
   <213> Artificial Sequence
<220>
   <223> Probe for Beta-actin
<400> 39
   acagcactgt gttggcatag 20
<210> 40
   <211> 20
   <212> DNA
   <213> Artificial Sequence
<220>
   <223> Probe for Beta-actin
<400> 40
   tgggtacatg gtggtgccac 20
<210> 41
   <211> 20
   <212> DNA
   <213> Artificial Sequence
<220>
   <223> Probe for Beta-actin
<400> 41
   ttctgcatcc tgtcagcaat 20
<210> 42
   <211> 20
   <212> DNA
   <213> Artificial sequence
<220>
   <223> Probe for Beta-actin
<400> 42
   taggagccag ggcagtaatc 20
<210> 43
   <211> 20
   <212> DNA
   <213> Artificial Sequence
<220>
   <223> Probe for Beta-actin
<400> 43
   aatgatcttg atcttcatgg 20
<210> 44
   <211> 20
   <212> DNA
   <213> Artificial Sequence
<220>
   <223> Probe for Beta-actin
<400> 44
   gagtacttgc gctcaggagg 20
<210> 45
   <211> 20
   <212> DNA
   <213> Artificial sequence
<220>
   <223> Probe for Beta-actin
<400> 45
   ggatagagcc accaatccac 20
<210> 46
   <211> 20
   <212> DNA
   <213> Artificial sequence
<220>
   <223> Probe for Beta-actin
<400> 46
   ctggaaggtg gacagtgagg 20
<210> 47
   <211> 20
   <212> DNA
   <213> Artificial Sequence
<220>
   <223> Probe for Beta-actin
<400> 47
   tcctgcttgc tgatccacat 20
<210> 48
   <211> 20
   <212> DNA
   <213> Artificial Sequence
<220>
   <223> Probe for Beta-actin
<400> 48
   tggaggggcc ggactcatcg 20
<210> 49
   <211> 20
   <212> DNA
   <213> Artificial sequence
<220>
   <223> Probe for Beta-actin
<400> 49
   ctagaagcat ttgcggtgca 20
<210> 50
   <211> 1815
   <212> DNA
   <213> Artificial sequence
<220>
   <223> Probe of COX-2
<400> 50
   atgctcgccc gcgccctgct gctgtgcgcg gtcctggcgc tcagccatac agcaaatcct      60
   tgctgttccc acccatgtca aaaccgaggt gtatgtatga gtgtgggatt tgaccagtat     120
   aagtgcgatt gtacccggac aggattctat ggagaaaact gctcaacacc ggaatttttg     180
   acaagaataa aattatttct gaaacccact ccaaacacag tgcactacat acttacccac     240
   ttcaagggat tttggaacgt tgtgaataac attcccttcc ttcgaaatgc aattatgagt     300
   tatgtgttga catccagatc acatttgatt gacagtccac caacttacaa tgctgactat     360
   ggctacaaaa gctgggaagc cttctctaac ctctcctatt atactagagc ccttcctcct     420
   gtgcctgatg attgcccgac tcccttgggt gtcaaaggta aaaagcagct tcctgattca     480
   aatgagattg tggaaaaatt gcttctaaga agaaagttca tccctgatcc ccagggctca     540
   aacatgatgt ttgcattctt tgcccagcac ttcacgcatc agtttttcaa gacagatcat     600
   aagcgagggc cagctttcac caacgggctg ggccatgggg tggacttaaa tcatatttac     660
   ggtgaaactc tggctagaca gcgtaaactg cgccttttca aggatggaaa aatgaaatat     720
   cagataattg atggagagat gtatcctccc acagtcaaag atactcaggc agagatgatc     780
   taccctcctc aagtccctga gcatctacgg tttgctgtgg ggcaggaggt ctttggtctg     840
   gtgcctggtc tgatgatgta tgccacaatc tggctgcggg aacacaacag agtatgcgat     900
   gtgcttaaac aggagcatcc tgaatggggt gatgagcagt tgttccagac aagcaggcta     960
   atactgatag gagagactat taagattgtg attgaagatt atgtgcaaca cttgagtggc      1020
   tatcacttca aactgaaatt tgacccagaa ctacttttca acaaacaatt ccagtaccaa      1080
   aatcgtattg ctgctgaatt taacaccctc tatcactggc atccccttct gcctgacacc      1140
   tttcaaattc atgaccagaa atacaactat caacagttta tctacaacaa ctctatattg      1200
   ctggaacatg gaattaccca gtttgttgaa tcattcacca ggcaaattgc tggcagggtt      1260
   gctggtggta ggaatgttcc acccgcagta cagaaagtat cacaggcttc cattgaccag      1320
   agcaggcaga tgaaatacca gtcttttaat gagtaccgca aacgctttat gctgaagccc      1380
   tatgaatcat ttgaagaact tacaggagaa aaggaaatgt ctgcagagtt ggaagcactc      1440
   tatggtgaca tcgatgctgt ggagctgtat cctgcccttc tggtagaaaa gcctcggcca      1500
   gatgccatct ttggtgaaac catggtagaa gttggagcac cattctcctt gaaaggactt      1560
   atgggtaatg ttatatgttc tcctgcctac tggaagccaa gcacttttgg tggagaagtg      1620
   ggttttcaaa tcatcaacac tgcctcaatt cagtctctca tctgcaataa cgtgaagggc      1680
   tgtcccttta cttcattcag tgttccagat ccagagctca ttaaaacagt caccatcaat      1740
   gcaagttctt cccgctccgg actagatgat atcaatccca cagtactact aaaagaacgt      1800
   tcgactgaac tgtag                                                       1815
<210> 51
   <211> 20
   <212> DNA
   <213> Artificial Sequence
<220>
   <223> Probe of COX-2
<400> 51
   gcagcagggc gcgggcgagc 20
<210> 52
   <211> 40
   <212> DNA
   <213> Artificial sequence
<220>
   <223> Probe of COX-2
<400> 52
   cgcaggatgg catgagggac gcaggatggc atgagggagg 40
<210> 53
   <211> 20
   <212> DNA
   <213> Artificial Sequence
<220>
   <223> Probe of COX-2
<400> 53
   ctcatacata cacctcggtt 20
<210> 54
   <211> 20
   <212> DNA
   <213> Artificial Sequence
<220>
   <223> Probe of COX-2
<400> 54
   atcgcactta tactggtcaa 20
<210> 55
   <211> 20
   <212> DNA
   <213> Artificial Sequence
<220>
   <223> Probe of COX-2
<400> 55
   gtgttgagca gttttctcca 20
<210> 56
   <211> 20
   <212> DNA
   <213> Artificial Sequence
<220>
   <223> Probe of COX-2
<400> 56
   gtttggagtg ggtttcagaa 20
<210> 57
   <211> 20
   <212> DNA
   <213> Artificial Sequence
<220>
   <223> Probe of COX-2
<400> 57
   atcccttgaa gtgggtaagt 20
<210> 58
   <211> 20
   <212> DNA
   <213> Artificial Sequence
<220>
   <223> Probe of COX-2
<400> 58
   ttgcatttcg aaggaaggga 20
<210> 59
   <211> 20
   <212> DNA
   <213> Artificial Sequence
<220>
   <223> Probe of COX-2
<400> 59
   gtgatctgga tgtcaacaca 20
<210> 60
   <211> 20
   <212> DNA
   <213> Artificial sequence
<220>
   <223> Probe of COX-2
<400> 60
   tgtaagttgg tggactgtca 20
<210> 61
   <211> 20
   <212> DNA
   <213> Artificial Sequence
<220>
   <223> Probe of COX-2
<400> 61
   cttcccagct tttgtagcca 20
<210> 62
   <211> 20
   <212> DNA
   <213> Artificial Sequence
<220>
   <223> Probe of COX-2
<400> 62
   gaggaagggc tctagtataa 20
<210> 63
   <211> 20
   <212> DNA
   <213> Artificial sequence
<220>
   <223> Probe of COX-2
<400> 63
   caggaagctg ctttttacct 20
<210> 64
   <211> 20
   <212> DNA
   <213> Artificial sequence
<220>
   <223> Probe of COX-2
<400> 64
   gcaatttttc cacaatctca 20
<210> 65
   <211> 20
   <212> DNA
   <213> Artificial Sequence
<220>
   <223> Probe of COX-2
<400> 65
   tcagggatga actttcttct 20
<210> 66
   <211> 20
   <212> DNA
   <213> Artificial Sequence
<220>
   <223> Probe of COX-2
<400> 66
   agtgctgggc aaagaatgca 20
<210> 67
   <211> 20
   <212> DNA
   <213> Artificial sequence
<220>
   <223> Probe of COX-2
<400> 67
   cgcttatgat ctgtcttgaa 20
<210> 68
   <211> 20
   <212> DNA
   <213> Artificial Sequence
<220>
   <223> Probe of COX-2
<400> 68
   atggcccagc ccgttggtga 20
<210> 69
   <211> 20
   <212> DNA
   <213> Artificial sequence
<220>
   <223> Probe of COX-2
<400> 69
   gccagagttt caccgtaaat 20
<210> 70
   <211> 20
   <212> DNA
   <213> Artificial Sequence
<220>
   <223> Probe of COX-2
<400> 70
   atccttgaaa aggcgcagtt 20
<210> 71
   <211> 20
   <212> DNA
   <213> Artificial Sequence
<220>
   <223> Probe for COX-2
<400> 71
   gaggatacat ctctccatca 20
<210> 72
   <211> 20
   <212> DNA
   <213> Artificial sequence
<220>
   <223> Probe for COX-2
<400> 72
   tcatctctgc ctgagtatct 20
<210> 73
   <211> 20
   <212> DNA
   <213> Artificial Sequence
<220>
   <223> Probe for COX-2
<400> 73
   tcagaccagg caccagacca 20
<210> 74
   <211> 20
   <212> DNA
   <213> Artificial Sequence
<220>
   <223> Probe for COX-2
<400> 74
   gttgtgttcc cgcagccaga 20
<210> 75
   <211> 20
   <212> DNA
   <213> Artificial Sequence
<220>
   <223> Probe for COX-2
<400> 75
   tcctgtttaa gcacatcgca 20
<210> 76
   <211> 20
   <212> DNA
   <213> Artificial Sequence
<220>
   <223> Probe for COX-2
<400> 76
   aacaactgct catcacccca 20
<210> 77
   <211> 20
   <212> DNA
   <213> Artificial Sequence
<220>
   <223> Probe for COX-2
<400> 77
   atagtctctc ctatcagtat 20
<210> 78
   <211> 20
   <212> DNA
   <213> Artificial sequence
<220>
   <223> Probe for COX-2
<400> 78
   ccactcaagt gttgcacata 20
<210> 79
   <211> 20
   <212> DNA
   <213> Artificial Sequence
<220>
   <223> Probe for COX-2
<400> 79
   gttctgggtc aaatttcagt 20
<210> 80
   <211> 20
   <212> DNA
   <213> Artificial Sequence
<220>
   <223> Probe for COX-2
<400> 80
   gtactggaat tgtttgttga 20
<210> 81
   <211> 20
   <212> DNA
   <213> Artificial Sequence
<220>
   <223> Probe for COX-2
<400> 81
   gggtgttaaa ttcagcagca 20
<210> 82
   <211> 20
   <212> DNA
   <213> Artificial Sequence
<220>
   <223> Probe for COX-2
<400> 82
   gttgtatttc tggtcatgaa 20
<210> 83
   <211> 20
   <212> DNA
   <213> Artificial Sequence
<220>
   <223> Probe for COX-2
<400> 83
   ggtaattcca tgttccagca 20
<210> 84
   <211> 20
   <212> DNA
   <213> Artificial Sequence
<220>
   <223> Probe for COX-2
<400> 84
   gcctggtgaa tgattcaaca 20
<210> 85
   <211> 20
   <212> DNA
   <213> Artificial sequence
<220>
   <223> Probe for COX-2
<400> 85
   caatggaagc ctgtgatact 20
<210> 86
   <211> 20
   <212> DNA
   <213> Artificial sequence
<220>
   <223> Probe for COX-2
<400> 86
   gcgtttgcgg tactcattaa 20
<210> 87
   <211> 20
   <212> DNA
   <213> Artificial sequence
<220>
   <223> Probe for COX-2
<400> 87
   cctgtaagtt cttcaaatga 20
<210> 88
   <211> 20
   <212> DNA
   <213> Artificial Sequence
<220>
   <223> Probe for COX-2
<400> 88
   caactctgca gacatttcct 20
<210> 89
   <211> 20
   <212> DNA
   <213> Artificial Sequence
<220>
   <223> Probe for COX-2
<400> 89
   ccacagcatc gatgtcacca 20
<210> 90
   <211> 20
   <212> DNA
   <213> Artificial Sequence
<220>
   <223> Probe for COX-2
<400> 90
   tttctaccag aagggcagga 20
<210> 91
   <211> 20
   <212> DNA
   <213> Artificial Sequence
<220>
   <223> Probe for COX-2
<400> 91
   cttctaccat ggtttcacca 20
<210> 92
   <211> 20
   <212> DNA
   <213> Artificial Sequence
<220>
   <223> Probe for COX-2
<400> 92
   cataagtcct ttcaaggaga 20
<210> 93
   <211> 20
   <212> DNA
   <213> Artificial sequence
<220>
   <223> Probe for COX-2
<400> 93
   cagtaggcag gagaacatat 20
<210> 94
   <211> 20
   <212> DNA
   <213> Artificial sequence
<220>
   <223> Probe for COX-2
<400> 94
   aaaacccact tctccaccaa 20
<210> 95
   <211> 20
   <212> DNA
   <213> Artificial Sequence
<220>
   <223> Probe for COX-2
<400> 95
   attgcagatg agagactgaa 20
<210> 96
   <211> 20
   <212> DNA
   <213> Artificial Sequence
<220>
   <223> Probe for COX-2
<400> 96
   ctggaacact gaatgaagta 20
<210> 97
   <211> 20
   <212> DNA
   <213> Artificial Sequence
<220>
   <223> Probe for COX-2
<400> 97
   cttgcattga tggtgactgt 20
<210> 98
   <211> 20
   <212> DNA
   <213> Artificial sequence
<220>
   <223> Probe for COX-2
<400> 98
   cagttcagtc gaacgttctt 20
<210> 99
   <211> 958
   <212> DNA
   <213> Artificial Sequence
<220>
   <223> Probe for d2EGFP
<400> 99
   gcggatggtg agcaagggcg aggagctgtt caccggggtg gtgcccatcc tggtcgagct 60
   ggacggcgac gtaaacggcc acaagttcag cgtgtccggc gagggcgagg gcgatgccac 120
   ctacggcaag ctgaccctga agttcatctg caccaccggc aagctgcccg tgccctggcc 180
   caccctcgtg accaccctga cctacggcgt gcagtgcttc agccgctacc ccgaccacat 240
   gaagcagcac gacttcttca agtccgccat gcccgaaggc tacgtccagg agcgcaccat 300
   cttcttcaag gacgacggca actacaagac ccgcgccgag gtgaagttcg agggcgacac 360
   cctggtgaac cgcatcgagc tgaagggcat cgacttcaag gaggacggca acatcctggg 420
   gcacaagctg gagtacaact acaacagcca caacgtctat atcatggccg acaagcagaa 480
   gaacggcatc aaggtgaact tcaagatccg ccacaacatc gaggacggca gcgtgcagct 540
   cgccgaccac taccagcaga acacccccat cggcgacggc cccgtgctgc tgcccgacaa 600
   ccactacctg agcacccagt ccgccctgag caaagacccc aacgagaagc gcgatcacat 660
   ggtcctgctg gagttcgtga ccgccgccgg gatcactctc ggcatggacg agctgtacaa 720
   gaagcttagc catggcttcc cgccggaggt ggaggagcag gatgatggca cgctgcccat 780
   gtcttgtgcc caggagagcg ggatggaccg tcaccctgca gcctgtgctt ctgctaggat 840
   caatgtgtag gaattcgtga catgataaga tacattgatg agtttggaca aaccacaact 900
   agaatgcagt gaaaaaaatg ctttatttgt gaaatttgtg atgctattgc tttatttg 958
<210> 100
   <211> 17
   <212> DNA
   <213> Artificial sequence
<220>
   <223> Probe of d2EGFP
<400> 100
   cgcccttgct caccatc 17
<210> 101
   <211> 17
   <212> DNA
   <213> Artificial sequence
<220>
   <223> Probe of d2EGFP
<400> 101
   caccccggtg aacagct 17
<210> 102
   <211> 17
   <212> DNA
   <213> Artificial sequence
<220>
   <223> Probe of d2EGFP
<400> 102
   agctcgacca ggatggg 17
<210> 103
   <211> 17
   <212> DNA
   <213> Artificial Sequence
<220>
   <223> Probe of d2EGFP
<400> 103
   ggccgtttac gtcgccg 17
<210> 104
   <211> 17
   <212> DNA
   <213> Artificial sequence
<220>
   <223> Probe of d2EGFP
<400> 104
   gccggacacg ctgaact 17
<210> 105
   <211> 17
   <212> DNA
   <213> Artificial sequence
<220>
   <223> Probe of d2EGFP
<400> 105
   gtggcatcgc cctcgcc 17
<210> 106
   <211> 17
   <212> DNA
   <213> Artificial sequence
<220>
   <223> Probe of d2EGFP
<400> 106
   tcagggtcag cttgccg 17
<210> 107
   <211> 17
   <212> DNA
   <213> Artificial sequence
<220>
   <223> Probe of d2EGFP
<400> 107
   gccggtggtg cagatga 17
<210> 108
   <211> 17
   <212> DNA
   <213> Artificial Sequence
<220>
   <223> Probe of d2EGFP
<400> 108
   ggccagggca cgggcag 17
<210> 109
   <211> 17
   <212> DNA
   <213> Artificial Sequence
<220>
   <223> Probe of d2EGFP
<400> 109
   tcagggtggt cacgagg 17
<210> 110
   <211> 17
   <212> DNA
   <213> Artificial Sequence
<220>
   <223> Probe of d2EGFP
<400> 110
   gaagcactgc acgccgt 17
<210> 111
   <211> 17
   <212> DNA
   <213> Artificial sequence
<220>
   <223> Probe of d2EGFP
<400> 111
   atgtggtcgg ggtagcg 17
<210> 112
   <211> 17
   <212> DNA
   <213> Artificial Sequence
<220>
   <223> Probe of d2EGFP
<400> 112
   tgaagaagtc gtgctgc 17
<210> 113
   <211> 17
   <212> DNA
   <213> Artificial Sequence
<220>
   <223> Probe of d2EGFP
<400> 113
   gccttcgggc atggcgg 17
<210> 114
   <211> 17
   <212> DNA
   <213> Artificial sequence
<220>
   <223> Probe of d2EGFP
<400> 114
   atggtgcgct cctggac 17
<210> 115
   <211> 17
   <212> DNA
   <213> Artificial Sequence
<220>
   <223> Probe of d2EGFP
<400> 115
   tgccgtcgtc cttgaag 17
<210> 116
   <211> 17
   <212> DNA
   <213> Artificial sequence
<220>
   <223> Probe of d2EGFP
<400> 116
   ctcggcgcgg gtcttgt 17
<210> 117
   <211> 17
   <212> DNA
   <213> Artificial sequence
<220>
   <223> Probe of d2EGFP
<400> 117
   gtgtcgccct cgaactt 17
<210> 118
   <211> 17
   <212> DNA
   <213> Artificial sequence
<220>
   <223> Probe of d2EGFP
<400> 118
   gctcgatgcg gttcacc 17
<210> 119
   <211> 17
   <212> DNA
   <213> Artificial sequence
<220>
   <223> Probe of d2EGFP
<400> 119
   cttgaagtcg atgccct 17
<210> 120
   <211> 17
   <212> DNA
   <213> Artificial Sequence
<220>
   <223> Probe of d2EGFP
<400> 120
   cccaggatgt tgccgtc 17
<210> 121
   <211> 17
   <212> DNA
   <213> Artificial sequence
<220>
   <223> Probe of d2EGFP
<400> 121
   agttgtactc cagcttg 17
<210> 122
   <211> 17
   <212> DNA
   <213> Artificial Sequence
<220>
   <223> Probe of d2EGFP
<400> 122
   atagacgttg tggctgt 17
<210> 123
   <211> 17
   <212> DNA
   <213> Artificial Sequence
<220>
   <223> Probe of d2EGFP
<400> 123
   ttctgcttgt cggccat 17
<210> 124
   <211> 17
   <212> DNA
   <213> Artificial Sequence
<220>
   <223> Probe of d2EGFP
<400> 124
   agttcacctt gatgccg 17
<210> 125
   <211> 17
   <212> DNA
   <213> Artificial sequence
<220>
   <223> Probe of d2EGFP
<400> 125
   gatgttgtgg cggatct 17
<210> 126
   <211> 17
   <212> DNA
   <213> Artificial Sequence
<220>
   <223> Probe of d2EGFP
<400> 126
   agctgcacgc tgccgtc 17
<210> 127
   <211> 17
   <212> DNA
   <213> Artificial Sequence
<220>
   <223> Probe of d2EGFP
<400> 127
   tctgctggta gtggtcg 17
<210> 128
   <211> 17
   <212> DNA
   <213> Artificial Sequence
<220>
   <223> Probe of d2EGFP
<400> 128
   gccgtcgccg atggggg 17
<210> 129
   <211> 17
   <212> DNA
   <213> Artificial sequence
<220>
   <223> Probe of d2EGFP
<400> 129
   ttgtcgggca gcagcac 17
<210> 130
   <211> 17
   <212> DNA
   <213> Artificial sequence
<220>
   <223> Probe of d2EGFP
<400> 130
   actgggtgct caggtag 17
<210> 131
   <211> 17
   <212> DNA
   <213> Artificial sequence
<220>
   <223> Probe of d2EGFP
<400> 131
   ggggtctttg ctcaggg 17
<210> 132
   <211> 17
   <212> DNA
   <213> Artificial Sequence
<220>
   <223> Probe of d2EGFP
<400> 132
   atgtgatcgc gcttctc 17
<210> 133
   <211> 17
   <212> DNA
   <213> Artificial Sequence
<220>
   <223> Probe of d2EGFP
<400> 133
   tcacgaactc cagcagg 17
<210> 134
   <211> 17
   <212> DNA
   <213> Artificial sequence
<220>
   <223> Probe of d2EGFP
<400> 134
   gagagtgatc ccggcgg 17
<210> 135
   <211> 17
   <212> DNA
   <213> Artificial Sequence
<220>
   <223> Probe of d2EGFP
<400> 135
   ttgtacagct cgtccat 17
<210> 136
   <211> 17
   <212> DNA
   <213> Artificial Sequence
<220>
   <223> Probe of d2EGFP
<400> 136
   ggaagccatg gctaagc 17
<210> 137
   <211> 17
   <212> DNA
   <213> Artificial Sequence
<220>
   <223> Probe of d2EGFP
<400> 137
   ctgctcctcc acctccg 17
<210> 138
   <211> 17
   <212> DNA
   <213> Artificial Sequence
<220>
   <223> Probe of d2EGFP
<400> 138
   atgggcagcg tgccatc 17
<210> 139
   <211> 17
   <212> DNA
   <213> Artificial Sequence
<220>
   <223> Probe of d2EGFP
<400> 139
   cgctctcctg ggcacaa 17
<210> 140
   <211> 17
   <212> DNA
   <213> Artificial Sequence
<220>
   <223> Probe of d2EGFP
<400> 140
   tgcagggtga cggtcca 17
<210> 141
   <211> 17
   <212> DNA
   <213> Artificial Sequence
<220>
   <223> Probe of d2EGFP
<400> 141
   atcctagcag aagcaca 17
<210> 142
   <211> 17
   <212> DNA
   <213> Artificial Sequence
<220>
   <223> Probe of d2EGFP
<400> 142
   acgaattcct acacatt 17
<210> 143
   <211> 17
   <212> DNA
   <213> Artificial Sequence
<220>
   <223> Probe of d2EGFP
<400> 143
   tcaatgtatc ttatcat 17
<210> 144
   <211> 17
   <212> DNA
   <213> Artificial sequence
<220>
   <223> Probe of d2EGFP
<400> 144
   ttgtggtttg tccaaac 17
<210> 145
   <211> 17
   <212> DNA
   <213> Artificial Sequence
<220>
   <223> Probe of d2EGFP
<400> 145
   tttttttcac tgcattc 17
<210> 146
   <211> 17
   <212> DNA
   <213> Artificial Sequence
<220>
   <223> Probe of d2EGFP
<400> 146
   caaatttcac aaataaa 17
<210> 147
   <211> 17
   <212> DNA
   <213> Artificial sequence
<220>
   <223> Probe of d2EGFP
<400> 147
   caaataaagc aatagca 17
<210> 148
   <211> 1767
   <212> DNA
   <213> Artificial Sequence
<220>
   <223> Probe for dpp
<400> 148
   atgcgcgcat ggcttctact cctcgcagtg ctggcgactt ttcaaacgat tgttcgagtt      60
   gctagcaccg aggatatatc ccagagattc atcgccgcca tagcgcccgt tgccgctcat     120
   attccgctgg catcagcatc aggatcagga tcaggacgat ctggatctag atcggtagga     180
   gcctcgacca gcacagcatt agcaaaagca tttaatccat tcagcgagcc cgcctcgttc     240
   agtgatagtg ataaaagcca tcggagtaaa acaaacaaaa aacctagcaa aagtgacgcg     300
   aaccgacagt tcaacgaagt gcataagcca agaacagacc aattagaaaa ttccaaaaat     360
   aagtctaaac aattagttaa taaacccaac cacaacaaaa tggctgtcaa ggagcagagg     420
   agccaccaca agaagagcca ccaccatcgc agccaccagc caaagcaggc cagtgcatcc     480
   acagaatctc atcaatcctc gtcgattgaa tcaatcttcg tggaggagcc gacgctggtg     540
   ctcgaccgcg aggtggcctc catcaacgtg cccgccaacg ccaaggccat catcgccgag     600
   cagggcccgt ccacctacag caaggaggcg ctcatcaagg acaagctgaa gccagacccc     660
   tccactctag tcgagatcga gaagagcctg ctctcgctgt tcaacatgaa gcggccgccc     720
   aagatcgacc gctccaagat catcatcccc gagccgatga agaagctcta cgccgagatc     780
   atgggccacg agctcgactc ggtcaacatc cccaagccgg gtctgctgac caagtcggcc     840
   aacacagtgc gaagttttac acacaaagat agtaaaatcg acgatcgatt tccgcaccac     900
   caccggtttc ggctgcactt cgacgtgaag agcattcccg ccgacgagaa gctgaaggcg     960
   gcggagctgc agctgacccg ggacgcactc agtcaacagg tggtggccag cagatcgtcg    1020
   gcgaatcgga cgcgctacca ggtgcttgtc tacgacatca cgcgcgtcgg ggtgcgtggt    1080
   cagcgggagc cgagctatct gctgttggac accaagacgg tccggcttaa cagcacggac    1140
   acggtgagcc tcgatgtcca gccggccgtg gaccggtggc tggcgagtcc gcagcgcaac    1200
   tacggactgc tggtggaggt gcggacggtc cgctccctga agccggcccc acaccaccat    1260
   gtacgcctgc gccgcagcgc ggacgaggcg cacgagcggt ggcagcacaa gcagccgctc    1320
   ctgttcacct acacggacga cgggcggcac aaggcgcgct ccattcggga cgtgtctggc    1380
   ggagagggcg gtggcaaggg cggccggaac aagcggcagc cgagacggcc tacgaggcgc    1440
   aagaaccacg acgacacctg ccggcggcac tcgctgtacg tggacttctc ggacgtgggc    1500
   tgggacgact ggattgtggc gcctctgggc tacgatgcat attactgcca cgggaagtgc    1560
   cccttcccgc tggccgacca ctttaactcg accaatcacg ccgtggtgca gaccctggtc    1620
   aacaatatga atcccggcaa ggtgccgaag gcgtgctgcg tgcccacgca actggacagc    1680
   gtggccatgc tctatctcaa cgaccaaagt acggtggtgc tgaagaacta ccaggagatg    1740
   accgtggtgg gctgtggctg tcgatag                                        1767
<210> 149
   <211> 20
   <212> DNA
   <213> Artificial Sequence
<220>
   <223> Probe for dpp
<400> 149
   ggagtagaag ccatgcgcgc 20
<210> 150
   <211> 20
   <212> DNA
   <213> Artificial Sequence
<220>
   <223> Probe for dpp
<400> 150
   gtttgaaaag tcgccagcac 20
<210> 151
   <211> 20
   <212> DNA
   <213> Artificial Sequence
<220>
   <223> Probe for dpp
<400> 151
   cctcggtgct agcaactcga 20
<210> 152
   <211> 20
   <212> DNA
   <213> Artificial Sequence
<220>
   <223> Probe for dpp
<400> 152
   cgggcgctat ggcggcgatg 20
<210> 153
   <211> 19
   <212> DNA
   <213> Artificial Sequence
<220>
   <223> Probe for dpp
<400> 153
   ctgatgctga tgccagcgg 19
<210> 154
   <211> 20
   <212> DNA
   <213> Artificial Sequence
<220>
   <223> Probe for dpp
<400> 154
   gctggtcgag gctcctaccg 20
<210> 155
   <211> 20
   <212> DNA
   <213> Artificial Sequence
<220>
   <223> Probe for dpp
<400> 155
   tgaacgaggc gggctcgctg 20
<210> 156
   <211> 20
   <212> DNA
   <213> Artificial Sequence
<220>
   <223> Probe for dpp
<400> 156
   ctccgatggc ttttatcact 20
<210> 157
   <211> 20
   <212> DNA
   <213> Artificial Sequence
<220>
   <223> Probe for dpp
<400> 157
   cgttgaactg tcggttcgcg 20
<210> 158
   <211> 20
   <212> DNA
   <213> Artificial Sequence
<220>
   <223> Probe for dpp
<400> 158
   ggtggctcct ctgctccttg 20
<210> 159
   <211> 20
   <212> DNA
   <213> Artificial sequence
<220>
   <223> Probe for dpp
<400> 159
   ggctgcgatg gtggtggctc 20
<210> 160
   <211> 20
   <212> DNA
   <213> Artificial Sequence
<220>
   <223> Probe for dpp
<400> 160
   ctgtggatgc actggcctgc 20
<210> 161
   <211> 20
   <212> DNA
   <213> Artificial Sequence
<220>
   <223> Probe for dpp
<400> 161
   ccagcgtcgg ctcctccacg 20
<210> 162
   <211> 20
   <212> DNA
   <213> Artificial sequence
<220>
   <223> Probe for dpp
<400> 162
   ccttggcgtt ggcgggcacg 20
<210> 163
   <211> 20
   <212> DNA
   <213> Artificial Sequence
<220>
   <223> Probe for dpp
<400> 163
   ggtggacggg ccctgctcgg 20
<210> 164
   <211> 20
   <212> DNA
   <213> Artificial Sequence
<220>
   <223> Probe for dpp
<400> 164
   ggaggggtct ggcttcagct 20
<210> 165
   <211> 20
   <212> DNA
   <213> Artificial Sequence
<220>
   <223> Probe for dpp
<400> 165
   agcgagagca ggctcttctc 20
<210> 166
   <211> 20
   <212> DNA
   <213> Artificial sequence
<220>
   <223> Probe for dpp
<400> 166
   gagcggtcga tcttgggcgg 20
<210> 167
   <211> 20
   <212> DNA
   <213> Artificial Sequence
<220>
   <223> Probe for dpp
<400> 167
   cttcatcggc tcggggatga 20
<210> 168
   <211> 20
   <212> DNA
   <213> Artificial Sequence
<220>
   <223> Probe for dpp
<400> 168
   tcgtggccca tgatctcggc 20
<210> 169
   <211> 20
   <212> DNA
   <213> Artificial Sequence
<220>
   <223> Probe for dpp
<400> 169
   gcagacccgg cttggggatg 20
<210> 170
   <211> 20
   <212> DNA
   <213> Artificial Sequence
<220>
   <223> Probe for dpp
<400> 170
   cgcactgtgt tggccgactt 20
<210> 171
   <211> 20
   <212> DNA
   <213> Artificial Sequence
<220>
   <223> Probe for dpp
<400> 171
   gcagccgaaa ccggtggtgg 20
<210> 172
   <211> 20
   <212> DNA
   <213> Artificial Sequence
<220>
   <223> Probe for dpp
<400> 172
   ccgccgcctt cagcttctcg 20
<210> 173
   <211> 20
   <212> DNA
   <213> Artificial sequence
<220>
   <223> Probe for dpp
<400> 173
   agtgcgtccc gggtcagctg 20
<210> 174
   <211> 20
   <212> DNA
   <213> Artificial sequence
<220>
   <223> Probe for dpp
<400> 174
   ccgacgatct gctggccacc 20
<210> 175
   <211> 20
   <212> DNA
   <213> Artificial Sequence
<220>
   <223> Probe for dpp
<400> 175
   agcacctggt agcgcgtccg 20
<210> 176
   <211> 20
   <212> DNA
   <213> Artificial Sequence
<220>
   <223> Probe for dpp
<400> 176
   cacgcacccc gacgcgcgtg 20
<210> 177
   <211> 20
   <212> DNA
   <213> Artificial sequence
<220>
   <223> Probe for dpp
<400> 177
   gccggaccgt cttggtgtcc 20
<210> 178
   <211> 20
   <212> DNA
   <213> Artificial Sequence
<220>
   <223> Probe for dpp
<400> 178
   gaggctcacc gtgtccgtgc 20
<210> 179
   <211> 20
   <212> DNA
   <213> Artificial sequence
<220>
   <223> Probe for dpp
<400> 179
   gccagccacc ggtccacggc 20
<210> 180
   <211> 20
   <212> DNA
   <213> Artificial Sequence
<220>
   <223> Probe for dpp
<400> 180
   cagcagtccg tagttgcgct 20
<210> 181
   <211> 20
   <212> DNA
   <213> Artificial Sequence
<220>
   <223> Probe for dpp
<400> 181
   gggagcggac cgtccgcacc 20
<210> 182
   <211> 20
   <212> DNA
   <213> Artificial Sequence
<220>
   <223> Probe for dpp
<400> 182
   caggcgtaca tggtggtgtg 20
<210> 183
   <211> 20
   <212> DNA
   <213> Artificial sequence
<220>
   <223> Probe for dpp
<400> 183
   cgtgcgcctc gtccgcgctg 20
<210> 184
   <211> 20
   <212> DNA
   <213> Artificial Sequence
<220>
   <223> Probe for dpp
<400> 184
   gagcggctgc ttgtgctgcc 20
<210> 185
   <211> 20
   <212> DNA
   <213> Artificial Sequence
<220>
   <223> Probe for dpp
<400> 185
   ccgcccgtcg tccgtgtagg 20
<210> 186
   <211> 20
   <212> DNA
   <213> Artificial Sequence
<220>
   <223> Probe for dpp
<400> 186
   gccaccgccc tctccgccag 20
<210> 187
   <211> 20
   <212> DNA
   <213> Artificial sequence
<220>
   <223> Probe for dpp
<400> 187
   cgtaggccgt ctcggctgcc 20
<210> 188
   <211> 20
   <212> DNA
   <213> Artificial Sequence
<220>
   <223> Probe for dpp
<400> 188
   gcgagtgccg ccggcaggtg 20
<210> 189
   <211> 20
   <212> DNA
   <213> Artificial sequence
<220>
   <223> Probe for dpp
<400> 189
   cgtcccagcc cacgtccgag 20
<210> 190
   <211> 20
   <212> DNA
   <213> Artificial Sequence
<220>
   <223> Probe for dpp
<400> 190
   cgtagcccag aggcgccaca 20
<210> 191
   <211> 20
   <212> DNA
   <213> Artificial Sequence
<220>
   <223> Probe for dpp
<400> 191
   cggccagcgg gaaggggcac 20
<210> 192
   <211> 20
   <212> DNA
   <213> Artificial Sequence
<220>
   <223> Probe for dpp
<400> 192
   gggtctgcac cacggcgtga 20
<210> 193
   <211> 20
   <212> DNA
   <213> Artificial Sequence
<220>
   <223> Probe for dpp
<400> 193
   gccttcggca ccttgccggg 20
<210> 194
   <211> 20
   <212> DNA
   <213> Artificial Sequence
<220>
   <223> Probe for dpp
<400> 194
   gtccagttgc gtgggcacgc 20
<210> 195
   <211> 20
   <212> DNA
   <213> Artificial sequence
<220>
   <223> Probe for dpp
<400> 195
   gcaccaccgt actttggtcg 20
<210> 196
   <211> 20
   <212> DNA
   <213> Artificial Sequence
<220>
   <223> Probe for dpp
<400> 196
   gccacagccc accacggtca 20
<210> 197
   <211> 1302
   <212> DNA
   <213> Artificial Sequence
<220>
   <223> Probe for elt-2
<400> 197
   atggataata actacaatga taatgtcaac ggctgggccg aaatggaacc atctcaacca 60
   atgggaggtc tgcgcctacc aactcagaac atggatccac cagagcaaaa taatgagtca 120
   caattgagtg aactaccgag aatgaaaatt gataatgatt acgcatctcc aattgaacgg 180
   caaagtgtta tcacaagtgg cacaaataac tatgagccga aagtggaaac tgttacatca 240
   tttttccata ctggcataga ctactcaaac tttggaatgt tggaccaaac taccatgcaa 300
   ccgttttatc ctctttacag tggaattccc gtaaacactc ttggaacttt ttcgggatat 360
   acaaactcca tatacgacaa accctctctg tacgacccca gtattcctac cattaacatc 420
   ccttctactt atccaactgt ggctccaact tacgaatgcg tcaaatgctc acaaagttgt 480
   ggggccggga tgaaggcagt aaacggagga atgatgtgcg tcaactgttc aacaccaaaa 540
   accacgtatt ctcctccagt cgcgtatagc acttctttgg gacaaccccc gattctggaa 600
   ataccttcag agcagccaac tgctaaaatt gccaagcaat cctctaaaaa gtcaagtagc 660
   tcaaataggg ggtcaaacgg atctgcgtcc cgtcggcagg gacttgtgtg ctccaattgc 720
   aatggtacca acacaactct ctggagaaga aatgctgaag gagatccggt ctgcaatgct 780
   tgcgggcttt acttcaaact ccatcacatc cctcggccga cctcaatgaa gaaagaaggt 840
   gctttacaga caagaaagag aaaatcaaaa agcggagact cttccacacc atcaacgtca 900
   cgggcccgag aaaggaagtt tgagagagcc tcttcttcga ccgaaaaggc tcaaaggtca 960
   tctaaccggc gtgcgggaag tgcaaaagca gaccgagaac tgagcactgc tgccgtcgca 1020
   gctgcgactg ccacatatgt gtcacatgcc gacttgtatc ccgtttcctc agctgccgtc 1080
   accttgccag atcaaacgta cagtaattac tatcaatgga acactgccgc tacagctggg 1140
   ttgatgatgg ttccaaacga tcaaaactac gtgtatgcag caacaaacta ccagactggc 1200
   ctaagacctg ccgataacat ccaagttcat gtgatgccag ttcaggatga tgaaaccaaa 1260
   gctgcggctc gcgatttgga agcggtcgac ggagattctt aa 1302
<210> 198
   <211> 20
   <212> DNA
   <213> Artificial Sequence
<220>
   <223> Probe for elt-2
<400> 198
   ccatttcggc ccagccgttg 20
<210> 199
   <211> 20
   <212> DNA
   <213> Artificial sequence
<220>
   <223> Probe for elt-2
<400> 199
   gacctcccat tggttgagat 20
<210> 200
   <211> 20
   <212> DNA
   <213> Artificial sequence
<220>
   <223> Probe for elt-2
<400> 200
   ccatgttctg agttggtagg 20
<210> 201
   <211> 20
   <212> DNA
   <213> Artificial Sequence
<220>
   <223> Probe for elt-2
<400> 201
   actcattatt ttgctctggt 20
<210> 202
   <211> 20
   <212> DNA
   <213> Artificial sequence
<220>
   <223> Probe for elt-2
<400> 202
   ttctcggtag ttcactcaat 20
<210> 203
   <211> 20
   <212> DNA
   <213> Artificial Sequence
<220>
   <223> Probe for elt-2
<400> 203
   taacactttg ccgttcaatt 20
<210> 204
   <211> 20
   <212> DNA
   <213> Artificial sequence
<220>
   <223> Probe for elt-2
<400> 204
   catagttatt tgtgccactt 20
<210> 205
   <211> 20
   <212> DNA
   <213> Artificial Sequence
<220>
   <223> Probe for elt-2
<400> 205
   atgtaacagt ttccactttc 20
<210> 206
   <211> 20
   <212> DNA
   <213> Artificial Sequence
<220>
   <223> Probe for elt-2
<400> 206
   agtctatgcc agtatggaaa 20
<210> 207
   <211> 20
   <212> DNA
   <213> Artificial Sequence
<220>
   <223> Probe for elt-2
<400> 207
   ggtccaacat tccaaagttt 20
<210> 208
   <211> 20
   <212> DNA
   <213> Artificial Sequence
<220>
   <223> Probe for elt-2
<400> 208
   gataaaacgg ttgcatggta 20
<210> 209
   <211> 20
   <212> DNA
   <213> Artificial Sequence
<220>
   <223> Probe for elt-2
<400> 209
   ttacgggaat tccactgtaa 20
<210> 210
   <211> 20
   <212> DNA
   <213> Artificial Sequence
<220>
   <223> Probe for elt-2
<400> 210
   atcccgaaaa agttccaaga 20
<210> 211
   <211> 20
   <212> DNA
   <213> Artificial Sequence
<220>
   <223> Probe for elt-2
<400> 211
   gtttgtcgta tatggagttt 20
<210> 212
   <211> 20
   <212> DNA
   <213> Artificial Sequence
<220>
   <223> Probe for elt-2
<400> 212
   gaatactggg gtcgtacaga 20
<210> 213
   <211> 20
   <212> DNA
   <213> Artificial Sequence
<220>
   <223> Probe for elt-2
<400> 213
   aagtagaagg gatgttaatg 20
<210> 214
   <211> 20
   <212> DNA
   <213> Artificial Sequence
<220>
   <223> Probe for elt-2
<400> 214
   cgtaagttgg agccacagtt 20
<210> 215
   <211> 20
   <212> DNA
   <213> Artificial sequence
<220>
   <223> Probe for elt-2
<400> 215
   aactttgtga gcatttgacg 20
<210> 216
   <211> 20
   <212> DNA
   <213> Artificial sequence
<220>
   <223> Probe for elt-2
<400> 216
   ttactgcctt catcccggcc 20
<210> 217
   <211> 20
   <212> DNA
   <213> Artificial sequence
<220>
   <223> Probe for elt-2
<400> 217
   agttgacgca catcattcct 20
<210> 218
   <211> 20
   <212> DNA
   <213> Artificial Sequence
<220>
   <223> Probe for elt-2
<400> 218
   aatacgtggt ttttggtgtt 20
<210> 219
   <211> 20
   <212> DNA
   <213> Artificial sequence
<220>
   <223> Probe for elt-2
<400> 219
   aagtgctata cgcgactgga 20
<210> 220
   <211> 20
   <212> DNA
   <213> Artificial sequence
<220>
   <223> Probe for elt-2
<400> 220
   ccagaatcgg gggttgtccc 20
<210> 221
   <211> 20
   <212> DNA
   <213> Artificial Sequence
<220>
   <223> Probe for elt-2
<400> 221
   cagttggctg ctctgaaggt 20
<210> 222
   <211> 20
   <212> DNA
   <213> Artificial sequence
<220>
   <223> Probe for elt-2
<400> 222
   tagaggattg cttggcaatt 20
<210> 223
   <211> 20
   <212> DNA
   <213> Artificial sequence
<220>
   <223> Probe for elt-2
<400> 223
   ccctatttga gctacttgac 20
<210> 224
   <211> 20
   <212> DNA
   <213> Artificial Sequence
<220>
   <223> Probe for elt-2
<400> 224
   gacgggacgc agatccgttt 20
<210> 225
   <211> 20
   <212> DNA
   <213> Artificial Sequence
<220>
   <223> Probe for elt-2
<400> 225
   aattggagca cacaagtccc 20
<210> 226
   <211> 20
   <212> DNA
   <213> Artificial sequence
<220>
   <223> Probe for elt-2
<400> 226
   agagagttgt gttggtacca 20
<210> 227
   <211> 20
   <212> DNA
   <213> Artificial sequence
<220>
   <223> Probe for elt-2
<400> 227
   gatctccttc agcatttctt 20
<210> 228
   <211> 20
   <212> DNA
   <213> Artificial Sequence
<220>
   <223> Probe for elt-2
<400> 228
   aaagcccgca agcattgcag 20
<210> 229
   <211> 20
   <212> DNA
   <213> Artificial sequence
<220>
   <223> Probe for elt-2
<400> 229
   gagggatgtg atggagtttg 20
<210> 230
   <211> 20
   <212> DNA
   <213> Artificial sequence
<220>
   <223> Probe for elt-2
<400> 230
   cttctttctt cattgaggtc 20
<210> 231
   <211> 20
   <212> DNA
   <213> Artificial Sequence
<220>
   <223> Probe for elt-2
<400> 231
   tggaagagtc tccgcttttt 20
<210> 232
   <211> 20
   <212> DNA
   <213> Artificial Sequence
<220>
   <223> Probe for elt-2
<400> 232
   ctcgggcccg tgacgttgat 20
<210> 233
   <211> 20
   <212> DNA
   <213> Artificial sequence
<220>
   <223> Probe for elt-2
<400> 233
   aagaggctct ctcaaacttc 20
<210> 234
   <211> 20
   <212> DNA
   <213> Artificial sequence
<220>
   <223> Probe for elt-2
<400> 234
   acctttgagc cttttcggtc 20
<210> 235
   <211> 20
   <212> DNA
   <213> Artificial Sequence
<220>
   <223> Probe for elt-2
<400> 235
   cacttcccgc acgccggtta 20
<210> 236
   <211> 20
   <212> DNA
   <213> Artificial Sequence
<220>
   <223> Probe for elt-2
<400> 236
   tgctcagttc tcggtctgct 20
<210> 237
   <211> 20
   <212> DNA
   <213> Artificial Sequence
<220>
   <223> Probe for elt-2
<400> 237
   agtcggcatg tgacacatat 20
<210> 238
   <211> 20
   <212> DNA
   <213> Artificial Sequence
<220>
   <223> Probe for elt-2
<400> 238
   cggcagctga ggaaacggga 20
<210> 239
   <211> 20
   <212> DNA
   <213> Artificial Sequence
<220>
   <223> Probe for elt-2
<400> 239
   tgtacgtttg atctggcaag 20
<210> 240
   <211> 20
   <212> DNA
   <213> Artificial Sequence
<220>
   <223> Probe for elt-2
<400> 240
   ccatcatcaa cccagctgta 20
<210> 241
   <211> 20
   <212> DNA
   <213> Artificial Sequence
<220>
   <223> Probe for elt-2
<400> 241
   cagtctggta gtttgttgct 20
<210> 242
   <211> 20
   <212> DNA
   <213> Artificial Sequence
<220>
   <223> Probe for elt-2
<400> 242
   ggatgttatc ggcaggtctt 20
<210> 243
   <211> 20
   <212> DNA
   <213> Artificial sequence
<220>
   <223> Probe for elt-2
<400> 243
   cctgaactgg catcacatga 20
<210> 244
   <211> 20
   <212> DNA
   <213> Artificial Sequence
<220>
   <223> Probe for elt-2
<400> 244
   gagccgcagc tttggtttca 20
<210> 245
   <211> 20
   <212> DNA
   <213> Artificial Sequence
<220>
   <223> Probe for elt-2
<400> 245
   ctccgtcgac cgcttccaaa 20
<210> 246
   <211> 1374
   <212> DNA
   <213> Artificial sequence
<220>
   <223> Probe for FKBP5
<400> 246
   atgactactg atgaaggtgc caagaacaat gaagaaagcc ccacagccac tgttgctgag      60
   cagggagagg atattacctc caaaaaagac aggggagtat taaagattgt caaaagagtg     120
   gggaatggtg aggaaacgcc gatgattgga gacaaagttt atgtccatta caaaggaaaa     180
   ttgtcaaatg gaaagaagtt tgattccagt catgatagaa atgaaccatt tgtctttagt     240
   cttggcaaag gccaagtcat caaggcatgg gacattgggg tggctaccat gaagaaagga     300
   gagatatgcc atttactgtg caaaccagaa tatgcatatg gctcggctgg cagtctccct     360
   aaaattccct cgaatgcaac tctctttttt gagattgagc tccttgattt caaaggagag     420
   gatttatttg aagatggagg cattatccgg agaaccaaac ggaaaggaga gggatattca     480
   aatccaaacg aaggagcaac agtagaaatc cacctggaag gccgctgtgg tggaaggatg     540
   tttgactgca gagatgtggc attcactgtg ggcgaaggag aagaccacga cattccaatt     600
   ggaattgaca aagctctgga gaaaatgcag cgggaagaac aatgtatttt atatcttgga     660
   ccaagatatg gttttggaga ggcagggaag cctaaatttg gcattgaacc taatgctgag     720
   cttatatatg aagttacact taagagcttc gaaaaggcca aagaatcctg ggagatggat     780
   accaaagaaa aattggagca ggctgccatt gtcaaagaga agggaaccgt atacttcaag     840
   ggaggcaaat acatgcaggc ggtgattcag tatgggaaga tagtgtcctg gttagagatg     900
   gaatatggtt tatcagaaaa ggaatcgaaa gcttctgaat catttctcct tgctgccttt     960
   ctgaacctgg ccatgtgcta cctgaagctt agagaataca ccaaagctgt tgaatgctgt    1020
   gacaaggccc ttggactgga cagtgccaat gagaaaggct tgtataggag gggtgaagcc     1080
   cagctgctca tgaacgagtt tgagtcagcc aagggtgact ttgagaaagt gctggaagta    1140
   aacccccaga ataaggctgc aagactgcag atctccatgt gccagaaaaa ggccaaggag    1200
   cacaacgagc gggaccgcag gatatacgcc aacatgttca agaagtttgc agagcaggat    1260
   gccaaggaag aggccaataa agcaatgggc aagaagactt cagaaggggt cactaatgaa    1320
   aaaggaacag acagtcaagc aatggaagaa gagaaacctg agggccacgt atga          1374
<210> 247
   <211> 17
   <212> DNA
   <213> Artificial Sequence
<220>
   <223> Probe for FKBP5
<400> 247
   gttcttggca ccttcat 17
<210> 248
   <211> 17
   <212> DNA
   <213> Artificial Sequence
<220>
   <223> Probe for FKBP5
<400> 248
   aatcatcggc gtttcct 17
<210> 249
   <211> 17
   <212> DNA
   <213> Artificial Sequence
<220>
   <223> Probe for FKBP5
<400> 249
   tggacataaa ctttgtc 17
<210> 250
   <211> 17
   <212> DNA
   <213> Artificial sequence
<220>
   <223> Probe for FKBP5
<400> 250
   ttgacaattt tcctttg 17
<210> 251
   <211> 17
   <212> DNA
   <213> Artificial sequence
<220>
   <223> Probe for FKBP5
<400> 251
   ggaatcaaac ttctttc 17
<210> 252
   <211> 17
   <212> DNA
   <213> Artificial sequence
<220>
   <223> Probe for FKBP5
<400> 252
   ggttcatttc tatcatg 17
<210> 253
   <211> 17
   <212> DNA
   <213> Artificial Sequence
<220>
   <223> Probe for FKBP5
<400> 253
   tgccaagact aaagaca 17
<210> 254
   <211> 17
   <212> DNA
   <213> Artificial Sequence
<220>
   <223> Probe for FKBP5
<400> 254
   tgccttgatg acttggc 17
<210> 255
   <211> 17
   <212> DNA
   <213> Artificial sequence
<220>
   <223> Probe for FKBP5
<400> 255
   gtagccaccc caatgtc 17
<210> 256
   <211> 17
   <212> DNA
   <213> Artificial Sequence
<220>
   <223> Probe for FKBP5
<400> 256
   atatctctcc tttcttc 17
<210> 257
   <211> 17
   <212> DNA
   <213> Artificial sequence
<220>
   <223> Probe for FKBP5
<400> 257
   tggtttgcac agtaaat 17
<210> 258
   <211> 17
   <212> DNA
   <213> Artificial sequence
<220>
   <223> Probe for FKBP5
<400> 258
   gccgagccat atgcata 17
<210> 259
   <211> 17
   <212> DNA
   <213> Artificial sequence
<220>
   <223> Probe for FKBP5
<400> 259
   gaattttagg gagactg 17
<210> 260
   <211> 17
   <212> DNA
   <213> Artificial sequence
<220>
   <223> Probe for FKBP5
<400> 260
   aaagagagtt gcattcg 17
<210> 261
   <211> 17
   <212> DNA
   <213> Artificial Sequence
<220>
   <223> Probe for FKBP5
<400> 261
   tcaaggagct caatctc 17
<210> 262
   <211> 17
   <212> DNA
   <213> Artificial Sequence
<220>
   <223> Probe for FKBP5
<400> 262
   ataaatcctc tcctttg 17
<210> 263
   <211> 17
   <212> DNA
   <213> Artificial Sequence
<220>
   <223> Probe for FKBP5
<400> 263
   gataatgcct ccatctt 17
<210> 264
   <211> 17
   <212> DNA
   <213> Artificial sequence
<220>
   <223> Probe for FKBP5
<400> 264
   cctttccgtt tggttct 17
<210> 265
   <211> 17
   <212> DNA
   <213> Artificial Sequence
<220>
   <223> Probe for FKBP5
<400> 265
   ttggatttga atatccc 17
<210> 266
   <211> 17
   <212> DNA
   <213> Artificial sequence
<220>
   <223> Probe for FKBP5
<400> 266
   ttctactgtt gctcctt 17
<210> 267
   <211> 17
   <212> DNA
   <213> Artificial Sequence
<220>
   <223> Probe for FKBP5
<400> 267
   cagcggcctt ccaggtg 17
<210> 268
   <211> 17
   <212> DNA
   <213> Artificial Sequence
<220>
   <223> Probe for FKBP5
<400> 268
   agtcaaacat ccttcca 17
<210> 269
   <211> 17
   <212> DNA
   <213> Artificial Sequence
<220>
   <223> Probe for FKBP5
<400> 269
   agtgaatgcc acatctc 17
<210> 270
   <211> 17
   <212> DNA
   <213> Artificial Sequence
<220>
   <223> Probe for FKBP5
<400> 270
   tggtcttctc cttcgcc 17
<210> 271
   <211> 17
   <212> DNA
   <213> Artificial Sequence
<220>
   <223> Probe for FKBP5
<400> 271
   caattccaat tggaatg 17
<210> 272
   <211> 17
   <212> DNA
   <213> Artificial Sequence
<220>
   <223> Probe for FKBP5
<400> 272
   cattttctcc agagctt 17
<210> 273
   <211> 17
   <212> DNA
   <213> Artificial sequence
<220>
   <223> Probe for FKBP5
<400> 273
   atacattgtt cttcccg 17
<210> 274
   <211> 17
   <212> DNA
   <213> Artificial Sequence
<220>
   <223> Probe for FKBP5
<400> 274
   atcttggtcc aagatat 17
<210> 275
   <211> 17
   <212> DNA
   <213> Artificial Sequence
<220>
   <223> Probe for FKBP5
<400> 275
   ccctgcctct ccaaaac 17
<210> 276
   <211> 17
   <212> DNA
   <213> Artificial sequence
<220>
   <223> Probe for FKBP5
<400> 276
   tcaatgccaa atttagg 17
<210> 277
   <211> 17
   <212> DNA
   <213> Artificial Sequence
<220>
   <223> Probe for FKBP5
<400> 277
   atataagctc agcatta 17
<210> 278
   <211> 17
   <212> DNA
   <213> Artificial Sequence
<220>
   <223> Probe for FKBP5
<400> 278
   gctcttaagt gtaactt 17
<210> 279
   <211> 17
   <212> DNA
   <213> Artificial sequence
<220>
   <223> Probe for FKBP5
<400> 279
   gattctttgg ccttttc 17
<210> 280
   <211> 17
   <212> DNA
   <213> Artificial sequence
<220>
   <223> Probe for FKBP5
<400> 280
   ctttggtatc catctcc 17
<210> 281
   <211> 17
   <212> DNA
   <213> Artificial sequence
<220>
   <223> Probe for FKBP5
<400> 281
   ggcagcctgc tccaatt 17
<210> 282
   <211> 17
   <212> DNA
   <213> Artificial sequence
<220>
   <223> Probe for FKBP5
<400> 282
   gttcccttct ctttgac 17
<210> 283
   <211> 17
   <212> DNA
   <213> Artificial sequence
<220>
   <223> Probe for FKBP5
<400> 283
   tgcctccctt gaagtat 17
<210> 284
   <211> 17
   <212> DNA
   <213> Artificial Sequence
<220>
   <223> Probe for FKBP5
<400> 284
   aatcaccgcc tgcatgt 17
<210> 285
   <211> 17
   <212> DNA
   <213> Artificial Sequence
<220>
   <223> Probe for FKBP5
<400> 285
   gacactatct tcccata 17
<210> 286
   <211> 17
   <212> DNA
   <213> Artificial sequence
<220>
   <223> Probe for FKBP5
<400> 286
   catattccat ctctaac 17
<210> 287
   <211> 17
   <212> DNA
   <213> Artificial sequence
<220>
   <223> Probe for FKBP5
<400> 287
   cgattccttt tctgata 17
<210> 288
   <211> 17
   <212> DNA
   <213> Artificial sequence
<220>
   <223> Probe for FKBP5
<400> 288
   agaaatgatt cagaagc 17
<210> 289
   <211> 17
   <212> DNA
   <213> Artificial sequence
<220>
   <223> Probe for FKBP5
<400> 289
   ggttcagaaa ggcagca 17
<210> 290
   <211> 17
   <212> DNA
   <213> Artificial sequence
<220>
   <223> Probe for FKBP5
<400> 290
   cttcaggtag cacatgg 17
<210> 291
   <211> 17
   <212> DNA
   <213> Artificial Sequence
<220>
   <223> Probe for FKBP5
<400> 291
   gctttggtgt attctct 17
<210> 292
   <211> 17
   <212> DNA
   <213> Artificial sequence
<220>
   <223> Probe for FKBP5
<400> 292
   ccttgtcaca gcattca 17
<210> 293
   <211> 17
   <212> DNA
   <213> Artificial Sequence
<220>
   <223> Probe for FKBP5
<400> 293
   ggcactgtcc agtccaa 17
<210> 294
   <211> 17
   <212> DNA
   <213> Artificial Sequence
<220>
   <223> Probe for FKBP5
<400> 294
   ctatacaagc ctttctc 17
<210> 295
   <211> 17
   <212> DNA
   <213> Artificial Sequence
<220>
   <223> Probe for FKBP5
<400> 295
   gcagctgggc ttcaccc 17
<210> 296
   <211> 17
   <212> DNA
   <213> Artificial Sequence
<220>
   <223> Probe for FKBP5
<400> 296
   tgactcaaac tcgttca 17
<210> 297
   <211> 17
   <212> DNA
   <213> Artificial sequence
<220>
   <223> Probe for FKBP5
<400> 297
   ttctggcaca tggagat 17
<210> 298
   <211> 17
   <212> DNA
   <213> Artificial sequence
<220>
   <223> Probe for FKBP5
<400> 298
   cgttgtgctc cttggcc 17
<210> 299
   <211> 17
   <212> DNA
   <213> Artificial Sequence
<220>
   <223> Probe for FKBP5
<400> 299
   gtatatcctg cggtccc 17
<210> 300
   <211> 17
   <212> DNA
   <213> Artificial sequence
<220>
   <223> Probe for FKBP5
<400> 300
   aacttcttga acatgtt 17
<210> 301
   <211> 17
   <212> DNA
   <213> Artificial Sequence
<220>
   <223> Probe for FKBP5
<400> 301
   ccttggcatc ctgctct 17
<210> 302
   <211> 17
   <212> DNA
   <213> Artificial sequence
<220>
   <223> Probe for FKBP5
<400> 302
   cattgcttta ttggcct 17
<210> 303
   <211> 17
   <212> DNA
   <213> Artificial Sequence
<220>
   <223> Probe for FKBP5
<400> 303
   ccttctgaag tcttctt 17
<210> 304
   <211> 17
   <212> DNA
   <213> Artificial Sequence
<220>
   <223> Probe for FKBP5
<400> 304
   ttcctttttc attagtg 17
<210> 305
   <211> 17
   <212> DNA
   <213> Artificial Sequence
<220>
   <223> Probe for FKBP5
<400> 305
   ttccattgct tgactgt 17
<210> 306
   <211> 18
   <212> DNA
   <213> Artificial Sequence
<220>
   <223> Probe for FKBP5
<400> 306
   stggccctca ggtttctc 18
<210> 307
   <211> 1071
   <212> DNA
   <213> Artificial sequence
<220>
   <223> Probe for FLJ11127
<4> 307
<210> 308
   <211> 17
   <212> DNA
   <213> Artificial sequence
<220>
   <223> Probe for FLJ11127
<400> 308
   cccgcgtggg gctcctt 17
<210> 309
   <211> 17
   <212> DNA
   <213> Artificial Sequence
<220>
   <223> Probe for FLJ11127
<400> 309
   agaccgctcc cgctccc 17
<210> 310
   <211> 17
   <212> DNA
   <213> Artificial Sequence
<220>
   <223> Probe for FLJ11127
<400> 310
   cttcctgcgg cgggagc 17
<210> 311
   <211> 17
   <212> DNA
   <213> Artificial Sequence
<220>
   <223> Probe for FLJ11127
<400> 311
   tccaggagtg aacttgg 17
<210> 312
   <211> 17
   <212> DNA
   <213> Artificial Sequence
<220>
   <223> Probe for FLJ11127
<400> 312
   ggcttggctt gtagcta 17
<210> 313
   <211> 17
   <212> DNA
   <213> Artificial Sequence
<220>
   <223> Probe for FLJ11127
<400> 313
   attctccaga cagtgtc 17
<210> 314
   <211> 17
   <212> DNA
   <213> Artificial sequence
<220>
   <223> Probe for FLJ11127
<400> 314
   acatcacaaa gcctttt 17
<210> 315
   <211> 17
   <212> DNA
   <213> Artificial Sequence
<220>
   <223> Probe for FLJ11127
<400> 315
   caccagaaat gaaactg 17
<210> 316
   <211> 17
   <212> DNA
   <213> Artificial sequence
<220>
   <223> Probe for FLJ11127
<400> 316
   cggaagtagc agatggc 17
<210> 317
   <211> 17
   <212> DNA
   <213> Artificial sequence
<220>
   <223> Probe for FLJ11127
<400> 317
   ctgaatataa atgtagc 17
<210> 318
   <211> 17
   <212> DNA
   <213> Artificial Sequence
<220>
   <223> Probe for FLJ11127
<400> 318
   ccaccatttc agcttgt 17
<210> 319
   <211> 17
   <212> DNA
   <213> Artificial sequence
<220>
   <223> Probe for FLJ11127
<400> 319
   tttctctgca gatatcc 17
<210> 320
   <211> 17
   <212> DNA
   <213> Artificial Sequence
<220>
   <223> Probe for FLJ11127
<400> 320
   cactgaggtt ccttttg 17
<210> 321
   <211> 17
   <212> DNA
   <213> Artificial sequence
<220>
   <223> Probe for FLJ11127
<400> 321
   taaatcaacc tctgcct 17
<210> 322
   <211> 17
   <212> DNA
   <213> Artificial sequence
<220>
   <223> Probe for FLJ11127
<400> 322
   tctcttgcac aataact 17
<210> 323
   <211> 17
   <212> DNA
   <213> Artificial Sequence
<220>
   <223> Probe for FLJ11127
<400> 323
   ggggtgtctc tcctttc 17
<210> 324
   <211> 17
   <212> DNA
   <213> Artificial sequence
<220>
   <223> Probe for FLJ11127
<400> 324
   cttcctcatc agcttgt 17
<210> 325
   <211> 17
   <212> DNA
   <213> Artificial sequence
<220>
   <223> Probe for FLJ11127
<400> 325
   caaaatagct cctcata 17
<210> 326
   <211> 17
   <212> DNA
   <213> Artificial Sequence
<220>
   <223> Probe for FLJ11127
<400> 326
   cacatttaat gtgatgc 17
<210> 327
   <211> 17
   <212> DNA
   <213> Artificial Sequence
<220>
   <223> Probe for FLJ11127
<400> 327
   atctctcctt acttgtc 17
<210> 328
   <211> 17
   <212> DNA
   <213> Artificial sequence
<220>
   <223> Probe for FUJ1127
<400> 328
   gatctgagag catcata 17
<210> 329
   <211> 17
   <212> DNA
   <213> Artificial sequence
<220>
   <223> Probe for FUJ1127
<400> 329
   tgaatatctg aaataac 17
<210> 330
   <211> 17
   <212> DNA
   <213> Artificial sequence
<220>
   <223> Probe for FLJ11127
<400> 330
   tggaaaagag atgccct 17
<210> 331
   <211> 17
   <212> DNA
   <213> Artificial sequence
<220>
   <223> Probe for FLJ11127
<400> 331
   tccttttctt tcatcca 17
<210> 332
   <211> 17
   <212> DNA
   <213> Artificial sequence
<220>
   <223> Probe for FLJ11127
<400> 332
   tttcaggaag cttaaca 17
<210> 333
   <211> 17
   <212> DNA
   <213> Artificial Sequence
<220>
   <223> Probe for FLJ11127
<400> 333
   accttgtgaa aacagca 17
<210> 334
   <211> 17
   <212> DNA
   <213> Artificial Sequence
<220>
   <223> Probe for FLJ11127
<400> 334
   tactgctgaa tccaatt 17
<210> 335
   <211> 17
   <212> DNA
   <213> Artificial Sequence
<220>
   <223> Probe for FLJ11127
<400> 335
   acttctcagg accaaaa 17
<210> 336
   <211> 17
   <212> DNA
   <213> Artificial sequence
<220>
   <223> Probe for FLJ11127
<400> 336
   aaacacattc gagcctg 17
<210> 337
   <211> 17
   <212> DNA
   <213> Artificial sequence
<220>
   <223> Probe for FLJ11127
<400> 337
   acatatttcc gtagttt 17
<210> 338
   <211> 17
   <212> DNA
   <213> Artificial Sequence
<220>
   <223> Probe for FLJ11127
<400> 338
   actgtgtttt caataat 17
<210> 339
   <211> 17
   <212> DNA
   <213> Artificial Sequence
<220>
   <223> Probe for FLJ11127
<400> 339
   aatgccatta aattcag 17
<210> 340
   <211> 17
   <212> DNA
   <213> Artificial Sequence
<220>
   <223> Probe for FLJ11127
<400> 340
   cctctcttgt gataatc 17
<210> 341
   <211> 17
   <212> DNA
   <213> Artificial Sequence
<220>
   <223> Probe for FLJ11127
<400> 341
   aaagggtgtt gcacata 17
<210> 342
   <211> 17
   <212> DNA
   <213> Artificial Sequence
<220>
   <223> Probe for FLJ11127
<400> 342
   ttccagaatg gcatctg 17
<210> 343
   <211> 17
   <212> DNA
   <213> Artificial Sequence
<220>
   <223> Probe for FLJ11127
<400> 343
   aaagcttcat aaagttt 17
<210> 344
   <211> 17
   <212> DNA
   <213> Artificial Sequence
<220>
   <223> Probe for FLJ11127
<400> 344
   gatacagcat gatgaac 17
<210> 345
   <211> 17
   <212> DNA
   <213> Artificial Sequence
<220>
   <223> Probe for FLJ11127
<400> 345
   ttcataaact tcagtga 17
<210> 346
   <211> 17
   <212> DNA
   <213> Artificial Sequence
<220>
   <223> Probe for FLJ11127
<400> 346
   acctttttag tcttcat 17
<210> 347
   <211> 17
   <212> DNA
   <213> Artificial sequence
<220>
   <223> Probe for FLJ11127
<400> 347
   gtctaaaaag actggga 17
<210> 348
   <211> 17
   <212> DNA
   <213> Artificial sequence
<220>
   <223> Probe for FLJ11127
<400> 348
   tgtctccctg gaaaaca 17
<210> 349
   <211> 17
   <212> DNA
   <213> Artificial Sequence
<220>
   <223> Probe for FLJ11127
<400> 349
   aagctcaaag gatcaga 17
<210> 350
   <211> 17
   <212> DNA
   <213> Artificial Sequence
<220>
   <223> Probe for FLJ11127
<400> 350
   aattcaggtg attcatc 17
<210> 351
   <211> 17
   <212> DNA
   <213> Artificial Sequence
<220>
   <223> Probe for FLJ11127
<400> 351
   tccacatgtg tcgccta 17
<210> 352
   <211> 17
   <212> DNA
   <213> Artificial Sequence
<220>
   <223> Probe for FLJ11127
<400> 352
   aacatatcaa tctgctc 17
<210> 353
   <211> 17
   <212> DNA
   <213> Artificial Sequence
<220>
   <223> Probe for FLJ11127
<400> 353
   caagggagta tccaagt 17
<210> 354
   <211> 17
   <212> DNA
   <213> Artificial Sequence
<220>
   <223> Probe for FLJ11127
<400> 354
   gaacactttt atcttta 17
<210> 355
   <211> 17
   <212> DNA
   <213> Artificial sequence
<220> Probe for FLJ11127
<400> 355
   gagttaaact tgaacag 17
<210> 356
   <211> 17
   <212> DNA
   <213> Artificial sequence
<220>
   <223> Probe for FLJ11127
<400> 356
   agcagacttc aaagtct 17
<210> 357
   <211> 17
   <212> DNA
   <213> Artificial sequence
<220>
   <223> Probe for FLJ11127
<400> 357
   cctgagaggc tcctctg 17
<210> 358
   <211> 17
   <212> DNA
   <213> Artificial Sequence
<220>
   <223> Probe for FLJ11127
<400> 358
   agggagatct ccggcca 17
<210> 359
   <211> 17
   <212> DNA
   <213> Artificial Sequence
<220>
   <223> Probe for FLJ11127
<400> 359
   ggcggtcgtt ctcggtc 17
<210> 360
   <211> 17
   <212> DNA
   <213> Artificial sequence
<220>
   <223> Probe for FUJ1127
<400> 360
   aaagactgga atgtggt 17
<210> 361
   <211> 5478
   <212> DNA
   <213> Artificial Sequence
<220>
   <223> Probe for Map2
<400> 361
   atggctgacg agaggaaaga cgaaggaaag gcaccacact ggacatcagc ctcactcaca 60
   gaggcagctg cacaccccca ctcgccagag atgaaggacc agggtggctc aggggaaggg 120
   ctgagccgca gcgccaatgg atttccatac agagaggagg aggaaggcgc ctttggggag 180
   cacgggtcac agggcaccta ttcagatacc aaagagaacg ggatcaacgg agagctgacc 240
   tcagctgaca gagaaacagc agaggaagtg tctgcaagga tagttcaagt agtcacagct 300
   gaagctgtag cagtcctgaa aggtgaacaa gagaaggagg cccaacacaa ggatcagcct 360
   gcagctctgc ctttagcagc tgaagaaaca gttaatctgc caccttcccc accaccatcg 420
   ccagcatcag aacaaacagc tgcactggaa gaagcctcga agatggaatt ccctgagcag 480
   cagaaattgc cttcctcatt cgctgagcct ttagacaagg aggaaacgga gtttaagatg 540
   caaagtaagc ctggtgaaga ctttgaacat gctgccttag ttcctcagcc ggacacaagt 600
   aaaactcccc aggataaaaa ggatccccaa gacatggaag gagaaaagtc gcctgccagt 660
   ccatttgcgc agactttcgg taccaacctg gaagacataa aacagatcac agaaccaagc 720
   ataacagtac ctagcattgg cctctccgca gagcccctag ctccaaaaga tcagaaagac 780
   tggttcatcg aaatgcccgt ggaatcaaag aaggatgaat ggggtttagc tgccccaata 840
   tctcctggcc ccttgacacc catgagggaa aaagatgtgc tggaggatat cccaagatgg 900
   gaaggaaagc agtttgactc tcccatgcct agccccttcc acagtggaag tttcactctt 960
   cccttagata ctgtgaaaga tgagagagtc acagaagggt cacaaccctt tgcccctgtc 1020
   ttcttccaat cagatgacaa aatgtctctg caggacacca gtggttcagc tacttccaaa 1080
   gagagttcta aagatgagga gccacagaaa gataaagcag acaaagtggc agatgttcct 1140
   gtctcagaag ctaccactgt actgggagat gttcacagtc cagctgtgga aggctttgtc 1200
   ggggagaaca tttcaggaga agaaaagggt accacagatc aagagaaaaa agagacttcg 1260
   acacccagtg tacaggaacc tacactcact gaaactgaac cacagacaaa gcttgaagag 1320
   acatcaaagg tttccatcga agaaactgtg gcaaaagaag aggaatcctt gaaattaaaa 1380
   gatgataaag caggtgtaat tcagacttcc accgagcatt ctttctccaa agaagaccag 1440
   aaaggcgaag aacagacaat cgaagcatta aaacaagact cctttcctat aagtctagaa 1500
   caggcagtta cagatgcagc catggccacc aagaccttgg aaaaggttac gtctgagcca 1560
   gaggcagtaa gtgaaaagag agaaatccag ggactttttg aagaggatat agctgacaag 1620
   agtaagctcg aaggcgctgg gtctgcaaca gtagccgagg ttgagatgcc attttatgaa 1680
   gataaatcag ggatgtccaa gtactttgaa acatctgcat tgaaagaaga tgtgaccaga 1740
   agcactgggt tgggcagtga ttactacgag ctgagtgact caagaggaaa tgcccaggaa 1800
   tctcttgata ctgtatctcc caagaaccaa caagatgaaa aggaacttct ggcaaaagct 1860
   tcccagccta gtcctccagc acacgaagca gggtacagca ctcttgccca gagttataca 1920
   tctgatcatc cgtccgagtt acctgaagaa ccaagttctc ctcaagaaag aatgttcact 1980
   attgacccca aagtttatgg ggagaaaagg gaccttcata gtaagaacaa agatgatctg 2040
   acacttagtc gaagcttggg gctgggcgga aggtctgcaa tagaacagag aagcatgtcc 2100
   attaacttgc ctatgtcttg ccttgattct attgcccttg ggtttaactt tggccggggc 2160
   catgatcttt cccctctggc ttctgatatt ctaaccaaca ctagcggaac gatggatgaa 2220
   ggagatgatt acctgccccc caccacacct gcagtggaga agattccttg ctttccaata 2280
   gagagcaaag aggaagaaga taagacagag caagcaaaag tgactggagg gcaaactacc 2340
   caagttgaaa catcctccga gtcacccttc ccagccaaag aatattacaa aaatggcact 2400
   gtcatggccc ctgacctgcc tgagatgcta gatctagcag ggaccaggtc cagattagct 2460
   tctgtgagtg cagatgctga ggttgccagg aggaaatcag tcccatcgga ggctgtggtt 2520
   gcagagagca gtactggttt gccacctgtt gctgatgaca gccaacccgt aaaaccagac 2580
   agtcaacttg aagacatggg gtactgtgtg ttcaacaagt acacagtccc tctcccatcg 2640
   ccagttcaag acagtgagaa tttgtcagga gagagtggtt cgttttatga aggaaccgat 2700
   gacaaagtcc gtagagattt ggccactgac ctttcactaa ttgaggtaaa acttgcagct 2760
   gctggaagag tcaaagatga attcactgct gagaaagagg catctccacc ctcttctgct 2820
   gacaaatcag gactgagtag ggagtttgac caagacagga aagctaatga caagctggat 2880
   actgtcctag aaaagagcga agagcatgtt gattcaaaag aacatgccaa ggagtcagaa 2940
   gaggttgggg ataaagtaga gctcttcgga ttaggtgtaa cctatgagca aacctctgcc 3000
   aaagaactga taacaactaa agaaacagca cctgagagag cagagaaagg tctcagttca 3060
   gtgccagagg tagctgaggt agaaacaacc acaaaagctg accaaggtct agatgttgct 3120
   gccaagaaag atgatcagag tccattagat ataaaagtca gtgactttgg acagatggct 3180
   tctgggatga gtgtagatgc tgggaaaacc atagagctta agttcgaggt tgatcagcag 3240
   ctgactctct catccgaagc acctcaggaa acagattcat tcatgggtat tgagtccagc 3300
   cacgtgaagg atggtgccaa agtcagtgaa acagaagtca aagagaaggt ggcaaagcct 3360
   gacttggtgc atcaggaggc tgtggacaaa gaagagtcct atgagtctag tggtgagcat 3420
   gaaagcctca ccatggagtc cctgaagcct gatgagggca agaaagaaac atctccagag 3480
   acatcactga tacaagatga agttgccctc aaactgtctg tagaaatccc ttgcccacct 3540
   ccagtttccg aagctgattc atccattgat gagaaggcgg aggtccagat ggaatttatt 3600
   cagctgccaa aggaagagag cacagagact ccggatatac ctgccatacc ttctgatgtc 3660
   acccagccac agcctgaagc agttgtgtcc gaaccagcag aggttcgagg tgaggaagaa 3720
   gagatcgaag ctgagggaga atatgacaaa ctgctcttcc gctcagacac cctccagatc 3780
   accgacctgc ttgttccagg aagtagggag gagtttgtgg agacctgccc aggggagcac 3840
   aaaggtgtgg ttgagtccgt ggtaaccatc gaggatgatt tcatcactgt agtacaaacc 3900
   acgactgatg agggagagtt gggatcccac agtgtgcgct ttgcagctcc agttcagcct 3960
   gaggaagaaa ggagaccata ccctcatgat gaagagcttg aagtactgat ggcagcagaa 4020
   gcccaggcag agcccaagga tggctctcca gatgctccag ctacccctga gaaagaagag 4080
   gttccattct cagaatataa aacagaaacc tacgacgatt acaaagatga gaccaccatt 4140
   gatgactcca ttatggatgc cgacagcctg tgggtggaca ctcaagatga tgatagaagc 4200
   atcttgacag agcagttaga aactattcct aaagaggaga gagctgagaa ggaagctcgg 4260
   agaccgtctc tcgagaaaca tagaaaagaa aaacctttta aaactgggag aggcagaatt 4320
   tccactcctg aaagaaaagt agctaaaaag gaacctagca cggtctccag ggatgaagtg 4380
   agaaggaaaa aagcagttta taagaaggct gaacttgcta aaaaatcaga agttcaggcc 4440
   cactctcctt ccaggaaact cattttaaaa cctgctatca aatacactag accaactcat 4500
   ctctcctgtg ttaagcggaa aaccacagca acaagtggtg aatcagctca ggctcccagt 4560
   gcgtttaaac aggcgaagga caaagtcact gatggaataa ccaagagccc agaaaaacgt 4620
   tcttccctcc caagaccttc ctccatcctc cctcctcgca ggggcgtatc aggagacagg 4680
   gaggagaact cgttctctct gaacagctcc atctcttcag cacgacggac caccaggtca 4740
   gaaccaattc gcagagcagg aaaaagcggc acctcaacac ctactacccc tggatctact 4800
   gcaatcaccc ctggcactcc tccaagctac tcttcacgta ccccaggcac ccctggaacc 4860
   ccgagctatc ccaggacacc aggaaccccc aaatttggca tcttggtgcc cagtgagaag 4920
   aaagttgcca tcattcgcac tcctccaaag tccccagcta ctcccaagca gcttcggctc 4980
   attaaccaac ctctgccaga cctgaagaac gtcaagtcca aaatcggatc aaccgacaac 5040
   atcaaatacc agcctaaggg gggtcaggta caaattgtta ctaagaagat agacttaagc 5100
   catgtgactt ccaaatgtgg ctctctaaag aacatccgtc acaggccagg tggtggacgc 5160
   gtgaagattg agagtgtaaa gctggatttc aaggagaagg cccaagctaa agttggctca 5220
   cttgacaatg ctcaccatgt acctggaggt ggtaacgtga agattgacag ccaaaagctg 5280
   aacttccgag agcatgcaaa ggcccgcgtc gaccacgggg ctgagatcat cacacagtcg 5340
   ccaagcaggt caagcgtggc gtctccccgg cgactcagca atgtctcctc ttctggaagc 5400
   atcaacctgc tcgaatcccc tcagctggcc actttggctg aggacgtcac tgcggcgctc 5460
   gctaagcagg gcttgtga 5478
<210> 362
   <211> 20
   <212> DNA
   <213> Artificial Sequence
<220>
   <223> Probe for Map2
<400> 362
   tgtggtgcct ttccttcgtc 20
<210> 363
   <211> 20
   <212> DNA
   <213> Artificial sequence
<220>
   <223> Probe for Map2
<400> 363
   gctgcctctg tgagtgaggc 20
<210> 364
   <211> 20
   <212> DNA
   <213> Artificial Sequence
<220>
   <223> Probe for Map2
<400> 364
   tccttcatct ctggcgagtg 20
<210> 365
   <211> 20
   <212> DNA
   <213> Artificial Sequence
<220>
   <223> Probe for Map2
<400> 365
   cggctcagcc cttcccctga 20
<210> 366
   <211> 18
   <212> DNA
   <213> Artificial Sequence
<220>
   <223> Probe for Map2
<400> 366
   tcctctctgt atggaaat 18
<210> 367
   <211> 20
   <212> DNA
   <213> Artificial Sequence
<220>
   <223> Probe for Map2
<400> 367
   gacccgtgct ccccaaaggc 20
<210> 368
   <211> 20
   <212> DNA
   <213> Artificial sequence
<220>
   <223> Probe for Map2
<400> 368
   ttctctttgg tatctgaata 20
<210> 369
   <211> 20
   <212> DNA
   <213> Artificial Sequence
<220>
   <223> Probe for Map2
<400> 369
   tcagctgagg tcagctctcc 20
<210> 370
   <211> 20
   <212> DNA
   <213> Artificial Sequence
<220>
   <223> Probe for Map2
<400> 370
   cttgcagaca cttcctctgc 20
<210> 371
   <211> 20
   <212> DNA
   <213> Artificial sequence
<220>
   <223> Probe for Map2
<400> 371
   acagcttcag ctgtgactac 20
<210> 372
   <211> 20
   <212> DNA
   <213> Artificial Sequence
<220>
   <223> Probe for Map2
<400> 372
   tccttctctt gttcaccttt 20
<210> 373
   <211> 20
   <212> DNA
   <213> Artificial sequence
<220> Probe for Map2
<400> 373
   agagctgcag gctgatcctt 20
<210> 374
   <211> 20
   <212> DNA
   <213> Artificial Sequence
<220>
   <223> Probe for Map2
<400> 374
   agattaactg tttcttcagc 20
<210> 375
   <211> 20
   <212> DNA
   <213> Artificial Sequence
<220>
   <223> Probe for Map2
<400> 375
   gatgctggcg atggtggtgg 20
<210> 376
   <211> 20
   <212> DNA
   <213> Artificial Sequence
<220>
   <223> Probe for Map2
<400> 376
   gaggcttctt ccagtgcagc 20
<210> 377
   <211> 20
   <212> DNA
   <213> Artificial Sequence
<220>
   <223> Probe for Map2
<400> 377
   aatttctgct gctcagggaa 20
<210> 378
   <211> 20
   <212> DNA
   <213> Artificial Sequence
<220>
   <223> Probe for Map2
<400> 378
   ttgtctaaag gctcagcgaa 20
<210> 379
   <211> 20
   <212> DNA
   <213> Artificial Sequence
<220>
   <223> Probe for Map2
<400> 379
   ttactttgca tcttaaactc 20
<210> 380
   <211> 20
   <212> DNA
   <213> Artificial Sequence
<220>
   <223> Probe for Map2
<400> 380
   ctgaaacttg tacgacggaa 20
<210> 381
   <211> 20
   <212> DNA
   <213> Artificial Sequence
<220>
   <223> Probe for Map2
<400> 381
   ggagttttac ttgtgtccgg 20
<210> 382
   <211> 20
   <212> DNA
   <213> Artificial sequence
<220>
   <223> Probe for Map2
<400> 382
   tccatgtctt ggggatcctt 20
<210> 383
   <211> 20
   <212> DNA
   <213> Artificial Sequence
<220>
   <223> Probe for Map2
<400> 383
   gcaaatggac tggcaggcga 20
<210> 384
   <211> 20
   <212> DNA
   <213> Artificial sequence
<220>
   <223> Probe for Map2
<400> 384
   atgtcttcca ggttggtacc 20
<210> 385
   <211> 20
   <212> DNA
   <213> Artificial sequence
<220>
   <223> Probe for Map2
<400> 385
   actgttatgc ttggttctgt 20
<210> 386
   <211> 20
   <212> DNA
   <213> Artificial Sequence
<220>
   <223> Probe for Map2
<400> 386
   aggggctctg cggagaggcc 20
<210> 387
   <211> 20
   <212> DNA
   <213> Artificial Sequence
<220> Probe for Map2
<400> 387
   atgaaccagt ctttctgatc 20
<210> 388
   <211> 20
   <212> DNA
   <213> Artificial Sequence
<220>
   <223> Probe for Map2
<400> 388
   tcatccttct ttgattccac 20
<210> 389
   <211> 20
   <212> DNA
   <213> Artificial sequence
<220>
   <223> Probe for Map2
<400> 389
   ccaggagata ttggggcagc 20
<210> 390
   <211> 20
   <212> DNA
   <213> Artificial sequence
<220>
   <223> Probe for Map2
<400> 390
   acatcttttt ccctcatggg 20
<210> 391
   <211> 20
   <212> DNA
   <213> Artificial sequence
<220>
   <223> Probe for Map2
<400> 391
   tttccttccc atcttgggat 20
<210> 392
   <211> 20
   <212> DNA
   <213> Artificial Sequence
<220>
   <223> Probe for Map2
<400> 392
   aaggggctag gcatgggaga 20
<210> 393
   <211> 20
   <212> DNA
   <213> Artificial sequence
<220>
   <223> Probe for Map2
<400> 393
   tctaagggaa gagtgaaact 20
<210> 394
   <211> 20
   <212> DNA
   <213> Artificial Sequence
<220>
   <223> Probe for Map2
<400> 394
   ccttctgtga ctctctcatc 20
<210> 395
   <211> 20
   <212> DNA
   <213> Artificial Sequence
<220>
   <223> Probe for Map2
<400> 395
   tggaagaaga caggggcaaa 20
<210> 396
   <211> 20
   <212> DNA
   <213> Artificial Sequence
<220>
   <223> Probe for Map2
<400> 396
   gtgtcctgca gagacatttt 20
<210> 397
   <211> 20
   <212> DNA
   <213> Artificial sequence
<220>
   <223> Probe for Map2
<400> 397
   gaactctctt tggaagtagc 20
<210> 398
   <211> 20
   <212> DNA
   <213> Artificial Sequence
<220>
   <223> Probe for Map2
<400> 398
   gctttatctt tctgtggctc 20
<210> 399
   <211> 20
   <212> DNA
   <213> Artificial Sequence
<220>
   <223> Probe for Map2
<400> 399
   tctgagacag gaacatctgc 20
<210> 400
   <211> 20
   <212> DNA
   <213> Artificial Sequence
<220>
   <223> Probe for Map2
<400> 400
   ctgtgaacat ctcccagtac 20
<210> 401
   <211> 20
   <212> DNA
   <213> Artificial Sequence
<220>
   <223> Probe for Map2
<400> 401
   ttctccccga caaagccttc 20
<210> 402
   <211> 20
   <212> DNA
   <213> Artificial sequence
<220>
   <223> Probe for Map2
<400> 402
   tctgtggtac ccttttcttc 20
<210> 403
   <211> 20
   <212> DNA
   <213> Artificial Sequence
<220>
   <223> Probe for Map2
<400> 403
   ctgggtgtcg aagtctcttt 20
<210> 404
   <211> 20
   <212> DNA
   <213> Artificial Sequence
<220> Probe for Map2
<400> 404
   tcagtttcag tgagtgtagg 20
<210> 405
   <211> 20
   <212> DNA
   <213> Artificial Sequence
<220>
   <223> Probe for Map2
<400> 405
   tttgatgtct cttcaagctt 20
<210> 406
   <211> 20
   <212> DNA
   <213> Artificial sequence
<220>
   <223> Probe for Map2
<400> 406
   tcttttgcca cagtttcttc 20
<210> 407
   <211> 20
   <212> DNA
   <213> Artificial sequence
<220>
   <223> Probe for Map2
<400> 407
   ttatcatctt ttaatttcaa 20
<210> 408
   <211> 20
   <212> DNA
   <213> Artificial sequence
<220>
   <223> Probe for Map2
<400> 408
   tgctcggtgg aagtctgaat 20
<210> 409
   <211> 20
   <212> DNA
   <213> Artificial sequence
<220>
   <223> Probe for Map2
<400> 409
   tcgcctttct ggtcttcttt 20
<210> 410
   <211> 20
   <212> DNA
   <213> Artificial sequence
<220>
   <223> Probe for Map2
<400> 410
   cagacccagc gccttcgagc 20
<210> 411
   <211> 20
   <212> DNA
   <213> Artificial Sequence
<220>
   <223> Probe for Map2
<400> 411
   gcccaaccca gtgcttctgg 20
<210> 412
   <211> 20
   <212> DNA
   <213> Artificial Sequence
<220>
   <223> Probe for Map2
<400> 412
   gctggaggac taggctggga 20
<210> 413
   <211> 20
   <212> DNA
   <213> Artificial sequence
<220>
   <223> Probe for Map2
<400> 413
   ccgcccagcc ccaagcttcg 20
<210> 414
   <211> 20
   <212> DNA
   <213> Artificial Sequence
<220>
   <223> Probe for Map2
<400> 414
   gatcatggcc ccggccaaag 20
<210> 415
   <211> 20
   <212> DNA
   <213> Artificial sequence
<220>
   <223> Probe for Map2
<400> 415
   caggtgtggt ggggggcagg 20
<210> 416
   <211> 20
   <212> DNA
   <213> Artificial Sequence
<220>
   <223> Probe for Map2
<400> 416
   ggctgggaag ggtgactcgg 20
<210> 417
   <211> 20
   <212> DNA
   <213> Artificial sequence
<220>
   <223> Probe for Map2
<400> 417
   ggcaggtcag gggccatgac 20
<210> 418
   <211> 20
   <212> DNA
   <213> Artificial Sequence
<220>
   <223> Probe for Map2
<400> 418
   ctggacctgg tccctgctag 20
<210> 419
   <211> 20
   <212> DNA
   <213> Artificial Sequence
<220>
   <223> Probe for Map2
<400> 419
   cacagcctcc gatgggactg 20
<210> 420
   <211> 20
   <212> DNA
   <213> Artificial sequence
<220>
   <223> Probe for Map2
<400> 420
   gaactggcga tgggagaggg 20
<210> 421
   <211> 20
   <212> DNA
   <213> Artificial Sequence
<220>
   <223> Probe for Map2
<400> 421
   gccctcatca ggcttcaggg 20
<210> 422
   <211> 20
   <212> DNA
   <213> Artificial sequence
<220>
   <223> Probe for Map2
<400> 422
   ccatctggac ctccgccttc 20
<210> 423
   <211> 20
   <212> DNA
   <213> Artificial Sequence
<220>
   <223> Probe for Map2
<400> 423
   tcaggctgtg gctgggtgac 20
<210> 424
   <211> 20
   <212> DNA
   <213> Artificial Sequence
<220>
   <223> Probe for Map2
<400> 424
   tctggagggt gtctgagcgg 20
<210> 425
   <211> 20
   <212> DNA
   <213> Artificial sequence
<220>
   <223> Probe for Map2
<400> 425
   gctcccctgg gcaggtctcc 20
<210> 426
   <211> 20
   <212> DNA
   <213> Artificial Sequence
<220>
   <223> Probe for Map2
<400> 426
   agcgcacact gtgggatccc 20
<210> 427
   <211> 20
   <212> DNA
   <213> Artificial sequence
<220>
   <223> Probe for Map2
<400> 427
   tccttgggct ctgcctgggc 20
<210> 428
   <211> 20
   <212> DNA
   <213> Artificial Sequence
<220>
   <223> Probe for Map2
<400> 428
   gctggagcat ctggagagcc 20
<210> 429
   <211> 20
   <212> DNA
   <213> Artificial sequence
<220>
   <223> Probe for Map2
<400> 429
   tccacccaca ggctgtcggc 20
<210> 430
   <211> 20
   <212> DNA
   <213> Artificial sequence
<220>
   <223> Probe for Map2
<400> 430
   gagacggtct ccgagcttcc 20
<210> 431
   <211> 20
   <212> DNA
   <213> Artificial Sequence
<220>
   <223> Probe for Map2
<400> 431
   tccctggaga ccgtgctagg 20
<210> 432
   <211> 20
   <212> DNA
   <213> Artificial Sequence
<220>
   <223> Probe for Map2
<400> 432
   cgcactggga gcctgagctg 20
<210> 433
   <211> 20
   <212> DNA
   <213> Artificial Sequence
<220>
   <223> Probe for Map2
<400> 433
   ggaggaaggt cttgggaggg 20
<210> 434
   <211> 1710
   <212> DNA
   <213> Artificial sequence
<220>
   <223> Probe for STL1
<400> 434
   atgaaggatt taaaattatc gaatttcaaa ggcaaattta taagcagaac cagtcactgg 60
   ggacttacgg gtaagaagtt gcggtatttc atcactatcg catctatgac gggcttctcc 120
   ctgtttggat acgaccaagg gttgatggca agtctaatta ctggtaaaca gttcaactat 180
   gaatttccag caaccaaaga aaatggcgat catgacagac acgcaactgt agtgcagggc 240
   gctacaacct cctgttatga attaggttgt ttcgcaggtt ctctattcgt tatgttctgc 300
   ggtgaaagaa ttggtagaaa accattaatc ctgatgggtt ccgtaataac catcattggt 360
   gccgttattt ctacatgcgc atttcgtggt tactgggcat taggccagtt tatcatcgga 420
   agagtcgtca ccggtgttgg aacagggttg aatacatcta ctattcccgt ttggcaatca 480
   gaaatgtcaa aagctgaaaa tagagggttg ctggtcaatt tagaaggttc cacaattgct 540
   tttggtacta tgattgctta ttggattgat tttgggttgt cttataccaa cagttctgtt 600
   cagtggagat tccccgtgtc aatgcaaatc gtttttgctc tcttcctgct tgctttcatg 660
   attaaactac ctgaatcgcc acgttggctg atttctcaaa gtcgaacaga agaagctcgc 720
   tacttggtag gaacactaga cgacgcggat ccaaatgatg aggaagttat aacagaagtt 780
   gctatgcttc acgatgctgt taacaggacc aaacacgaga aacattcact gtcaagtttg 840
   ttctccagag gcaggtccca aaatcttcag agggctttga ttgcagcttc aacgcaattt 900
   ttccagcaat ttactggttg taacgctgcc atatactact ctactgtatt attcaacaaa 960
   acaattaaat tagactatag attatcaatg atcataggtg gggtcttcgc aacaatctac 1020
   gccttatcta ctattggttc attttttcta attgaaaagc taggtagacg taagctgttt 1080
   ttattaggtg ccacaggtca agcagtttca ttcacaatta catttgcatg cttggtcaaa 1140
   gaaaataaag aaaacgcaag aggtgctgcc gtcggcttat ttttgttcat tacattcttt 1200
   ggtttgtctt tgctatcatt accatggata tacccaccag aaattgcatc aatgaaagtt 1260
   cgtgcatcaa caaacgcttt ctccacatgt actaattggt tgtgtaactt tgcggttgtc 1320
   atgttcaccc caatatttat tggacagtcc ggttggggtt gctacttatt ttttgctgtt 1380
   atgaattatt tatacattcc agttatcttc tttttctacc ctgaaaccgc cggaagaagt 1440
   ttggaggaaa tcgacatcat ctttgctaaa gcatacgagg atggcactca accatggaga 1500
   gttgctaacc atttgcccaa gttatcccta caagaagtcg aagatcatgc caatgcattg 1560
   ggctcttatg acgacgaaat ggaaaaagag gactttggtg aagatagagt agaagacacc 1620
   tataaccaaa ttaacggcga taattcgtct agttcttcaa acatcaaaaa tgaagataca 1680
   gtgaacgata aagcaaattt tgagggttga 1710
<210> 435
   <211> 20
   <212> DNA
   <213> Artificial Sequence
<220>
   <223> Probe for STL1
<400> 435
   gcctttgaaa ttcgataatt 20
<210> 436
   <211> 20
   <212> DNA
   <213> Artificial Sequence
<220>
   <223> Probe for STL1
<400> 436
   cagtgactgg ttctgcttat 20
<210> 437
   <211> 20
   <212> DNA
   <213> Artificial Sequence
<220>
   <223> Probe for STL1
<400> 437
   taccgcaact tcttacccgt 20
<210> 438
   <211> 20
   <212> DNA
   <213> Artificial Sequence
<220>
   <223> Probe for STL1
<400> 438
   gaagcccgtc atagatgcga 20
<210> 439
   <211> 20
   <212> DNA
   <213> Artificial sequence
<220>
   <223> Probe for STL1
<400> 439
   tcaacccttg gtcgtatcca 20
<210> 440
   <211> 20
   <212> DNA
   <213> Artificial sequence
<220>
   <223> Probe for STL1
<400> 440
   gttgaactgt ttaccagtaa 20
<210> 441
   <211> 20
   <212> DNA
   <213> Artificial Sequence
<220>
   <223> Probe for STL1
<400> 441
   tgtctgtcat gatcgccatt 20
<210> 442
   <211> 20
   <212> DNA
   <213> Artificial sequence
<220>
   <223> Probe for STL1
<400> 442
   gaacctgcga aacaacctaa 20
<210> 443
   <211> 20
   <212> DNA
   <213> Artificial Sequence
<220>
   <223> Probe for STL1
<400> 443
   ttcaccgcag aacataacga 20
<210> 444
   <211> 20
   <212> DNA
   <213> Artificial sequence
<220>
   <223> Probe for STL1
<400> 444
   tacggaaccc atcaggatta 20
<210> 445
   <211> 20
   <212> DNA
   <213> Artificial sequence
<220>
   <223> Probe for STL1
<400> 445
   cacgaaatgc gcatgtagaa 20
<210> 446
   <211> 20
   <212> DNA
   <213> Artificial Sequence
<220>
   <223> Probe for STL1
<400> 446
   tccgatgata aactggccta 20
<210> 447
   <211> 20
   <212> DNA
   <213> Artificial sequence
<220>
   <223> Probe for STL1
<400> 447
   ttctgattgc caaacgggaa 20
<210> 448
   <211> 20
   <212> DNA
   <213> Artificial Sequence
<220>
   <223> Probe for STL1
<400> 448
   gcaaccctct attttcagct 20
<210> 449
   <211> 20
   <212> DNA
   <213> Artificial Sequence
<220>
   <223> Probe for STL1
<400> 449
   gcaattgtgg aaccttctaa 20
<210> 450
   <211> 20
   <212> DNA
   <213> Artificial Sequence
<220>
   <223> Probe for STL1
<400> 450
   caacccaaaa tcaatccaat 20
<210> 451
   <211> 20
   <212> DNA
   <213> Artificial sequence
<220>
   <223> Probe for STL1
<400> 451
   cactgaacag aactgttggt 20
<210> 452
   <211> 20
   <212> DNA
   <213> Artificial Sequence
<220>
   <223> Probe for STL1
<400> 452
   caggaagaga gcaaaaacga 20
<210> 453
   <211> 20
   <212> DNA
   <213> Artificial sequence
<220>
   <223> Probe for STL1
<400> 453
   gtggcgattc aggtagttta 20
<210> 454
   <211> 20
   <212> DNA
   <213> Artificial Sequence
<220>
   <223> Probe for STL1
<400> 454
   cttctgttcg actttgagaa 20
<210> 455
   <211> 20
   <212> DNA
   <213> Artificial sequence
<220>
   <223> Probe for STL1
<400> 455
   ctgttataac ttcctcatca 20
<210> 456
   <211> 20
   <212> DNA
   <213> Artificial Sequence
<220>
   <223> Probe for STL1
<400> 456
   gttaacagca tcgtgaagca 20
<210> 457
   <211> 20
   <212> DNA
   <213> Artificial sequence
<220>
   <223> Probe for STL1
<400> 457
   acagtgaatg tttctcgtgt 20
<210> 458
   <211> 20
   <212> DNA
   <213> Artificial Sequence
<220>
   <223> Probe for STL1
<400> 458
   ggacctgcct ctggagaaca 20
<210> 459
   <211> 20
   <212> DNA
   <213> Artificial sequence
<220>
   <223> Probe for STL1
<400> 459
   caatcaaagc cctctgaaga 20
<210> 460
   <211> 20
   <212> DNA
   <213> Artificial Sequence
<220>
   <223> Probe for STL1
<400> 460
   gcagcgttac aaccagtaaa 20
<210> 461
   <211> 20
   <212> DNA
   <213> Artificial Sequence
<220>
   <223> Probe for STL1
<400> 461
   gaataataca gtagagtagt 20
<210> 462
   <211> 20
   <212> DNA
   <213> Artificial sequence
<220>
   <223> Probe for STL1
<400> 462
   ccccacctat gatcattgat 20
<210> 463
   <211> 20
   <212> DNA
   <213> Artificial Sequence
<220>
   <223> Probe for STL1
<400> 463
   aatagtagat aaggcgtaga 20
<210> 464
   <211> 20
   <212> DNA
   <213> Artificial sequence
<220>
   <223> Probe for STL1
<400> 464
   agcttacgtc tacctagctt 20
<210> 465
   <211> 20
   <212> DNA
   <213> Artificial Sequence
<220>
   <223> Probe for STL1
<400> 465
   ttgacctgtg gcacctaata 20
<210> 466
   <211> 20
   <212> DNA
   <213> Artificial sequence
<220>
   <223> Probe for STL1
<400> 466
   gaccaagcat gcaaatgtaa 20
<210> 467
   <211> 20
   <212> DNA
   <213> Artificial Sequence
<220>
   <223> Probe for STL1
<400> 467
   gcacctcttg cgttttcttt 20
<210> 468
   <211> 20
   <212> DNA
   <213> Artificial Sequence
<220>
   <223> Probe for STL1
<400> 468
   ccaaagaatg taatgaacaa 20
<210> 469
   <211> 20
   <212> DNA
   <213> Artificial Sequence
<220>
   <223> Probe for STL1
<400> 469
   tggtgggtat atccatggta 20
<210> 470
   <211> 20
   <212> DNA
   <213> Artificial Sequence
<220>
   <223> Probe for STL1
<400> 470
   cgtttgttga tgcacgaact 20
<210> 471
   <211> 20
   <212> DNA
   <213> Artificial Sequence
<220>
   <223> Probe for STL1
<400> 471
   ccgcaaagtt acacaaccaa 20
<210> 472
   <211> 20
   <212> DNA
   <213> Artificial sequence
<220>
   <223> Probe for STL1
<400> 472
   caaccggact gtccaataaa 20
<210> 473
   <211> 20
   <212> DNA
   <213> Artificial sequence
<220>
   <223> Probe for STL1
<400> 473
   aattcataac agcaaaaaat 20
<210> 474
   <211> 20
   <212> DNA
   <213> Artificial Sequence
<220>
   <223> Probe for STL1
<400> 474
   tcagggtaga aaaagaagat 20
<210> 475
   <211> 20
   <212> DNA
   <213> Artificial Sequence
<220>
   <223> Probe for STL1
<400> 475
   gatgatgtcg atttcctcca 20
<210> 476
   <211> 20
   <212> DNA
   <213> Artificial Sequence
<220>
   <223> Probe for STL1
<400> 476
   agtgccatcc tcgtatgctt 20
<210> 477
   <211> 20
   <212> DNA
   <213> Artificial sequence
<220>
   <223> Probe for STL1
<400> 477
   ggataacttg ggcaaatggt 20
<210> 478
   <211> 20
   <212> DNA
   <213> Artificial Sequence
<220>
   <223> Probe for STL1
<400> 478
   cgtcataaga gcccaatgca 20
<210> 479
   <211> 20
   <212> DNA
   <213> Artificial Sequence
<220>
   <223> Probe for STL1
<400> 479
   ccaaagtcct ctttttccat 20
<210> 480
   <211> 20
   <212> DNA
   <213> Artificial Sequence
<220>
   <223> Probe for STL1
<400> 480
   ttataggtgt cttctactct 20
<210> 481
   <211> 20
   <212> DNA
   <213> Artificial Sequence
<220>
   <223> Probe for STL1
<400> 481
   ctagacgaat tatcgccgtt 20
<210> 482
   <211> 20
   <212> DNA
   <213> Artificial sequence
<220>
   <223> Probe for STL1
<400> 482
   cgttcactgt atcttcattt 20

## Description

### Field of the invention

This invention is defined by the appended claims.

### Background

As it has become increasingly apparent that gene expression in individual cells deviates significantly from the average behavior of cell populations, new methods that provide accurate integer counts of mRNA copy numbers in individual cells are needed. Ideally, such methods should also reveal the intracellular locations of the mRNAs, as mRNA localization is often used by cells to spatially restrict the activity gene.

*In situ* hybridization, followed by microscopic analysis, is a well-established means of studying gene expression. The first generation of *in situ* hybridizations was performed with radioactive probes. Early improvements involved linking the probes to enzymes that catalyze chromogenic or fluorogenic reactions. However, because the products of these reactions were small molecules or precipitates that diffuse away from the probe, the location of the target molecules could not be precisely determined. Conversely, probes labeled directly with a few fluorophores maintained spatial resolution, but the sensitivity that can be achieved is relatively poor.

Robert Singer and colleagues developed an *in situ* hybridization procedure that was not only sensitive enough to permit the detection of single mRNA molecules, but also restricted the signals to close proximity of the targets. They hybridized five oligonucleotide probes simultaneously to each mRNA target, each of which was about 50-nucleotides in length and each of which was labeled with five fluorophore moieties. Although the authors convincingly demonstrated single molecule sensitivity and other groups have successfully used these probes, the system has not been widely adopted. One reason for this is difficulty in the synthesis and purification of heavily labeled oligonucleotides. Usually, flurophore moieties are introduced via primary amino groups that are incorporated into oligonucleotides during their synthesis. When multiple amino groups are introduced into the same oligonucleotide some are lost due to side reactions such as transamidation. Coupling of fluorophores to the remaining amino groups is inefficient and requires several consecutive coupling reactions and it is difficult to purify oligonucleotides in which all designed sites are coupled to fluorophores from those that are partially coupled. Also, when some fluorophores are present in multiple copies on the same oligonucleotide they interact with each other altering the hybridization characteristics of the oligonucleotides and exhibiting severe self-quenching. These problems are obviated if each probe had just a single terminal amino group to serve as the site of attachment.

Another issue with the use of small numbers of heavily labeled probes is that a significant portion of the fluorescence is lost for every probe that does not bind to the target, whereas every non-specific binding event increases the background. This leads to a widened distribution of number of probes bound to each target mRNA. For instance, when using 5 fluorescent probes targeted to a single mRNA, Femino et al estimated that the majority of the fluorescent spots observed had intensities indicating the presence of only 1 or 2 probes. Science 280, 585-590 (1998). This makes it difficult to unambiguously identify those fluorescent spots as mRNA molecules, since it is impossible to determine whether the detection of an individual probe arises from legitimate binding to the target mRNA or non-specific binding. These "thresholding" problems limit the ability of such methods to provide reliable counts of mRNA numbers in individual cells. Further documents describe probes for in-situ hybridization methods: WO97/14816 A1, US6329152 B1, WO2007/001986 A2, WO02/22874 A2, Lee Sanghoon et al., Marine Ecology Progress Series, 101, pp.193. 04-11-1993, Kosman D. et al., Science,305, pp. 846, 06.08.2004.

Thus there remains a need for improved methods to provide reliable counts of mRNA numbers in individual cells and a need for probes that are easily synthesized and purified.

### Summary of the invention

This disclosure provides a method for detecting individual nucleic acid molecules, such as, for example, RNA molecules, e.g., mRNA molecules in fixed, permeabilized cells using a plurality of nucleic acid hybridization probes that are singly fluorescently labeled, as with the same fluorophore. The inventors have surprisingly discovered that if at least 30, preferably 40-60, and very preferably 48 different probes, all labeled with the same fluorophore, are hybridized simultaneously to a target sequence of an mRNA molecule, a fluorescent spot is created that can be detected from the combined fluorescences of the multiple probes. The probes are non-overlapping; that is, the region of the target sequence to which each probe hybridizes is unique (or non-overlapping). Probes in a set of 30 or more for a selected target sequence can be designed to hybridize adjacently to one another or to hybridize non-adjacently, with stretches of the target sequence, from one nucleotide to a hundred nucleotides or more, not complementary to any of the probes. Accordingly, in one aspect, the disclosure provides a method for probing a target sequence of nucleic acid molecules such as, for example, mRNAs in a fixed, permeabilized cell, said target sequence including at least 30 non-overlapping probe binding regions of 15-100 nucleotides, comprising immersing said cell in an excess of at least 30 nucleic acid hybridization probes, each singly labeled with the same fluorescent label and each containing a nucleic acid sequence that is complementary to a different probe binding region of said target sequence; washing said fixed cell to remove unbound probes; and detecting fluorescence from said probes.

Probes useful in this invention may be DNA, RNA or mixtures of DNA and RNA. They may include non-natural nucleotides, and they may include non-natural internucleotide linkages. Non-natural nucleotides that increase the binding affinity of probes include 2'-O-methyl ribonucleotides. The lengths of probes useful in this disclosure are 15-40 nucleotides for typical DNA or RNA probes of average binding affinity. Preferred lengths of DNA probes and RNA probes are in the range of 15-20 nucleotides, more preferably 17-25 nucleotides and even more preferably 17-22 nucleotides. The inventors have constructed the probes to be about 20 nucleotides long. If means are included to increase a probe's binding affinity, the probe can be shorter, as short as seven nucleotides, as persons in the art will appreciate. A fluorophore can be attached to a probe at any position, including, without limitation, attaching a fluorophore to one end of a probe, preferably to the 3' end. The probes may be included in a hybridization solution that contains the multiple probes in excess, commonly in the range of 0.2-1 nanograms per microliter. Sufficient solution is added to cover and wet the cell so that the cell is immersed in the probe-containing solution.

A single cell can be probed simultaneously for multiple mRNA target sequences, either more than one target sequence of one mRNA molecule, or one or more sequences of different mRNA molecules. Additionally, one target sequence of an mRNA molecule can be probed with more than one set of probes, wherein each set is labeled with a distinguishable fluorophore, and the fluorophores are distinguishable. For example, in probing a gene sequence, at least 30 green-labeled probes can be used to probe one portion of the gene sequence as its target sequence, and at least 30 red-labeled probes can be used to probe a different portion of the gene sequence as its target sequence. Using more than one color for each of multiple targets permits use of color-coding schemes in highly multiplexed probing methods according to this disclosure.

Methods of this disclosure may include simply looking to see if one or more spots representing a target sequence are present. Methods according to this disclosure also include counting spots of a given color corresponding to a given mRNA species. When it is desired to detect more than one species of mRNA, different sets of probes labeled with distinct fluorophores can be used in the same hybridization mixture. A gene expression profile for each species of mRNA is constructed by counting spots of different colors.

Spots can be detected utilizing microscopic methods. It is not necessary to use a confocal microscope, as a wide-field fluorescence microscope is sufficient. To distinguish spots that positively reflect a target sequence from dim spots that may reflect background fluorescence or nonspecific binding, methods according to this disclosure include detection. In one embodiment, the detection comprises filtering images with a three-dimensional linear Laplacian of Gaussian filter and applying a detection threshold. If one plots the number of spots in three dimensions for all thresholds ranging from zero to the maximum pixel intensity in the filtered image, there is a wide plateau, indicative of a region in which the number of spots detected is insensitive to threshold. Thus, the method further comprises plotting the number of spots, determining the boundaries of a plateau region, and selecting the threshold preferably within that region.

In another aspect, this invention includes sets of probes for in situ hybridization that enable detection of individual mRNA molecules in cells. The probes render each molecule so intensely fluorescent that it can be seen as a fine fluorescent spot in fluorescence microscopy.

A computer program can be used to identify and count all the mRNA molecules in the cell from the microscopic image. In situ hybridizations performed with the sets of probes described above allow accurate and simple gene expression analysis, detection of pathogens and pathogenic states such as cancer.

Accordingly, in another aspect, provided is a method of screening for compounds which alter the amount of a subcellular distribution of the target sequence. The method includes incubating a cell with a test compound for a period of time sufficient to elicit a response, detecting the amount of distribution pattern of the target sequence, and comparing this amount or distribution with an amount or distribution of the target mRNA in a control cell which was treated identically, but not incubated with the test compound.

In yet another aspect, the disclosure provides a computer readable medium, comprising instructions for: obtaining a 3-D stack of 2-D fluorescent images; filtering said 3-D stack using a 3-D filter; counting a total number of 3-D spots in said filtered 3-D stack for each of a plurality of intensity thresholds; obtaining an optimum intensity threshold representative of a plateau region in a plot of said total number of 3-D spots verses the intensity threshold at which said total number was counted; and using the total number of 3-D spots obtained at said optimum threshold as representative of a number of fluorescing particles detected in said 3-D stack.

The invention also provides a kit, generally comprising the set of probes and the computer-readable media as described above.

### Brief description of the drawings

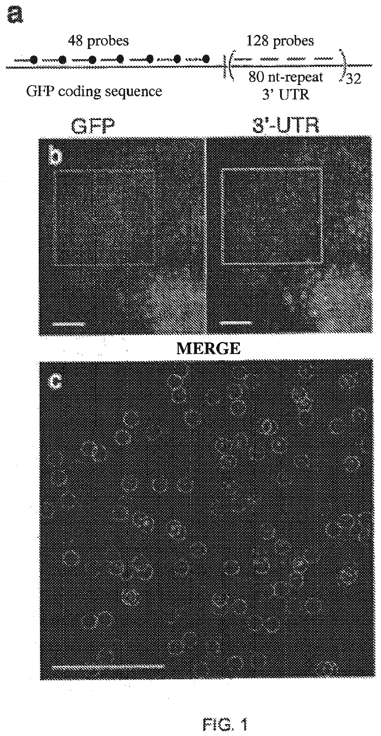

Figure 1 shows simultaneous detection of a unique sequence and a repeated sequence in individual mRNA molecules. FIG. 1A is a schematic depiction of the construct used. The 48 probes used to detect the GFP coding sequence were labeled with Alexa-594 and the four different probes used to detect the tandem repeat in the 3'-UTR were labeled with TMR. FIG. 1B illustrates maximum intensity merges of a pair of z-stack of fluorescent images of CHO cells taken in the Alexa-594 channel (left) and the TMR channel (right) corresponding to GFP coding region probes and UTR probes, respectively. FIG. 1C illustrates false color merge of the images in FIG. 1B enclosed by the red (GFP) and green (UTR) squares, with red circles representing computationally identified GFP mRNA particles, green circles representing UTR particles, and yellow circles representing co-localized particles. All scale bars are 5 µm long.

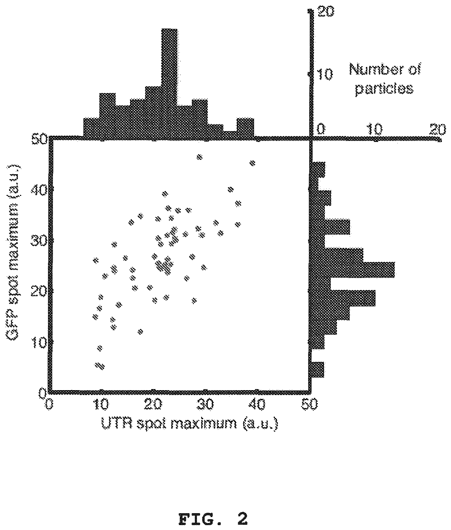

Figure 2 shows intensity analysis of colocalized spots. Spot intensities corresponding to the GFP-targeted probes (Alexa 594 channel, y axis) and multimeric UTR-targeted probes (TMR channel, x axis) were computed by taking the maximum intensity in the computationally identified spot region and subtracting the mean intensity of an annular region surrounding the spot. Marginal histograms show the distributions of GFP spot intensities (right) and UTR spot intensities (top).

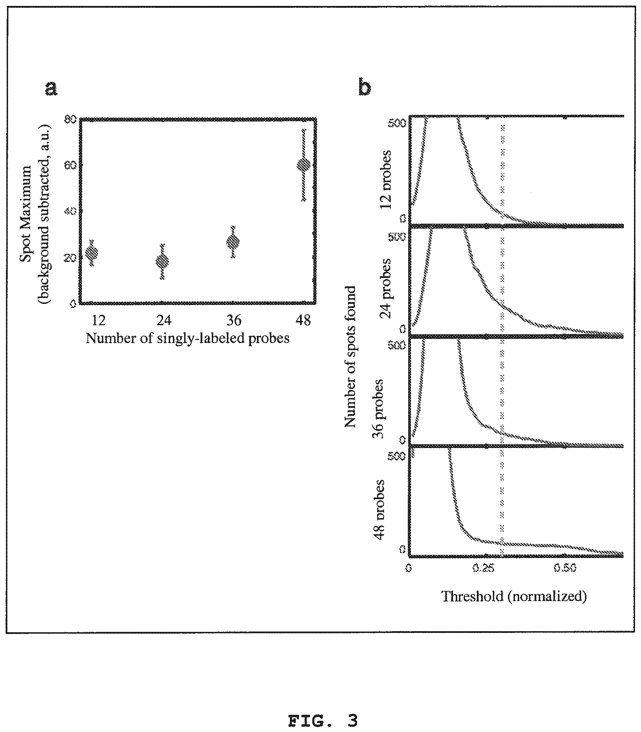

Figure 3 shows sensitivity of method when using different numbers of probes. FIG. 3A illustrates spot intensity (defined as maximum intensity within the spot minus the mean background taken in an annular region surrounding the spot) as a function of the number of probes chosen. Intensities for 12 and 24 probes are artifactual in that spots were not readily identifiable in those cases, so spots identified were biased towards being brighter. FIG. 3B illustrates the number of spots (i.e., connected components) found upon thresholding the filtered image plotted as a function of the threshold value, ranging from 0 to the maximum intensity of the filtered image (normalized to 1) for different numbers of probes. The grey bar indicates the threshold used for the analysis in FIG 3A.

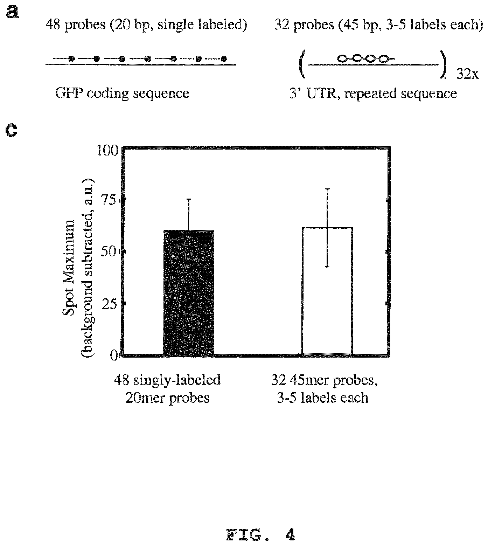

Figure 4 shows comparison with the mRNA detection method of Femino et al. (Science 1998). FIG. 4A is a schematic depicting the method described in this manuscript with 48 singly labeled probes (left) and the method of Femino et al. in which each 45 bp probe contains five fluorophores each and is targeted to a sequence element that is repeated 32 times in the 3'UTR of the target mRNA expressed from a transgene in Chinese hamster ovary cells. FIG. 3B illustrates a comparison of spot intensities when using 48 singly labeled probes or using a 45 bp probe labeled with five fluorophores. Error bars represent one standard deviation.

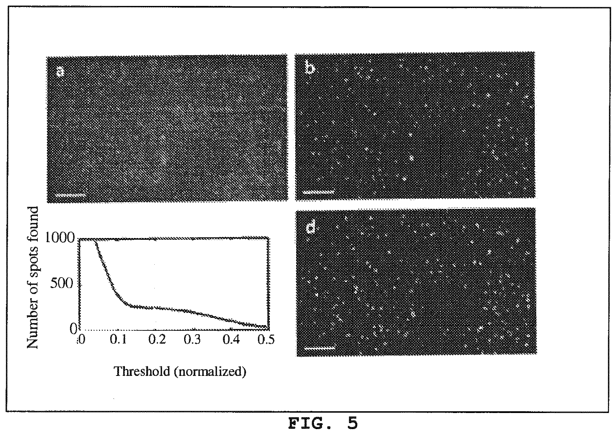

Figure 5 shows computational identification of mRNA spots. FIG. 5A illustrates raw image data (maximum intensity merge) obtained from imaging FKBP5 mRNA particles in A549 cells induced with dexamethasone. FIG. 5B illustrates mage (maximum merge) obtained by running raw data through Laplacian of a Gaussian filter to enhance spots. FIG. 5C illustrates the number of spots (i.e., connected components) found upon thresholding the filtered image from FIG. 5B is plotted as a function of the threshold value, ranging from 0 to the maximum intensity of the filtered image (normalized to 1). FIG. 5D is an image showing the results of using the threshold represented by the grey line in FIG. 5C with each distinct spot assigned a random color. All scale bars are 5
   µm long.

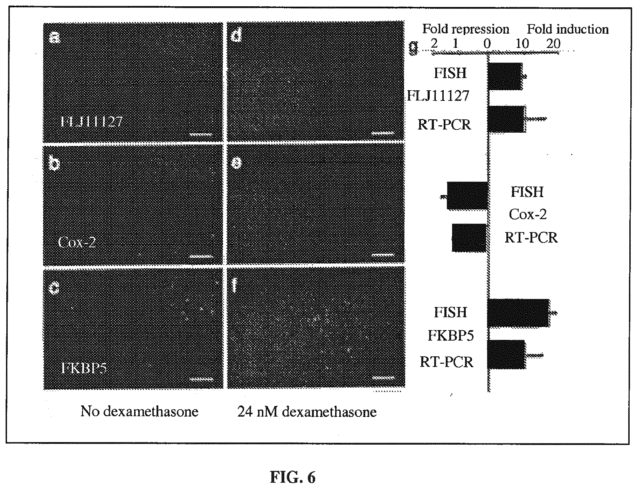

Figure 6 shows simultaneously imaging single molecules of three different mRNAs in mammalian cells. FIG. 6A- FIG. 6C illustrate images showing FLJ11127, Cox-2 and FKBP5 mRNA particles in the same set of A549 cells not treated with dexamethasone. FIG. 6D- FIG. 6F illustrate images showing FLJ11127, Cox-2 and FKBP5 particles in cells treated for 8 hours with 24nM dexamethasone. FIG. 6G illustrates fold induction for all three genes as measured by FISH and realtime RT-PCR; error bars for FISH were obtained by bootstrapping and those for RTPCR were obtained by repetition as described in the supplementary information. All images are maximum merges of a z-stack of fluorescent images spanning the extent of the cells with nuclear DAPI counterstaining in purple, and all scale bars are 5 µm long.

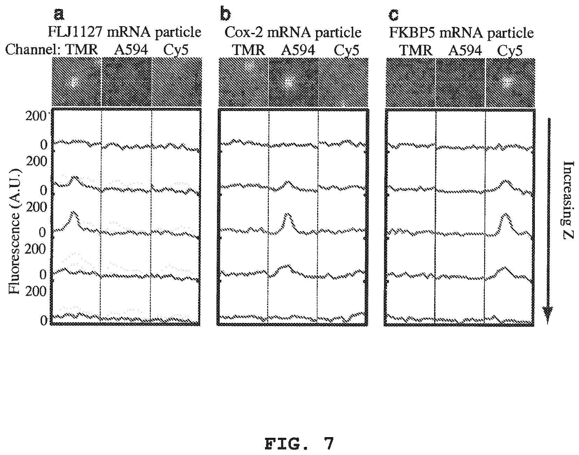

Figure 7 shows examination of fluorescent spot bleedthrough. FIG. 7A is an illustration of images of an FLJ11127 mRNA spot labeled with TMR as seen through the TMR, Alexa 594 and Cy5 filter channels. Linescans of fluorescent intensity corresponding to the line through the image are given below, with the different linescans corresponding to measurements taken at increasing z (0.25 µm spacing). The green linescan corresponds to the z-slice shown in the image itself. A similar analysis was performed for a Cox-2 mRNA spot labeled with Alexa 594 (FIG. 7B) and an FKBP5 mRNA particle labeled with Cy5 (FIG. 7C). All linescan intensity measurements had the camera background subtracted but range between 0 and 200 arbitrary fluorescence units.

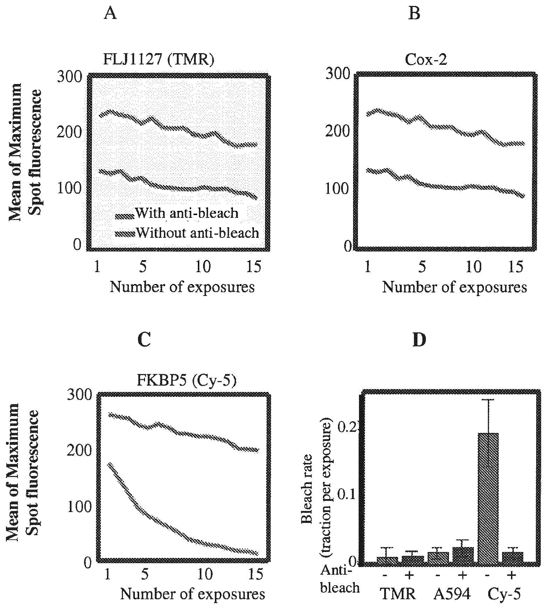

Figure 8 shows demonstration that the oxygen-scavenger increases photostability of Cy5. FIG. 8A illustrates the mean of the maximum spot fluorescence for a number of FLJ11127 mRNAs labeled using TMR conjugated probes was plotted as a function of the number of 2 second exposures using a filter specific for TMR. Curves were generated for images taken both with (blue) and without (red) the oxygen scavenging system. A similar analysis was performed for Cox-2 mRNAs labeled using Alexa-594 conjugated probes with 2 second exposures (FIG. 8B) and FKBP5 mRNAs labeled using Cy5 conjugated probes with 2.5 second exposures (FIG. 8C). FIG. 8D illustrates the bleach rate per exposure (in units of fraction of fluorescence lost per exposure) for the TMR, Alexa-594 and Cy5 conjugates probes in (FIG. 8A-FIG. 8C) both with and without the oxygen-scavenging anti-bleach system. The bleach rate was calculated by fitting each individual particle's decay curve to an exponential and taking the mean of the fitted decay constants. The error bars correspond to one standard deviation. A minimum of 6 particles were chosen in each condition.

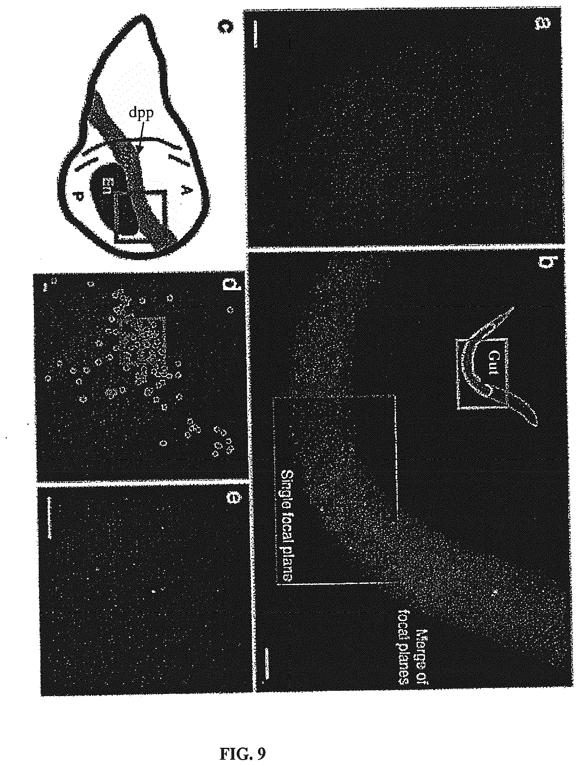

Figure 9 shows imaging localized mRNAs in *C. elegans* and *D. melanogaster.* FIG. 9A is an illustration of *elt-2* mRNA molecules (red) in an early stage embryo (∼100 cell stage) from *C*. *elegans;* the nuclei have been counterstained with DAPI (blue). FIG. 9B is an illustration of *elt-2* mRNA molecules in an L1 larva from *C. elegans.* Inside the blue box, a single focal plane is shown in which the intestinal track is visible. FIG. 9C illustrates a schematic depiction of *dpp* and *engrailed* expression in the imaginal wing discs of third instar larvae from *D. melanogaster.* FIG. 9D is an image showing the locations of the computationally identified *dpp* mRNA molecules (light blue circles) and Engrailed expression detected by immunofluorescence (dark blue). FIG. 9E is an image containing enhanced *dpp* mRNA molecule signals (light blue) and Engrailed protein expression detected by immunofluorescence (dark blue). All images except the boxed portion of FIG. 9B are maximum merges of a z-stack of fluorescent images, and all scale bars are 5 µm long.

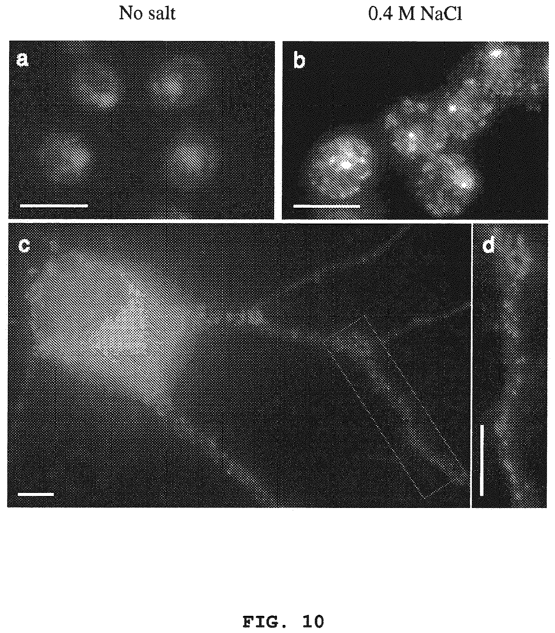

Figure 10 shows imaging single mRNA molecules in yeast and neurons. FIG. 10A and FIG. 10B illustrate *STL1* mRNA particles in both unperturbed cells (FIG. 10A) and cells subjected to a 10 minute 0.4M NaCl salt shock, with nuclear DAPI counterstaining in purple (FIG. 10B). FIG. 10C illustrates expression of β-actin (green) and Map2 (red) mRNAs in rat hippocampus neurons in a dissociated neuron culture. FIG. 10D illustrates an enlarged and contrasted image of a segment of a dendrite enclosed by the red box in FIG. 10C. All scale bars are 5 µm long.

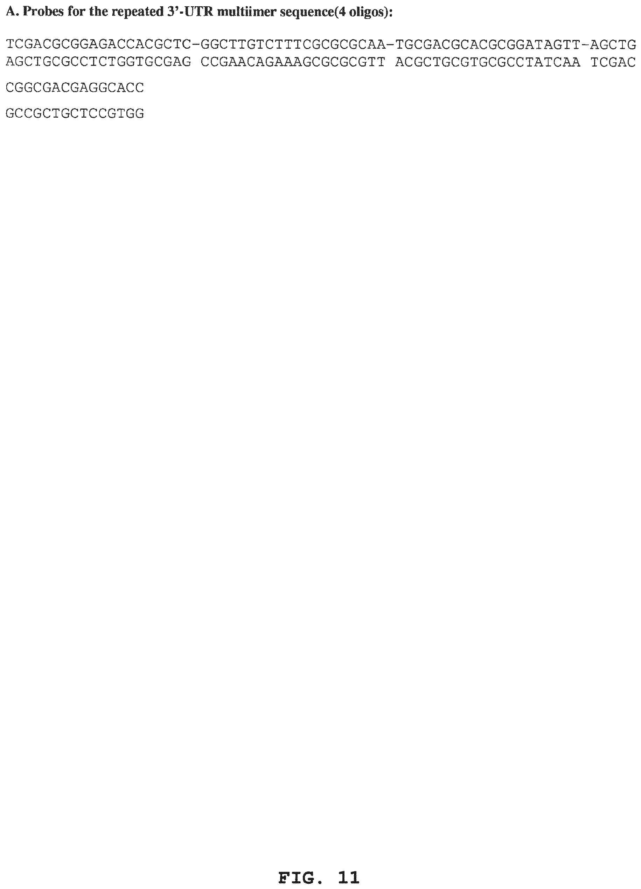

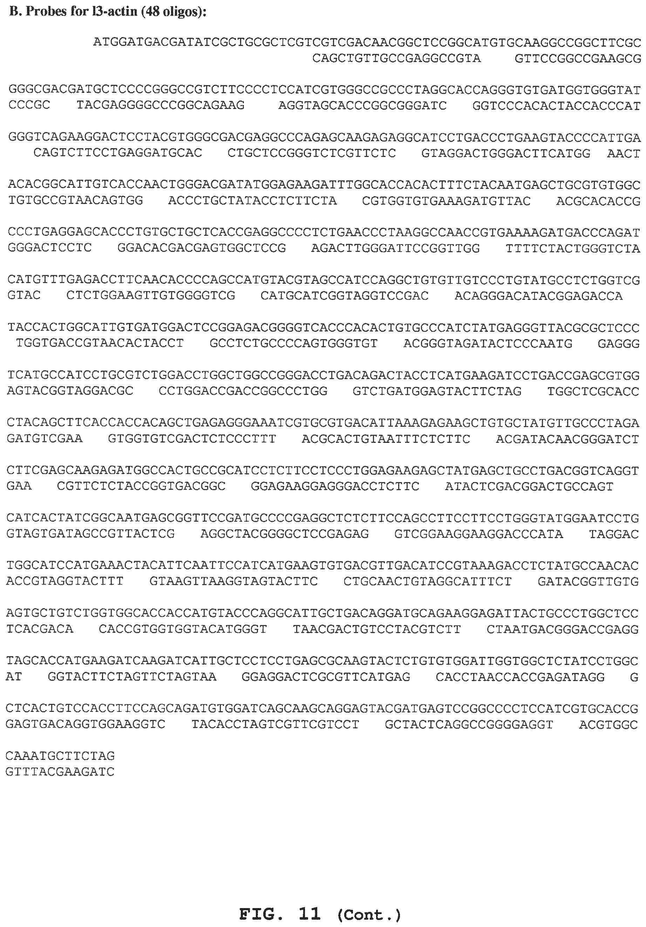

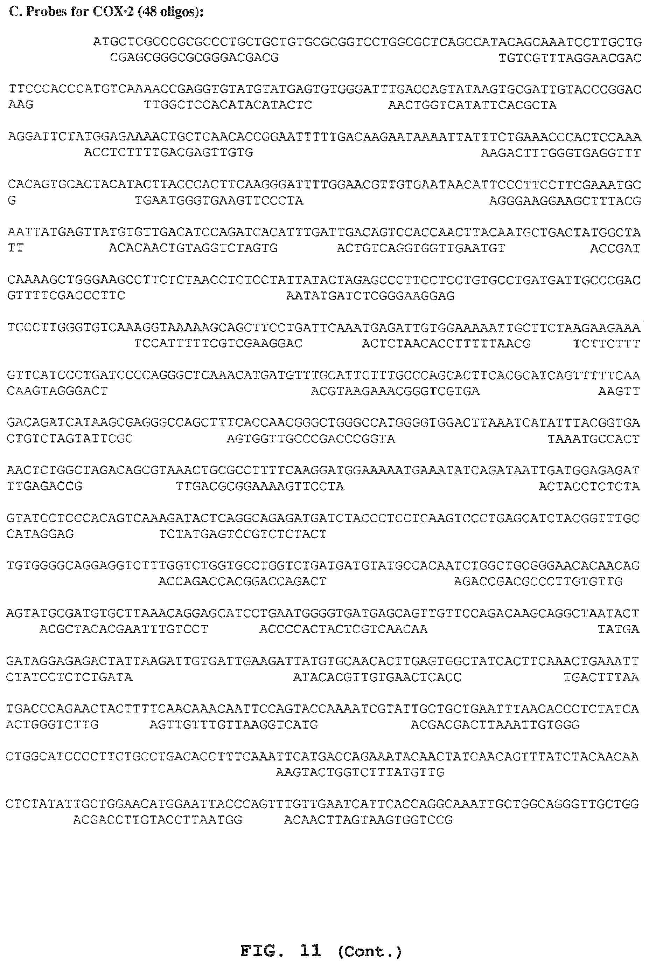

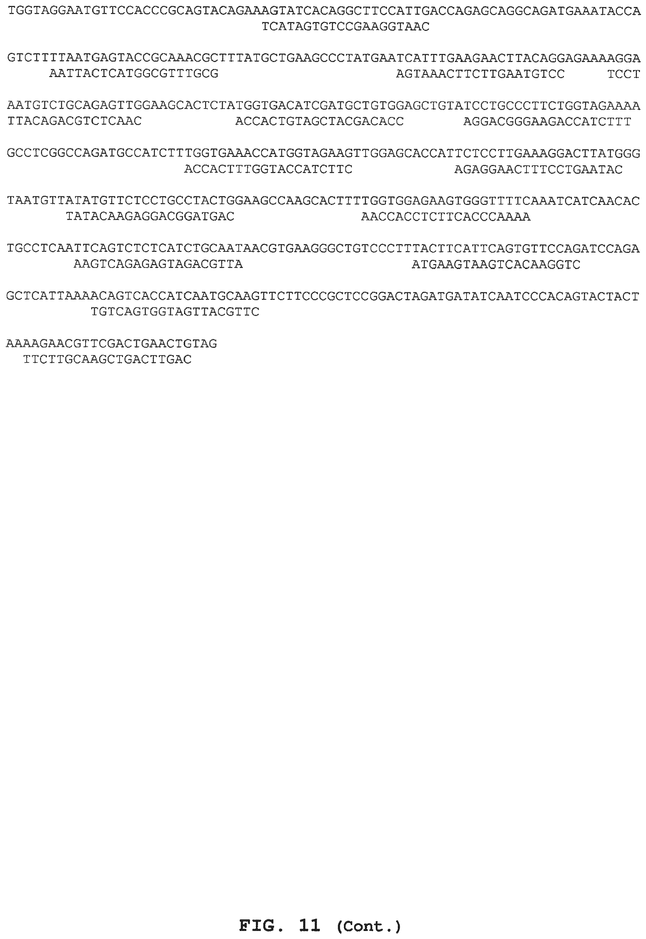

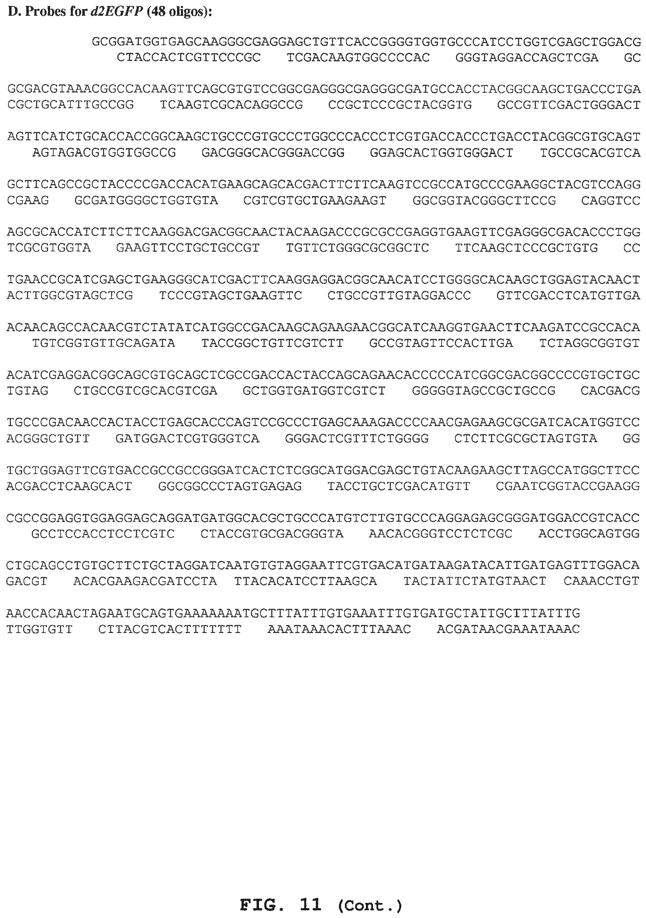

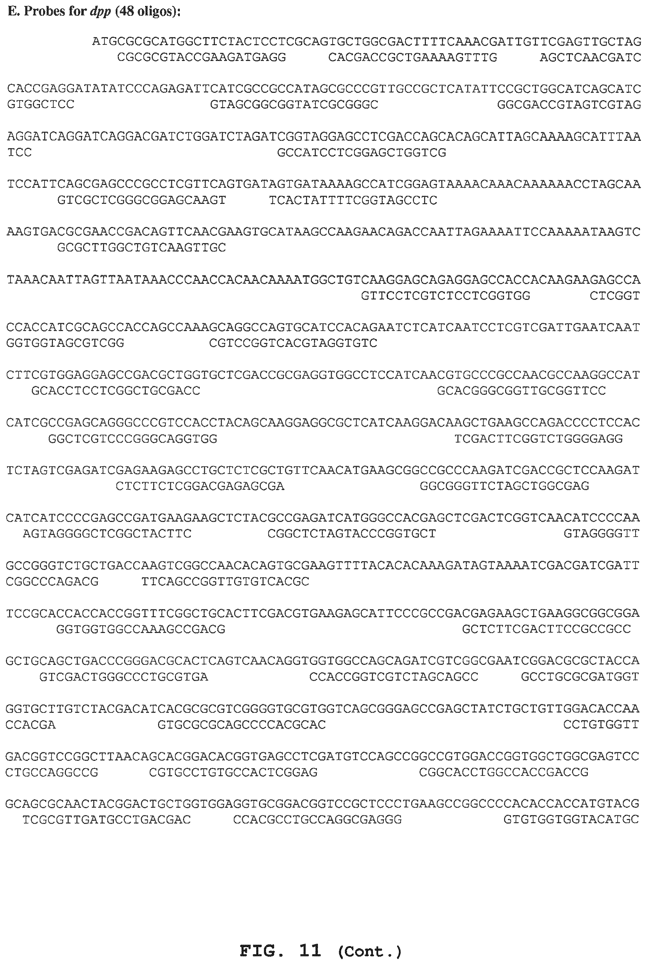

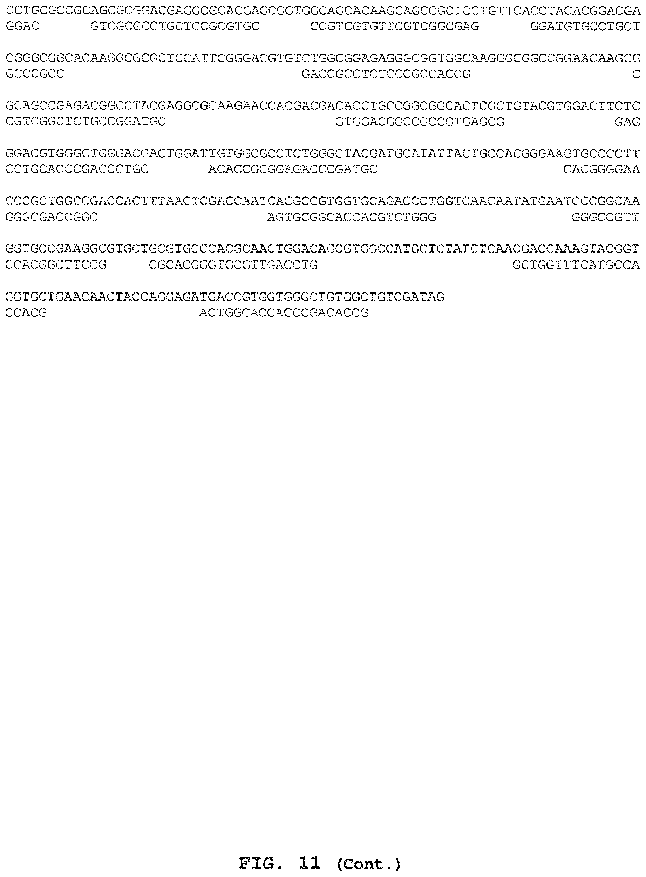

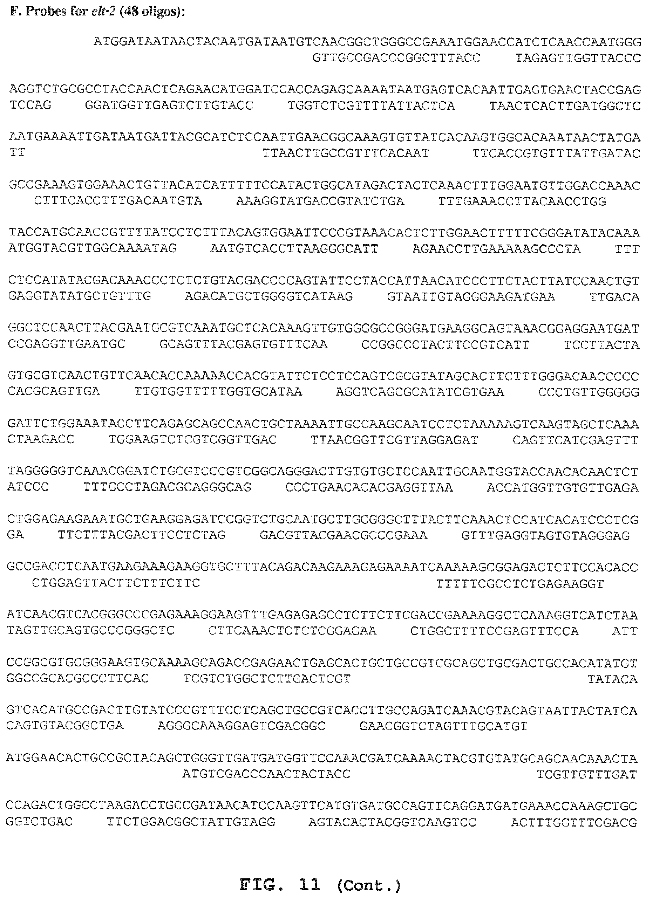

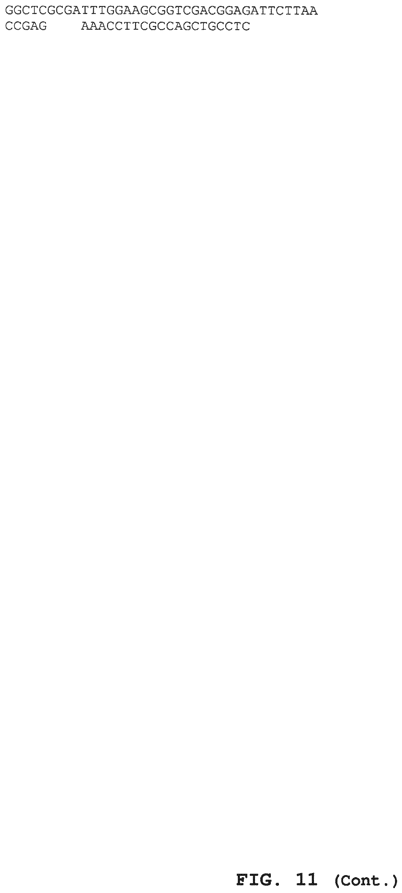

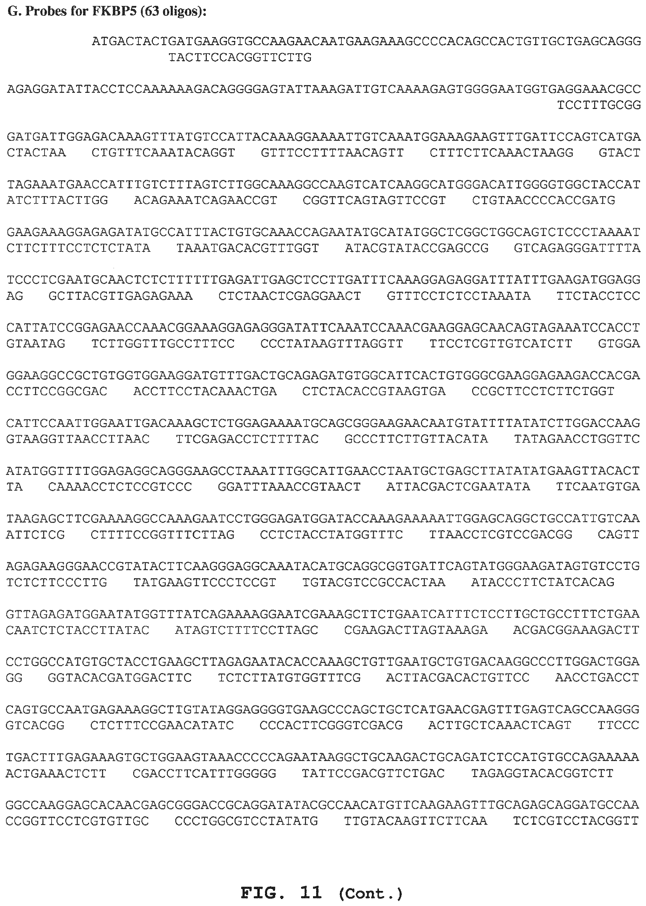

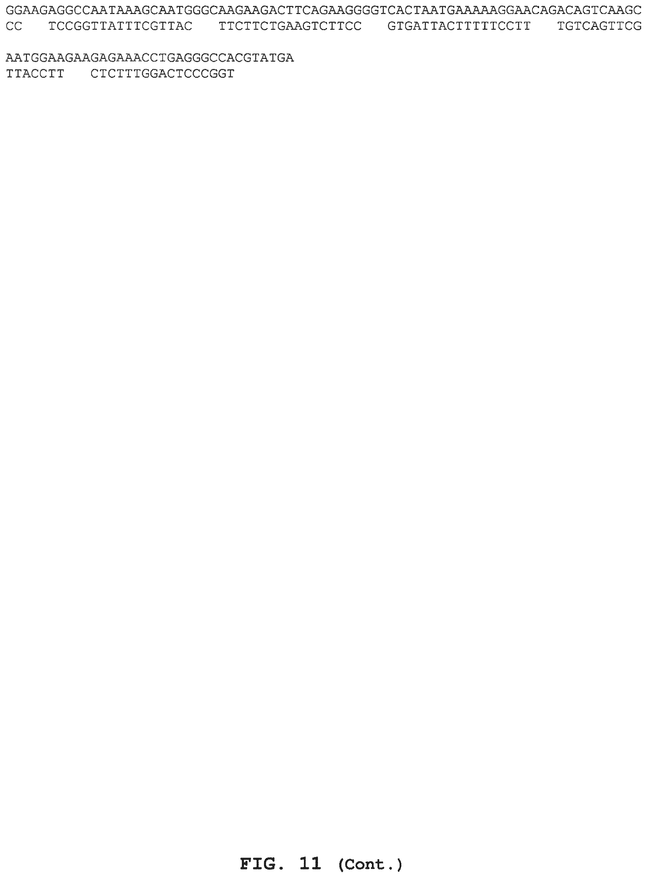

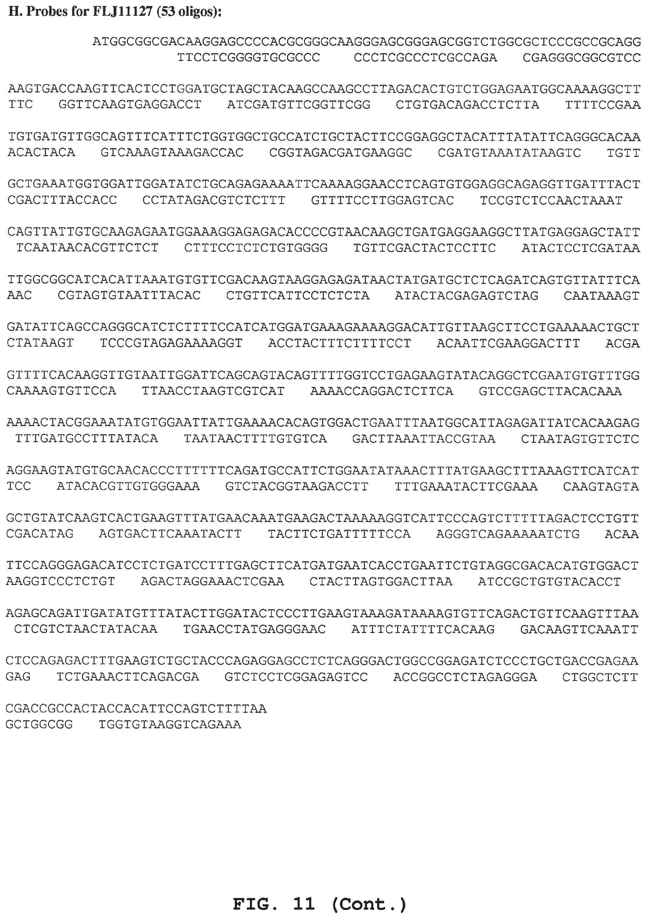

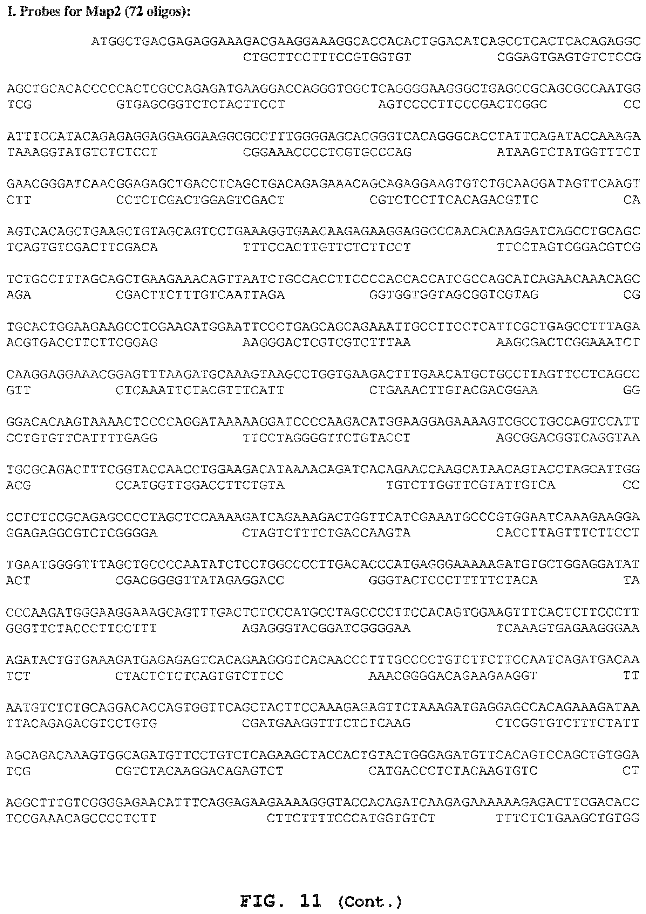

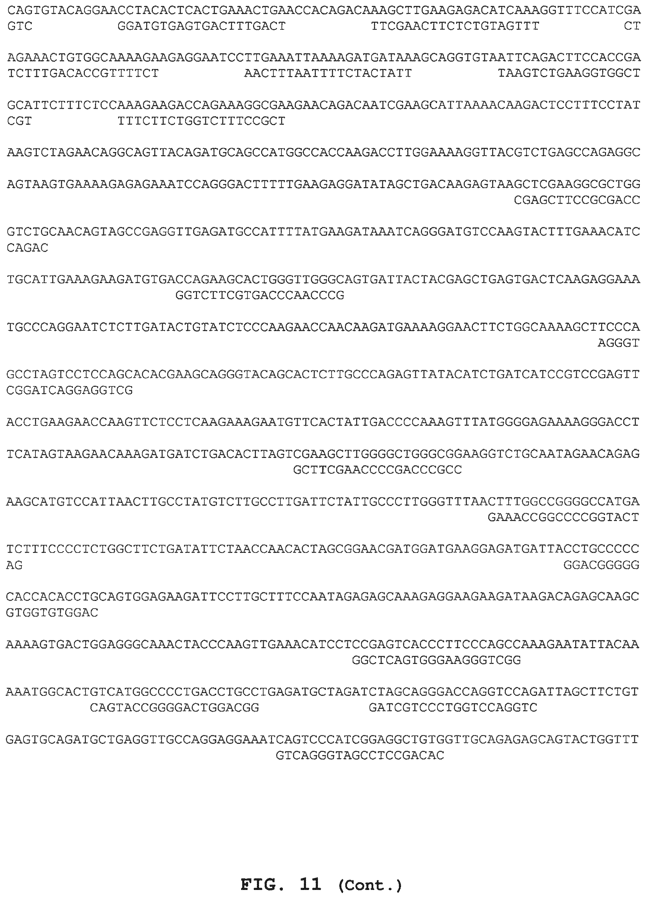

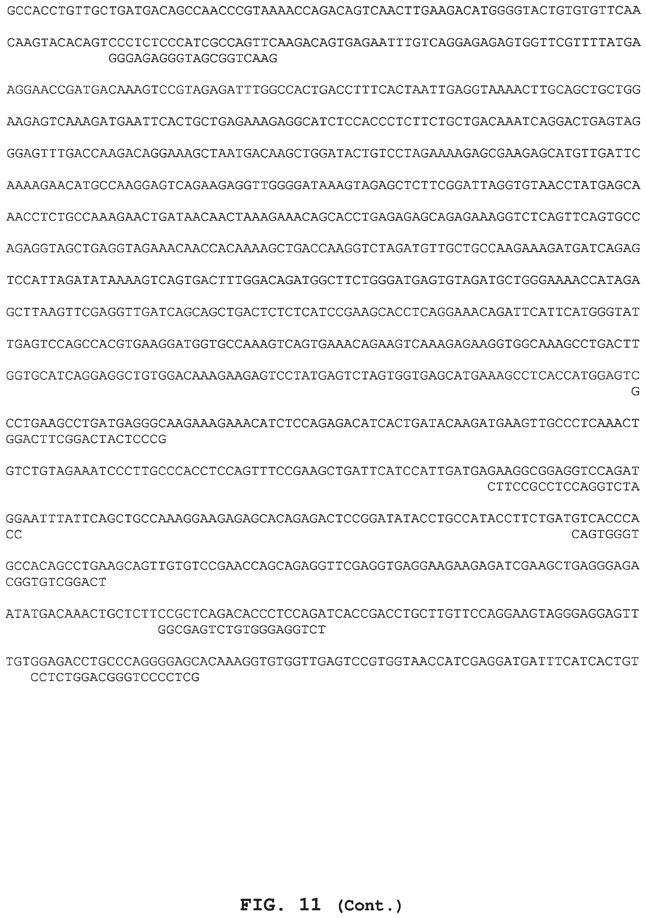

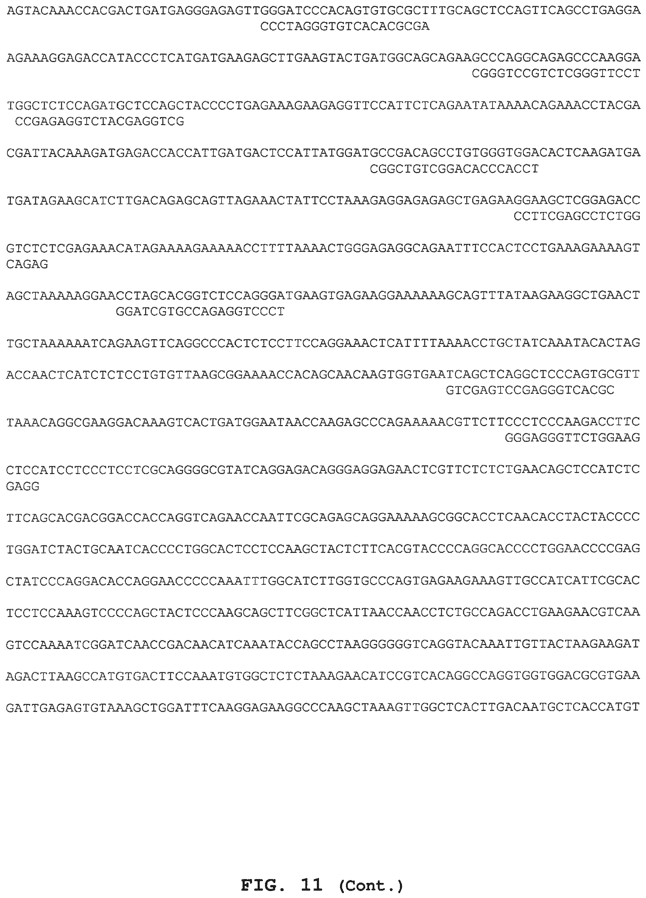

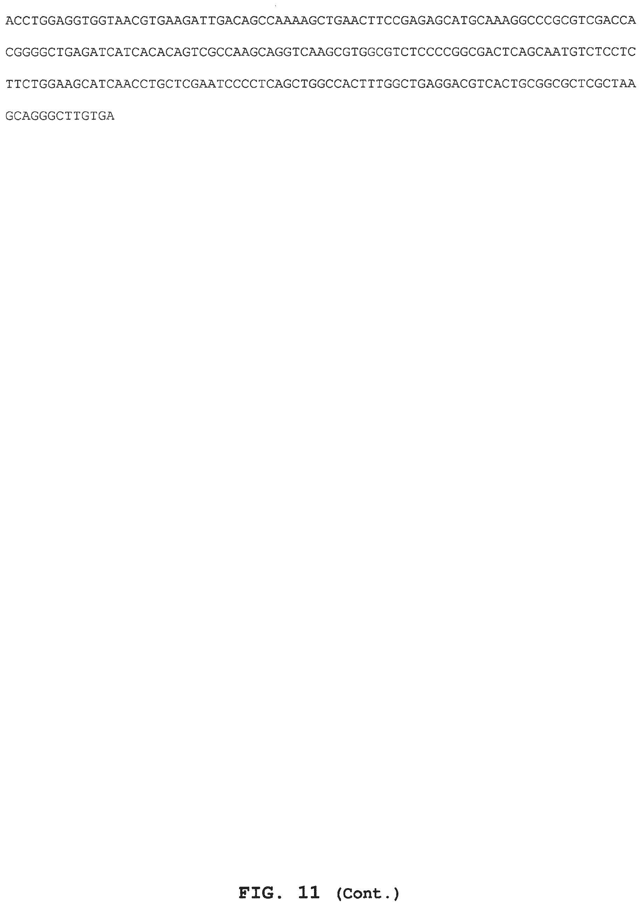

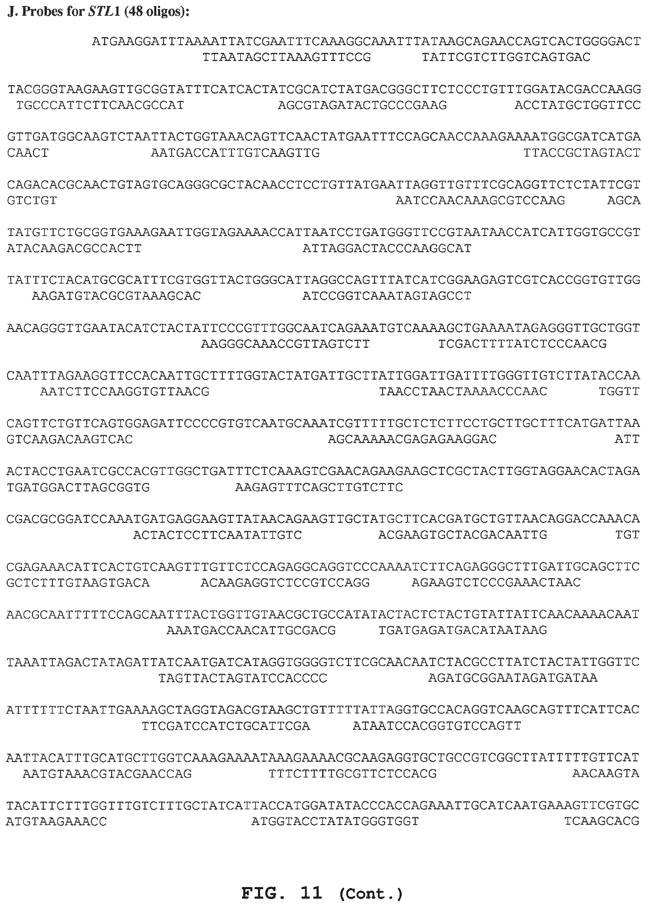

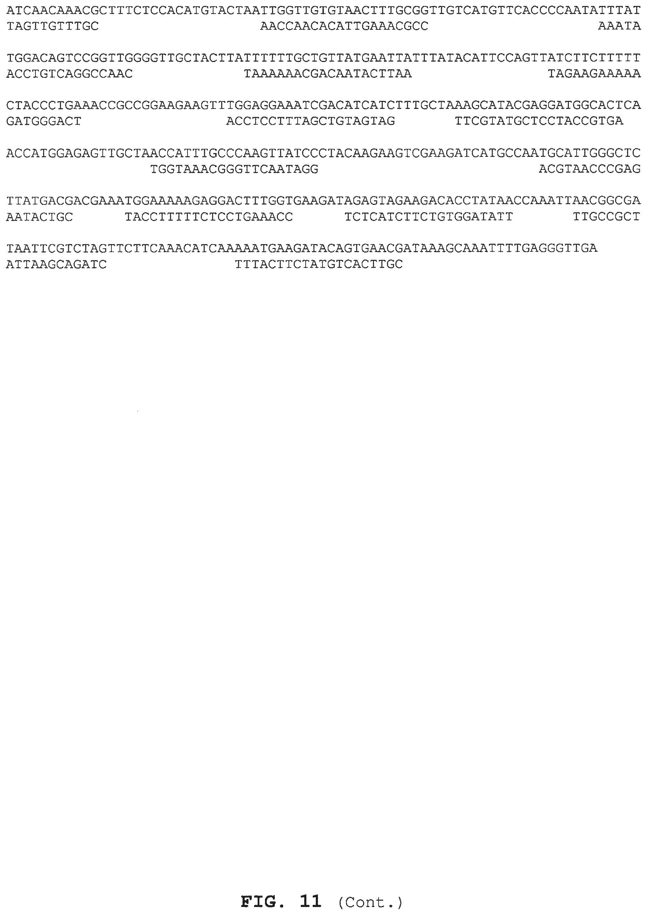

Figure 11 is an illustration of target sequences and probes used in the instant invention.

### Detailed description of the invention

This invention relates in part to the development of an image analysis algorithm that utilizes a principled thresholding strategy and shows that we can accurately and unambiguously identify and count all the target mRNA molecules present in the cell. The simplicity and robustness of this approach permits reliable detection of three different mRNA species within the same cells. Using a rigorous set of criteria the inventors have demonstrated that the method allows extremely specific single mRNA imaging across a wide spectrum of cell types and model organisms.

The inventors have taken advantage of the availability of 96 well DNA synthesizers to synthesize many different terminally labeled smaller probes for the same target. The obtained results show that when a set of at least 30, preferably at least 40, more preferably, about 48 (half of a 96-well plate that is used for high throughput DNA synthesis) or more singly labeled probes bind to the same mRNA molecule, they render it sufficiently fluorescent that it becomes visible as a diffraction-limited spot in wide-field microscopy. The non-specific sites only associate with one or a few probes, yielding diffused signals, whereas the legitimate targets bind to all or most of the probes yielding a clearly detectable spot for each mRNA molecule.

The inventors have also developed an image analysis algorithm that utilizes a principled thresholding strategy and shows that it is possible to accurately and unambiguously identify and count the all target mRNA molecules present in the cell. The simplicity and robustness of this approach permits reliable detection of three different mRNA species within the same cells. Using a rigorous set of criteria the inventors demonstrate that the method allows extremely specific single mRNA imaging across a wide spectrum of cell types and model organisms.

Thus, 48 or more singly labeled oligonucleotide probes allow the detection of individual mRNA molecules. The mRNA molecules were visualized as diffraction limited spots that can be easily detected in a standard wide-field microscopic set up. The spots were bright enough to be accurately counted with the spot detection image processing algorithm of the instant invention. The inventors obtained quantitative counts of three different species of mRNA molecules within individual cells. Such analysis facilitates accurate multiplex gene expression profiling of even lowly expressed genes across a host of model organisms.

The basis of specificity of the instantly disclosed system is that most or all of the probes bind to the intended target mRNA and yield a particulate signal whereas the non-specific binding sites elsewhere in the cell associate with fewer probe molecules and give a diffused signal that the spot counting algorithm ignores. This highlights a key advantage of the instant method over other *in situ* hybridization methods that use heavily labeled probes such as dendrimers. If every probe molecule is detectable, each non-specific binding event will result in a false positive and any mRNA to which the probe does not bind will result in a false negative. The likelihood of false negatives and positives decreases, however, as the number of probes is increased, and in general, given a certain efficiency of hybridization, increasing the number of different probes will narrow the distribution of probes bound per molecule. The image analysis according to the instant invention showed that increasing the number of the probes resulted in robust spot detection that does not depend on arbitrarily chosen thresholds. This is crucial for accurately counting the number of mRNAs per cell, which is a key feature of the method of the invention.

In a related point, a potential factor in the design of the probe set is uniformity in hybridization affinities. Since oligonucleotide affinity is largely dominated by its relative GC content, the inventors have created a computer program to design a set of probes with optimally uniform overall GC content. This computer program is publicly available.

From a practical standpoint, the instantly claimed method also yields significant benefits over previous single molecule mRNA FISH method both in terms of time and cost. Due to advances in synthesis, researchers can easily and cheaply purchase large numbers of oligonucleotides with 3' amine modifiers. These can then be pooled, coupled, and purified en-masse, significantly reducing the labor associated with the multiple couplings and purifications required to generate multiply labeled probe. The resulting simplicity and cost-effectiveness of the instant method will facilitate genomics-scale studies involving the detection of many different mRNAs. Furthermore, the flexibility of the hybridization procedure allows for it to be combined with other standard techniques, such as immunofluorescence.

In another embodiment, the fluorophores can be incorporated into the probes during automated DNA synthesis.

Other methods for quantifying the number of mRNAs in individual cells include single-cell RT-PCR and digital RT-PCR. One problem with these methods is the practical difficulties associated with assembling large numbers of individual reactions that require the use of microfluidic or robotic devices. Moreover, those methods suffer from concerns about stochastic variations in exponential amplification when the target inputs are just a few molecules. Such stochastic behavior complicates the analysis of single cell gene expression, which itself is subjected to stochastic forces. Moreover, these methods do not provide any information about the spatial location of the mRNAs.

Given the simplicity and broad applicability of our single-molecule mRNA detection method, such method is suitable for a variety of studies. By obtaining exact mRNA counts in individual cells, one can make accurate determinations of both expression differences in different conditions and the cell-to-cell variability in gene expression. By yielding quantitative, spatial measurements of individual mRNAs in single cells, this method is valuable in many studies in systems biology, cell biology, neurobiology and developmental biology.

Accordingly, this method may be utilized for multiple assays, including, without limitation a screening assay. In one embodiment, the screening assay determines whether a test compound affects an amount of a distribution of a target sequence of messenger ribonucleic acid molecules (mRNA's) said target sequence including at least 30 non-overlapping probe binding regions of 15-100 nucleotides in a cell. The assay generally comprises the following steps: incubating a cell with a test compound for a period of time sufficient to elicit a response; permeabilizing the cell; immersing said cell in an excess of at least 30 nucleic acid hybridization probes, each singly labeled with the same fluorescent label and each containing a nucleic acid sequence that is complementary to a different probe binding region of said target sequence; washing said fixed cell to remove unbound probes detecting an amount of a distribution of fluorescence from said probes, comparing said amount or said distribution with an amount of a distribution, respectively, obtained from a control cell, treated as described above, but with the exception of being incubated with the test compound.

Suitable test compound candidates include, without limitation, peptide-based compounds (e.g., antibodies or nanobodies), RNA interference agents (i.e., siRNA, shRNA, miRNA etc), and small molecules. All these compounds may be made according to the methods known in the art. For example Naito (US 20080113351) and Khvorova (US 20070031844) provide methods of selecting active RNA interference compounds. Antibodies may also be prepared by known techniques including the use of hybridomas, selection of monoclonal antibodies, use of phage display libraries, antibody humanization and the like.

Small molecule compounds may be selected from screening of the appropriate libraries. In one aspect, small molecule libraries are synthesized according to methods well known and routinely practiced in the art. See, for example, Thompson and Ellman, Chem. Rev. 1996, 96, 555-600, Shipps, et al., Proc. Natl. Acad. Sci. USA, Vol. 94, pp. 11833-11838, October 1997, and Combinatorial Library Design and EvaluationPrinciples, Software Tools and Applications in Drug Discovery, Ghose and Viswanadhan (eds), Marcel Dekker 2001. Alternatively, small libraries are obtained from any of a number of sources including, for example, the NIH Molecular *Libraries* Small Molecule Repository. Alternative sources include AnalytiCon Discovery GmbH (Potsdam, Germany) which makes available MEGAbolite®, pure natural product small molecule *libraries* and NatDiverse™, semi-synthetic natural product analogue small molecule *libraries;* Quantum Pharmaceuticals Ltd. (Moscow, Russian Federation); and Praecis Pharmaceuticals Incorporated (Waltham, Mass.).

In yet another aspect, the invention provides software implementing the thresholding algorithm as described above. Thus, in one embodiment, provided is a computer readable medium, comprising instructions for: obtaining a 3-D stack of 2-D fluorescent images; filtering said 3-D stack using a 3-D filter; counting a total number of 3-D spots in said filtered 3-D stack for each of a plurality of intensity thresholds; obtaining an optimum intensity threshold representative of a plateau region in a plot of said total number of 3-D spots verses the intensity threshold at which said total number was counted; and using the total number of 3-D spots obtained at said optimum threshold as representative of a number of fluorescing particles detected in said 3-D stack.

In one embodiment, the thresholding is accomplished using three dimensional linear Laplacian of Gaussian filter.

In another aspect, a kit is provided. The kit comprises a computer-readable media implementing the thresholding algorithm, as described above, and a set of probes against a pre-selected target sequence. The probes described in connection with the claimed method are also suitable for the instant kit.

Specific embodiments according to the methods of the present disclosure will now be described in the following examples. The examples are illustrative only, and are not intended to limit the remainder of the disclosure in any way.

### EXAMPLES

### Example 1: Materials and Methods

The procedures described in this section are applicable to all examples unless indicated otherwise.

### Probe Design

The sets of probes were designed to consist of at least 48 oligonucleotides each with lengths varying from 17 to 22 nucleotides long with a 3'-amine modification (FKBP5, FLJ11127, and Map2 mRNAs were probed using 63, 53 and 72 oligonucleotides respectively). Additionally, the GC content of the oligonucleotides was kept close to 45% when possible. The oligonucleotides were pooled and coupled to a fluorophore in a single reaction, after which the uncoupled oligonucleotides and remaining free fluorophores were removed by HPLC purification.

### Fluorescence in situ hybridization

In preparation for FISH, all samples were fixed with 3.7% formaldehyde and permeabilized with ethanol. The hybridization was performed using buffers and conditions similar to those outlined by Femino et al., with the key difference being the stringency of the hybridization, which was lowered by reducing the amount of formamide used to 10%. The concentration of the probe that gave optimal signal was determined empirically.

### Imaging and data analysis

All images were acquired using a standard wide-field fluorescence microscope. Computer-aided detection and counting of particles was performed with linear filters designed for enhancing particulate signals.

### Example 2: Probing repeated and unique sequences present in the same mRNA molecule

Utilizing small oligonucleotide probes labeled with a single fluorophore moiety, the inventors have shown that individual mRNA molecules that were engineered to contain 32-96 tandem copies of a probe-binding sequence can be detected by *in situ* hybridization. The inventors also demonstrated that the individual spots in the image represent single mRNA molecules, utilizing a number of different approaches, including correlating the average mRNA copy number obtained by directly counting the diffraction-limited spots to a measurement of the number of target molecules obtained by real-time RT-PCR. Thus, if many different probes are utilized, each targeted to a distinct region of a natural mRNA, it would be possible to obtain single-molecule sensitivity without resorting to the use of engineered genes.

For the initial test of this hypothesis, the inventors constructed a doxycycline-controlled gene that produced an mRNA encoding green fluorescent protein and possessed 32 tandemly repeated 80 nucleotide-long sequences in its 3 '-UTR; and then this engineered gene was stably integrated into the genome of a Chinese hamster ovary cell line. The mRNA expressed from this gene was probed simultaneously with 48 different oligonucleotides, each complementary to a unique region in the coding sequence, and a set of four oligonucleotides, each having a complementary sequence in the repeated motif (a total of 128 probes bound) (**FIG**. **1A**). Each oligonucleotide in the probe set that was specific for the coding sequence was labeled with a single Alexa-594 fluorophore, and each oligonucleotide in the set specific for the repeat sequence was labeled with a single tetramethylrhodamine (TMR) fluorophore. The use of appropriate filter sets ensured that the fluorescence emitted from TMR fluorophores was not detected in the Alexa-594 channel and vice versa, as described below.

After performing FISH with these probes, the inventors have found that many "particles" with a diameter of about 0.25 micrometers were visible in both the TMR and Alexa-594 channels (**FIG. 1B**). The particles were identified computationally using an image processing program (described in the next section) that categorizes the particles as being labeled with either the GFP-coding-sequence probes (TMR), the UTR-specific probes (Alexa-594), or both (**FIG. 1C**). Upon identifying and localizing particles in four fields of view similar to the ones shown in Figure 1c, a total of 599 particles corresponding to GFP-coding sequence-specific probes and 565 particles corresponding to the UTR-specific probes were counted. Of these particles, 85% of the "UTR particles" co-localized with the "GFP particles," whereas 81% of the GFP particles co-localized with the UTR particles. The high degree of co-localization between particles detected by the previously established tandem repeat detection method and the particles detected via simultaneous probing with 48 different singly-labeled oligonucleotides demonstrates the validity of using multiple single-labeled probes for the detection of endogenous transcripts. The fraction of particles that did not display co-localization likely correspond to mRNA molecules that lost either their coding sequence or their 3'-UTR in the natural processes of mRNA degradation.

The inventors also analyzed the fluorescent intensity of the co-localized spots in both the TMR and Alexa-594 channel and found that the spot intensities displayed a unimodal distribution (**FIG. 2**), arguing that the particles detected are not clumps of many mRNAs but rather individual molecules. The spot intensities displayed a strong correlation between the two channels (**FIG. 3**). Since there is no cross talk between the two channels, this indicates that the variability in spot intensity was not primarily due to random variability in probe hybridization (which would be uncorrelated between different probe sets) but rather other factors, such as mRNA integrity or accessibility, that affect both probes equally.

The inventors also explored how the signal intensity would vary with the number of probes by performing *in situ* hybridization using either first 12, 24, 36 probes or all 48 probes in the set. For this particular target mRNA, it was found that particles could be detected with fewer numbers of probes, albeit with decreased intensity (FIG. 3A). However, the automatic spot detection algorithm (described in details below) performed particularly well with 48 probes, detecting the same number of spots over a broad range of thresholds (**FIG. 3B**, see further discussion below). The number of probes required for robust signal is likely to depend on the target sequence, though, as the inventors have obtained clear mRNA signals using as few as 30 probes. When the instant method was compared to the method of Femino et al. by using a 45 bp long oligonucleotide labeled with 5 fluorophores and complementary to a sequence repeated 32 times in the 3'UTR of a gene, potentially yielding 160 fluorophores per mRNA (**FIG. 4A**), it was found that the signal to background were about the same in both methods (**FIG. 4B**), indicating that the instantly claimed method is at least as sensitive despite using fewer fluorophores.

Moreover, CHO cells lacking the reporter gene yielded no signals while CHO cells having the reporter gene that was turned off by addition of doxycycline, yielded mRNA particles in only a few cells, indicating that the signals observed were specific.

### Example 3: Computational algorithm for spot detection

In order to reliably identify large numbers of mRNA molecules, the inventors developed a semiautomated computational algorithm for finding spots in a three-dimensional stack of fluorescent images. One of the difficulties associated with spot detection is the nonuniform background arising from cellular autofluoresence and low levels of non-specific probe hybridization. To circumvent these issues, the inventors filtered image stacks using a three dimensional linear Laplacian of Gaussian filter designed to enhance spot-like signals of the correct size and shape (**FIG. 5A and FIG. 5B**) while removing the slowly varying background. In the next step in the algorithm, the inventors applied a threshold to the filtered image in order to define the spots. In order to make a rational choice of threshold, the number of spots in three dimensions for all thresholds ranging from zero to the maximum pixel intensity in the filtered image was counted. When the inventors plotted the number of particles as a function of the threshold, a wide plateau was found, indicating that there is a region over which the number of particles detected is fairly insensitive to the particular threshold chosen (**FIG. 5C**). When a threshold in this region is chosen, the spots detected correspond very well with those identified by eye, demonstrating the efficacy of the spot detection algorithm (**FIG. 5D**).

### Example 4: Gene expression profiling of three different mRNA species

A potential use of the instantly claimed method is the simultaneous detection of single molecules of multiple mRNAs in individual cells. To demonstrate this capability, the inventors designed probes specific to three mRNAs encoding FK506 binding protein 5 (FKBP5), Cox-2 and FLJ11127 in the human carcinoma cell line A549. These probes were coupled to the spectrally distinct fluorophores Cy5, Alexa 594 and TMR, respectively. Upon performing FISH with all three probes simultaneously, individual spots were visible in the three different fluorescence channels (**FIG. 6A****-****FIG. 6F**); an intensity analysis showed that fluorescent spots did not bleed through into other channels (**FIG. 7**).

To demonstrate that the claimed method of mRNA detection was specific and quantitative, the cells were incubated with the cell-permeable glucocorticoid dexamethasone, thus upregulating the expression of FKBP5 and FLJ1127 while mildly downregulating the expression of Cox-2 in this particular cell-line. The inventors found that the mean number of FKBP5 and FLJ1127 mRNAs measured by combining FISH with the instantly disclosed spot detection algorithm increased while the mean number of Cox-2 mRNAs decreased (compare **FIG. 6A****-****FIG. 6C to FIG. 6D****-** **FIG. 6F**). These numbers corresponded well to RT-PCR measurements of the fold induction and repression of these genes performed on the same samples, demonstrating that the fluorescent spots are the appropriate mRNAs and that a majority of the mRNA molecules (**FIG. 6G**) was detected using the instantly claimed methods. Moreover, this further demonstrates the effectiveness of the spot detection method for accurate gene expression quantification.

One technical challenge that arose in imaging multiple mRNAs simultaneously was fluorophore photolability, particularly in the case of Cy5. In order to image all of the mRNA molecules within a single cell, 10 to 30 "z-section" images for each visual field were acquired, utilizing a one-to-three second exposure for each image and a high numerical aperture objective. Only TMR and (to a lesser extent) Alexa-594 could withstand this intense and relatively prolonged exposure to light; Cy5, for instance, proved extremely photolabile under these conditions (**FIG. 8**). To overcome this problem, the inventors employed a special mounting medium in which fluorophores are much more photostable. This method was adapted from Yildiz *et al.* with minor modifications. In this medium, a mixture of catalase, glucose oxidase, and glucose enzymatically removes molecular oxygen from the medium, thereby inhibiting oxygen-dependent, light-initiated pathways that destroy fluorophores. The use of these enzymes lead to a dramatic 10-fold enhancement of Cy5 photostability while not adversely affecting the imaging of TMR and Alexa-594, thus facilitating the acquisition of multiple z-sections when performing three color imaging.

### Example 5: mRNA detection in model organisms and cell types

One of the canonical uses for *in situ* hybridization has been for the detection of mRNA localization during development. The inventors tested the instantly claimed method for efficacy in two commonly studied developmental systems: the nematode, *Caenorhabditis elegans,* and the fruit fly, *Drosophila melanogaster.* In the nematode, the inventors constructed probes to detect mRNA molecules from the gene *elt-2,* a transcription factor that is expressed only in the nematode gut, and only after the nematode embryo has developed to the 45-cell stage. After hybridization of the probe set to both embryos and larvae, it was found that *elt-2* mRNA molecules were present only within the gut region (**FIG. 9A**) of both the embryos and the larvae (**FIG. 9B**). However, consistent with the known timing of the onset of expression, *elt-2* mRNAs were only detected in the gut of embryos older than the 45-cell stage, again highlighting the specificity of the instantly claimed method. Furthermore, at those early stages, only a few transcripts were detected, showing that this method is sensitive enough to detect even small numbers of transcripts in complex tissues.

In the fruit fly, one of the most well-studied examples of the localization of gene expression occurs in wing imaginal disc development. The wing discs of fruit fly larvae display a remarkable set of gene expression patterns, one of which is the formation of a stripe of expression of the gene *dpp* in response to gradients of the proteins Hedgehog and Engrailed. In particular, Engrailed, which negatively regulates *dpp* mRNA synthesis, is high in the posterior compartment of the wing disc and low in the anterior compartment of the wing disc. Similarly, Hedgehog, which positively regulates *dpp* mRNA synthesis, is high in the posterior compartment of the wing disc and low in the anterior compartment of the wing disc. However, there is a region between the posterior and the anterior where the levels of Hedgehog is high enough to activate *dpp* but not high enough to activate *engrailed,* resulting in the synthesis of *dpp* mRNA in a narrow stripe (**FIG. 9C**).

To check whether this narrow stripe of *dpp* mRNA synthesis can be imaged, the inventors constructed a set of singly labeled probes against *dpp* mRNA and performed *in situ* hybridization on imaginal wing discs isolated from third-instar larvae. Moreover, this *in situ* procedure was combined with immunofluorescence against Engrailed protein (shown in blue). **FIG. 9D** shows a full image, in which the locations of the algorithmically identified mRNA molecules are presented as blue circles; and **FIG. 9E** shows an enlarged portion of the image with enhanced mRNA signals. The images show that mRNA molecules were found only at the anterior edge of the area of Engrailed expression, again confirming the specificity of detection.

The inventors also tested the instantly claimed method in *Saccharomyces cerevisae* by designing a set of probe to target transcripts from the gene *STL1. STL1* is one among a number of yeast genes whose expression is significantly up-regulated by the addition of salt to the growth medium. It was found that non-shocked cells contain virtually no *STL1* mRNA molecules (**FIG. 10A**), while cells subjected to a ten minute 0.4 M salt shock possessed a large numbers of *STL1* mRNA molecules (**FIG. 10B**).

Another cell type in which mRNA localization is commonly studied is neurons. To show efficacy of the instantly claimed method in that system the inventors imaged β-actin mRNA and Map2 mRNA in cultured hippocampal neurons. **FIG. 10C** shows that a β-actin probe set (labeled with TMR) and a differently colored Map2 probe set (labeled with Alexa-594) can be used to image and distinguish their targets with a single molecule resolution. A fraction of these mRNAs migrate to distant reaches of dendrites (**FIG. 10D**). Particle counts indicated that 14% of the 791 β-actin mRNA molecules were located in dendrites, whereas 37% of the 140 Map2 mRNA molecules were located in the dendrites, which is similar to the previously reported distributions.

### References

1. Kaufmann, B.B. & van Oudenaarden, A. Stochastic gene expression: from single molecules to the proteome. Curr Opin Genet Dev 17, 107-112 (2007).
2. St Johnston, D. Moving messages: the intracellular localization of mRNAs. Nat Rev Mol Cell Biol 6, 363-375 (2005).
3. Gall, J.G. Differential synthesis of the genes for ribosomal RNA during amphibian oogenesis. Proc Natl Acad Sci U S A 60, 553-560 (1968).
4. Levsky, J.M. & Singer, R.H. Fluorescence in situ hybridization: past, present and future. J Cell Sci 116, 2833-2838 (2003).
5. Tautz, D. & Pfeifle, C. A non-radioactive in situ hybridization method for the localization of specific RNAs in Drosophila embryos reveals translational control of the segmentation gene hunchback. Chromosoma 98, 81-85 (1989).
6. Raap, A.K. et al. Ultra-sensitive FISH using peroxidase-mediated deposition of biotin- or fluorochrome tyramides. Hum Mol Genet 4, 529-534 (1995).
7. Femino, A.M., Fay, F.S., Fogarty, K. & Singer, R.H. Visualization of single RNA transcripts in situ. Science 280, 585-590 (1998).
8. Tsokas, P. et al. Local protein synthesis mediates a rapid increase in dendritic elongation factor 1A after induction of late long-term potentiation. J Neurosci 25, 5833-5843 (2005).
9. Maamar, H., Raj, A. & Dubnau, D. Noise in gene expression determines cell fate in Bacillus subtilis. Science 317, 526-529 (2007).
10. Femino, A.M., Fogarty, K., Lifshitz, L.M., Carrington, W. & Singer, R.H. Visualization of single molecules of mRNA in situ. Methods Enzymol 361, 245- 304 (2003).
11. Randolph, J.B. & Waggoner, A.S. Stability, specificity and fluorescence brightness of multiply-labeled fluorescent DNA probes. Nucleic Acids Res 25, 2923-2929 (1997).
12. Sindelar, L.E. & Jaklevic, J.M. High-throughput DNA synthesis in a multichannel format. Nucleic Acids Res 23, 982-987 (1995).
13. Vargas, D.Y., Raj, A., Marras, S.A., Kramer, F.R. & Tyagi, S. Mechanism of mRNA transport in the nucleus. Proc Natl Acad Sci U S A 102, 17008-17013 (2005).
14. Raj, A., Peskin, C.S., Tranchina, D., Vargas, D.Y. & Tyagi, S. Stochastic mRNA synthesis in mammalian cells. PLoS Biol 4, e309 (2006).
15. Garneau, N.L., Wilusz, J. & Wilusz, C.J. The highways and byways of mRNA decay. Nat Rev Mol Cell Biol 8, 113-126 (2007).
16. Gonzalez, R.C., Woods, R.E. & Eddins, S.L. Digital Image Processing Using Matlab. (Pearson Prentice Hall, Upper Saddle River, NJ; 2004).
17. Wang, J.C. et al. Chromatin immunoprecipitation (ChIP) scanning identifies primary glucocorticoid receptor target genes. Proc Natl Acad Sci U S A 101, 15603-15608 (2004).
18. Yildiz, A. et al. Myosin V walks hand-over-hand: single fluorophore imaging with 1.5-nm localization. Science 300, 2061-2065 (2003).
19. Benson, D.M., Bryan, J., Plant, A.L., Gotto, A.M., Jr. & Smith, L.C. Digital imaging fluorescence microscopy: spatial heterogeneity of photobleaching rate constants in individual cells. J Cell Biol 100, 1309-1323 (1985).
20. Lecuyer, E. et al. Global analysis of mRNA localization reveals a prominent role in organizing cellular architecture and function. Cell 131, 174-187 (2007).
21. Fukushige, T., Hawkins, M.G. & McGhee, J.D. The GATA-factor elt-2 is essential for formation of the Caenorhabditis elegans intestine. Dev Biol 198, 286-302 (1998).
22. Sanicola, M., Sekelsky, J., Elson, S. & Gelbart, W.M. Drawing a stripe in Drosophila imaginal disks: negative regulation of decapentaplegic and patched expression by engrailed. Genetics 139, 745-756 (1995).
23. Rep, M., Krantz, M., Thevelein, J.M. & Hohmann, S. The transcriptional response of Saccharomyces cerevisiae to osmotic shock. Hot1p and Msn2p/Msn4p are required for the induction of subsets of high osmolarity glycerol pathwaydependent genes. J Biol Chem 275, 8290-8300 (2000).
24. Tiruchinapalli, D.M. et al. Activity-dependent trafficking and dynamic localization of zipcode binding protein 1 and beta-actin mRNA in dendrites and spines of hippocampal neurons. J Neurosci 23, 3251-3261 (2003).
25. Blichenberg, A. et al. Identification of a cis-acting dendritic targeting element in MAP2 mRNAs. J Neurosci 19, 8818-8829 (1999).
26. Warren, L., Bryder, D., Weissman, I.L. & Quake, S.R. Transcription factor profiling in individual hematopoietic progenitors by digital RT-PCR. Proc Natl Acad Sci U S A 103, 17807-17812 (2006).

## Claims

1. A probe set for *in situ* hybridization that enables detection of individual mRNA molecules in fixed, permeabilized cells, comprising at least 30 non-overlapping nucleic acid hybridization probes singly labelled with the same first fluorophore label of a first color, wherein the probes have sequences complementary to different probe binding regions of a target sequence of an individual mRNA molecule, wherein said probes are 7-30 nucleotides in length.

2. The probe set of Claim 1 wherein the probe set comprises 40 to 60 non-overlapping nucleic acid hybridization probes singly labelled with the same first fluorophore label of a first color.

3. The probe set of Claim 1 or Claim 2 wherein the probes have target-complementary sequences 15-30 nucleotides in length.

4. The probe set of any preceding claim wherein all probes in the set are labelled with a fluorophore at their 3' end.

5. A hybridization solution that enables detection of individual mRNA molecules in fixed, permeabilized cells comprising an excess of a first set of at least one probe set wherein the probe set comprises at least 30 non-overlapping nucleic acid hybridization probes singly labelled with the same first fluorophore label of a first color, wherein the probes have sequences complementary to different probe binding regions of a first target sequence of an individual mRNA molecule, wherein said probes are 7-30 nucleotides in length.

6. The hybridization solution of Claim 5 wherein the probe set comprises 40-60 non-overlapping nucleic acid hybridization probes singly labelled with the same first fluorophore label of a first color.

7. The hybridization solution of Claim 5 or 6 wherein the hybridization solution may comprise a mixture of more than one set of probes labelled where each set is labelled with a distinct fluorophore.

8. The hybridization solution of any of Claims 5 to 7, wherein each probe is included in the range of 0.2-1.0 nanograms per microliter.

9. A kit that enables detection of individual mRNA molecules in fixed, permeabilized cells comprising:
a) computer readable medium, comprising instructions, which when executed on a computer cause the computer to carry out steps of obtaining a 3-D stack of 2-D fluorescent images; filtering said 3-D stack using a 3-D filter; counting a total number of 3-D spots in said filtered 3-D stack for each of a plurality of intensity thresholds; obtaining an optimum intensity threshold representative of a plateau region in a plot of said total number of 3-D spots verses the intensity threshold at which said total number was counted; using the total number of 3-D spots obtained at said optimum threshold as representative of a number of fluorescing particles detected in said 3-D stack; and
b) the probe set of any one of claims 1-4.

10. The kit of Claim 9, wherein said probe set includes at least 30 probes singly labelled with a first fluorescent label and at least 30 probes labelled with a second fluorescent label.

11. The kit of Claim 9 or 10, wherein said mRNA molecule includes at least two target regions, and the fluorescent labels for each target region are distinguishable.

12. The probe set of any one of claims 1-4, the hybridization solution of any one of claims 5-8, or the kit of any one of claims 9-11, wherein one or more of the probes are DNA probes.

## Patentansprüche

1. Sondensatz für *in*-*situ*-Hybridisierung, der den Nachweis von einzelnen mRNA-Moleküle in fixierten permeabilisierten Zellen ermöglicht, der mindestens 30 nicht überlappende Nukleinsäure-Hybridisierungssonden umfasst, die einzeln mit der gleichen ersten Fluorophormarkierung einer ersten Farbe markiert sind, wobei die Sonden Sequenzen aufweisen, die zu anderen Sonden-bindenden Regionen einer Zielsequenz eines einzelnen mRNA-Moleküls komplementär sind, wobei die Sonden eine Länge von 7-30 Nukleotiden aufweisen.

2. Sondensatz nach Anspruch 1, wobei der Sondensatz 40 bis 60 nicht überlappende Nukleinsäure-Hybridisierungssonden umfasst, die einzeln mit der gleichen ersten Fluorophormarkierung einer ersten Farbe markiert sind.

3. Sondensatz nach Anspruch 1 oder Anspruch 2, wobei die Sonden zum Ziel komplementäre Sequenzen von einer Länge von 15-30 Nukleotiden aufweisen.

4. Sondensatz nach einem vorstehenden Anspruch, wobei alle Sonden in dem Satz mit einem Fluorophor an ihrem 3'-Ende markiert sind.

5. Hybridisierungslösung, die den Nachweis von einzelnen mRNA-Molekülen in fixierten permeabiliserten Zellen ermöglicht, die einen Überschuss eines ersten Satzes von mindestens einem Sondensatz umfasst, wobei der Sondensatz mindestens 30 nicht überlappende Nukleinsäure-Hybridisierungssonden umfasst, die einzeln mit der gleichen ersten Fluorophormarkierung einer ersten Farbe markiert sind, wobei die Sonden Sequenzen aufweisen, die zu anderen Sonden-bindenden Regionen einer ersten Zielsequenz eines einzelnen mRNA-Moleküls komplementär sind, wobei die Sonden eine Länge von 7-30 Nukleotiden aufweisen.

6. Hybridisierungslösung nach Anspruch 5, wobei der Sondensatz 40-60 nicht überlappende Nukleinsäure-Hybridisierungssonden umfasst, die einzeln mit der gleichen ersten Fluorophormarkierung einer ersten Farbe markiert sind.

7. Hybridisierungslösung nach Anspruch 5 oder 6, wobei die Hybridisierungslösung eine Mischung von mehr als einem Satz von markierten Sonden umfassen kann, wobei jeder Satz mit einem unterschiedlichen Fluorophor markiert ist.

8. Hybridisierungslösung nach einem der Ansprüche 5 bis 7, wobei jede Sonde in dem Bereich von 0,2-1,0 Nanogramm pro Mikroliter eingeschlossen ist.

9. Kit, der den Nachweis von einzelnen mRNA-Molekülen in fixierten permeabilisierten Zellen ermöglicht, der Folgendes umfasst:
a) computerlesbares Medium, das Anweisungen umfasst, welche bei Ausführung an einem Computer bewirken, dass der Computer die folgenden Schritte durchführt: Erhalten eines 3-D-Stapels von 2-D-Fluoreszenzbildern; Filtern des 3-D-Stapels unter Verwendung eines 3-D-Filters; Zählen einer Gesamtanzahl von 3-D-Flecken in dem gefilterten 3-D-Stapel für jeden einer Vielzahl von Intensitätsschwellenwerten; Erhalten eines optimalen Intensitätsschwellenwerts, der einen Plateaubereich in einem Diagramm der Gesamtanzahl von 3-D-Flecken gegenüber dem Intensitätsschwellenwert, bei dem die Gesamtanzahl gezählt wurde, repräsentiert; Verwenden der Gesamtanzahl von 3-D-Flecken, die bei dem optimalen Schwellenwert erhalten wurden, als repräsentativ für eine Anzahl von fluoreszierenden Partikeln, die in dem 3-D-Stapel nachgewiesen werden; und
b) den Sondensatz nach einem der Ansprüche 1-4.

10. Kit nach Anspruch 9, wobei der Sondensatz mindestens 30 Sonden, die einzeln mit einer ersten Fluoreszenzmarkierung markiert sind, und mindestens 30 Sonden, die mit einer zweiten Fluoreszenzmarkierung markiert sind, einschließt.

11. Kit nach Anspruch 9 oder 10, wobei das mRNA-Molekül mindestens zwei Zielregionen einschließt und die Fluoreszenzmarkierungen für jede Zielregion unterscheidbar sind.

12. Sondensatz nach einem der Ansprüche 1-4, die Hybridisierungslösung nach einem der Ansprüche 5-8 oder der Kit nach einem der Ansprüche 9-11, wobei eine oder mehrere der Sonden DNA-Sonden sind.

## Revendications

1. Ensemble de sondes destiné à l'hybridation *in situ* qui permet la détection de molécules d'ARNm individuelles dans des cellules fixes, perméabilisées, comprenant au moins 30 sondes d'hybridation d'acide nucléique non chevauchantes à marqueur unique avec le même premier marqueur à fluorophore d'une première couleur, dans lequel les sondes comportent des séquences complémentaires à différentes régions de liaison de sonde d'une séquence cible d'une molécule d'ARNm individuelle, dans lequel lesdites sondes ont une longueur comprise dans la plage allant de 7 à 30 nucléotides.

2. Ensemble de sondes selon la revendication 1, dans lequel l'ensemble de sondes comprend 40 à 60 sondes d'hybridation d'acide nucléique non chevauchantes à marqueur unique avec le même premier marqueur à fluorophore d'une première couleur.

3. Ensemble de sondes selon la revendication 1 ou la revendication 2, dans lequel les sondes possèdent des séquences complémentaires cibles de longueur comprise dans la plage allant de 15 à 30 nucléotides.

4. Ensemble de sondes selon l'une quelconque des revendications précédentes, dans lequel toutes les sondes dans l'ensemble sont marquées par un fluorophore au niveau de leur extrémité 3'.

5. Solution d'hybridation qui permet la détection de molécules d'ARNm individuelles dans des cellules fixes, perméabilisées comprenant un excès d'un premier ensemble d'au moins un ensemble de sondes, dans laquelle l'ensemble de sondes comprend au moins 30 sondes d'hybridation d'acide nucléique non chevauchantes à marqueur unique avec le même premier marqueur à fluorophore d'une première couleur, dans laquelle les sondes possèdent des séquences complémentaires à différentes régions de liaison de sonde d'une première séquence cible d'une molécule d'ARNm individuelle, dans laquelle lesdites sondes ont une longueur comprise dans la plage de 7 à 30 nucléotides.

6. Solution d'hybridation selon la revendication 5, dans laquelle l'ensemble de sondes comprend 40 à 60 sondes d'hybridation d'acide nucléique non chevauchantes à marqueur unique avec le même premier marqueur à fluorophore d'une première couleur.

7. Solution d'hybridation selon la revendication 5 ou la revendication 6, dans laquelle la solution d'hybridation peut comprendre un mélange de plus d'un ensemble de sondes marquées où chaque ensemble est marqué par un fluorophore distinct.

8. Solution d'hybridation selon l'une quelconque des revendications 5 à 7, dans laquelle chaque sonde est comprise dans la plage allant de 0,2 à 1,0 nanogramme par microlitre.

9. Kit qui permet la détection de molécules d'ARNm individuelles dans des cellules fixes, perméabilisées comprenant :
a) un support lisible par ordinateur, comprenant des instructions, qui lorsqu'elles sont exécutées sur un ordinateur amènent l'ordinateur à effectuer les étapes d'obtention d'un empilement tridimensionnel d'images fluorescentes bidimensionnelles ; de filtration dudit empilement tridimensionnel en utilisant un filtre tridimensionnel ; de comptage d'un nombre total de points tridimensionnels dans ledit empilement tridimensionnel filtré pour chacun d'une pluralité de seuils d'intensité ; d'obtention d'un seuil d'intensité optimum représentatif d'une région de plateau dans un graphique dudit nombre total de points tridimensionnels comparativement au seuil d'intensité auquel ledit nombre total a été compté ; de l'utilisation du nombre total de points tridimensionnels obtenus au niveau dudit seuil optimal comme représentatif d'un nombre de particules fluorescentes détectées dans ledit empilement tridimensionnel ; et
b) l'ensemble de sondes selon l'une quelconque des revendications 1 à 4.

10. Kit selon la revendication 9, dans lequel ledit ensemble de sondes comprend au moins 30 sondes à marqueur unique avec un premier marqueur fluorescent et au moins 30 sondes marquées par un second marqueur fluorescent.

11. Kit selon la revendication 9 ou la revendication 10, dans lequel ladite molécule d'ARNm comprend au moins deux régions cibles, et les marqueurs fluorescents pour chaque région cible sont distinguables.

12. Ensemble de sondes selon l'une quelconque des revendications 1 à 4, solution d'hybridation selon l'une quelconque des revendications 5 à 8, ou kit selon l'une quelconque des revendications 9 à 11, une ou plusieurs des sondes étant des sondes ADN.
